(19) **Europäisches Patentamt / European Patent Office / Office européen des brevets**

(11) **EP 2 977 912 A1**

(12) **EUROPÄISCHE PATENTANMELDUNG**

(43) Veröffentlichungstag:
**27.01.2016 Patentblatt 2016/04**

(21) Anmeldenummer: **14002593.3**

(22) Anmeldetag: **25.07.2014**

(51) Int Cl.:
**G06F 17/18** *(2006.01)*  **G06F 19/00** *(2011.01)*

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**

(71) Anmelder: **Liebel, Franz-Peter, Dr.**
**72663 Grossbettlingen (DE)**

(72) Erfinder: **Liebel, Franz-Peter, Dr.**
**72663 Grossbettlingen (DE)**

(54) **Automatisierte Diagnostik**

(57) Universell einsetzbares Verfahren zur Anwendung auf zahlreichen Wissensgebieten, zum Beispiel der Erdbebenforschung, der geologischen Prospektion, der Kriminalistik, der Flugunfalluntersuchungen, der on-board-Diagnostik im Automobil- und Flugzeugbau und in der Medizin, unter anderem zur Auswertung von EKG-Aufzeichnungen. Das Verfahren ist immer dann einsetzbar, wenn entschieden werden muß über das Zutreffen mehrerer hypothetischer Annahmen oder Diagnosen, die Ursachen sind für eine Anzahl von Indizien oder Symptomen. Neben einer solchen Symptommenge wird berücksichtigt, daß die Zunahme der Wahrscheinlichkeit für eine beliebige Diagnose zu einer Abnahme der Wahrscheinlichkeit einer konkurrierenden Diagnose führt, so daß die Verwendung eines algebraischen Verfahren angezeigt war.

In der praktischsten Version setzt man die a-priori-Wahrscheinlichkeit für alte Diagnosen als gleich an und berechnet den Aufwertungsfaktor AF als Quotienten aus a-posteriori- zu a-priori-Wahrschein-lichkeit. Ersetzt man die Gleichwahrscheinlichkeit durch die "wahre" a-priori-Wahrscheinlichkeit der einzelnen Diagnosen, oder durch eine bedingte Wahrscheinlichkeit, die in der Bedingung die Ursachen der Diagnosen berücksichtigt, so wird das System deutlich leistungsstärker, noch verstärkt bei zusätzlicher Berücksichtigung von Inhibitoren.

An Zahlenwerten benötigt werden nur die Wahrscheinlichkeit eines beliebigen Symptoms bei Vorliegen einer beliebigen Diagnose, d.h. es werden bedingte Wahrscheinlichkeiten verwendet, die in der Bedingung nur eine einzige nicht-negierte Diagnose enthalten, so daß die Ermittlung der Zahlenwerte problemlos wird.

Ausgestattet ist die Patentanmeldung mit einem vollständigen und perfekt arbeitenden Anwendungsbeispiel, das ohne jede Änderung unmittelbar für die Auswertung von EKG-Aufzeichnungen verwendet werden kann.

EP 2 977 912 A1

**Beschreibung**

a) Universell auf vielen Wissensgebieten einsetzbares Verfahren zur automatisierten Diagnostik, wobei Symptome und der Einfluß der Diagnosen untereinander genutzt werden. Das Verfahren bestimmt für jede Diagnose $K_i$' den jeweiligen Aufwertungsfaktor AF(i) und die a-posteriori-Wahrscheinlichkeit $x_i$, ausgehend von der jeweiligen a-priori-Wahrscheinlichkeit.

b) Stand der Technik ist EP 99105884.3. Dort werden Gleichungssysteme und auch einelementig bedingte Wahrscheinlichkeiten **verwendet.** Um solche theoretischen Verfahren jedoch praktikabel zu gestalten und weiter zu entwickeln, waren Verbesserungen und erfinderische Neuigkeiten nötig. Dazu zählen (zgl. Vorteile gegenüber Stand der Technik):

- Konkrete Angabe der vollständigen Gleichungssysteme, die in genau dieser Gestalt programmiert werden können, und zwar für 2, 3 und 4 Diagnosen.
- Einführung von Koeffizienten $a_{ik}$ und $b_{ik}$, die unverändert für andere Anwendungen übernommen bzw. aus dem definierenden Beispiel abgelesen werden können.
- Schema für eine mechanisierte Aufstellung der $a_{ik}$ und $b_{ik}$.
- Einführung schematisierter Tabellen mit identischen Folge-Ereignissen in einer Zeile.
- Verwendung von Aufwertungsfaktoren AF(i) bei gleicher a-priori-Wahrscheinlichkeit.
- Unkompliziertes Vorgehen bei der Berücksichtigung von Ursachen und Inhibitoren.
- Neue Faktoren $f_{ij}$ für einfache Vorgehensweise bei Nicht-Vorliegen eines Symptoms.
- Laufende Aktualisierung der verwendeten Wahrscheinlichkeiten.
- Keine iterativen Lösungsmethoden, sondern kommerzielle Berechnungsprogramme.
- Vollständiges arbeitsfähiges Anwendungsbeispiel, als Konstruktionsvorlage nutzbar.
- Bedienung (bei Maple 18) mit einem einzigen Mausklick.

c) Ausführlich anschließend nach Ziffer f).

d) Keine Zeichnungen.

e) Ein Beispiel wird vollständig vorgeführt und ist nach Ziffer f) angefügt (32 Seiten). In diesem Beispiel werden die in der Beschreibung enthaltene Abb. 1 und die darauf bezogene Tabelle 1 abgearbeitet. Es arbeitet perfekt und dient zur automatisierten Auswertung von EKG-Aufzeichnungen, kann jedoch auch für andere Zwecke und Anwendungsbereiche als Arbeitsvorlage genutzt werden.

f) Eine kommerzielle automatisierte EKG-Auswertung ist damit ermöglicht und durchführbar - ohne Änderungen. Das angefügte Beispiel arbeitet nach Anpassung bei allen Diagnoseentscheidungen.

Beschreibung

c) (Fortsetzung)

Automatisierte Diagnostik

Alle hypothetische Annahmen oder Diagnosen werden erfaßt und mit ihren jeweiligen Indizien bzw. Symptomen aufgelistet. Die zuvorderst zu diagnostizierende Annahme wird ausgewählt und mit $K_1$' bezeichnet, die zu $K_1$' gehörenden Differentialdiagnosen erhalten die Bezeichnungen $K_2$', $K_3$' und $K_4$'.

Die von den jeweiligen K'-Elementen erzeugten Folge-Ereignisse bilden jeweils die Mengen der F-Elemente; so gehören $\{F_{11},..., F_{16}\}$ zu $K_1$', $\{F_{21},..., F_{26}\}$ zu $K_2$', $\{F_{31},..., F_{36}\}$ zu $K_3$' und $\{F_{41},..., F_{46}\}$ zu $K_4$', wobei die F-Elemente zwar unterschiedlich indiziert sind, aber in zahlreichen Fällen dasselbe Indiz oder Krankheitssymptom bezeichnen. Allgemein verweist der in $F_{ij}$ enthaltene Index i auf das verursachende Element $K_i$, i := 1,...,4, während j die laufende Nummerierung darstellt, j :=1,...,6.

Als ein Beispiel wird nachfolgend in Tab. 1 eine willkürlich gewählte Kausalstruktur dargestellt. Eine solche Kausalstruktur wird berechnet, d.h. für alle aufgeführten K'-Elemente wird, ausgehend von der jeweiligen a-priori-Wahrscheinlichkeit, die a-posteriori-Wahrscheinlichkeit bestimmt. Einen Einfluß auf die a-posteriori-Wahrscheinlichkeit eines beliebig gewählten $K_1$' besitzen die Folge-Ereignisse $F_{1j}$ und die Konkurrenten $K_2$',..., $K_4$'. Die bei dem zu diagnostizierenden Fall schließlich festgestellte Symptommenge bestimmt, welche der F-Elemente negiert und welche nicht-negiert in die Berechnung eingehen.

Die Berechnungen, die für $K_1$' detailliert vorgestellt werden, sind in gleicher Weise auch für alle übrigen K'-Elemente durchzuführen, wobei die Diagnosenmenge, z.B. $\{K_1', K_2', K_3', K_4'\}$ stets erhalten bleibt. Ist nun etwa $K_5$' eine weitere Differentialdiagnose, so wird $\overline{K}_5$ in die Gruppe der K'-Elemente aufgenommen und als solches (gedanklich) mitgeführt, d.h. das Vorliegen von $K_5$ wird vorweg ausgeschlossen. Zur Sicherung des Berechnungsergebnisses kann im Anschluß jede derart ausgeschlossene Diagnose zur Leitdiagnose erhoben und, versehen mit einer eigenen K'-Gruppierung, in gleicher Weise berechnet werden.

Am Beginn steht die Berechnung der ersten Leitdiagnose (hier: $K_1$'), die dadurch ausgewählt wird, daß der Durchschnitt der Mengen, gebildet aus der Menge der $K_1$' zugeordneten F-Elemente und der Menge der festgestellten Symptome, maximal ist. Danach werden die zu $K_1$'gehörenden Differentialdiagnosen in gleicher Weise wie $K_1$' berechnet; die Menge der K'-Elemente bleibt unverändert, die F-Elemente sind dann den jeweiligen K'-Elementen zugehörig.

$\{K_1', K_2', K_3', K_4'\}$ enthält die gesuchten Diagnosen (K steht für Konkurrent). Die Elemente besitzen eine unbekannte Zutreffenswahrscheinlichkeit (0 < p < 1) und tragen deshalb eine Strichkennung. Die Anzahl dieser Konkurrenten ist aus Zweckmäßigkeitsgründen auf vier begrenzt. Vermindert oder erhöht man die Anzahl um jeweils ein Strichelement, so halbiert bzw. verdoppelt sich die Länge der Berechnungsgleichungen für die gesuchten Unbekannten. Die an die erste Stelle gesetzte Diagnose $K_1$' ist die Leitdiagnose, $\{K_2', K_3', K_4'\}$ sind die Differentialdiagnosen zu $K_1$'.

$\{F_{11}, F_{12}, F_{13}, F_{14}, F_{15}, F_{16}\}$ enthält die Symptome von $K_1$' (F steht für Folge-Ereignis). Die Anzahl der berücksichtigten Folge-Ereignisse ist **frei wählbar und hier willkürlich auf sechs Elemente** angesetzt. Die Ereignisse aus $\{F_{11}, F_{12}, F_{13}, F_{14}, F_{15}, F_{16}\}$ sind in der Struktur mit der Wahrscheinlichkeit (p =1) eingetragen, sie werden aber (p = 0) aufweisen, falls die Symptommenge sie mit dieser Wahrscheinlichkeit ausweist. Außerdem ist ein beliebiges Ereignis aus der Menge $\{F_{11}, F_{12}, F_{13}, F_{14}, F_{15}, F_{16}\}$ zu entfernen, falls dieses Ereignis nicht das folgende Kriterium erfüllt: **Jedes der von $K_1$' erzeugten Elemente aus der Menge $\{F_{11}, F_{12}, F_{13}, F_{14}, F_{15}, F_{16}\}$ besitzt mindestens eine weitere Ursache in $\{K_2', K_3', K_4'\}$.**

Aufgrund der letztgenannten Eigenschaft ist es zweckmäßig, wenn für die $K_i$' und die zugehörigen $F_{ij}$ eine tabellarische Übersicht angelegt wird, ergänzt durch die insgesamt in Betracht zu ziehenden Symptome S.

Zum Zweck geordneter Vorgehensweise werden zunächst für alle Diagnosen $K_i$', zum Beispiel für $K_{i0}$ die Zahlenwerte der $p(F_{i0j} \mid K_{i0} \sim)$, j:= 1,..., 6, ermittelt, wobei hier die i-Indizierung bei F und K gleich ist. Das Symbol ~ bezeichnet einen Verbund, der mit Ausnahme des vor ~ stehenden K-Elements alle konkurrierenden Diagnosen in negierter Form enthält.

| Symptome | $K_1$: Akute Myokarditis $p(F_{1j} \mid K_1\sim)$ | $K_2$: Dilatative Kardiomyopathie $p(F_{2j} \mid K_2\sim)$ | $K_3$: Koronare Herzkrankheit $p(F_{3j} \mid K_3\sim)$ | $K_4$: Toxische Kardiomyopathie $p(F_{4j} \mid K_4\sim)$ |
|---|---|---|---|---|
| S1: QRS-Komplex > 0.12 sec | $F_{11}$ * 0.6 | $F_{21}$ * 0.6 | | $F_{41}$ * 0.5 |
| S2: S-Zacken, tief in V1 + V2 | $F_{12}$ * 0.5 | $F_{22}$ * 0.5 | $F_{31}$ * 0.3 | $F_{42}$ * 0.3 |
| S3: QRS-Komplex gesplittert in V5 + V6 | $F_{13}$ * 0.5 | $F_{23}$ * 0.5 | | $F_{43}$ * 0.3 |
| S4: PQ-Zeit > 0.1 sec | $F_{14}$ * 0.6 | | $F_{32}$ * 0.2 | $F_{44}$ * 0.5 |
| S5: RR-Abstände variieren | $F_{15}$ * 0.9 | | $F_{33}$ * 0.5 | |
| S6: Herzfrequenz > 100/min | $F_{16}$ * 0.7 | $F_{24}$ * 0.4 | $F_{34}$ * 0.4 | |
| S7: P-Welle Sägezahnartig | | $F_{25}$ * 0.6 | $F_{35}$ * 0.6 | |
| S8: ST-Senkung | | $F_{26}$ * 0.7 | | $F_{45}$ * 0.2 |
| S9: T-Negativierung in V2 | | | $F_{36}$ * 0.7 | $F_{46}$ * 0.4 |
| Tabelle 1: Tabellarische Erfassung der Abb. 1. Die Symptome S1,..., S9 sind willkürlich gewählt. Die $p(F_{ij} \mid K_i \sim)$ sind geschätzte Zahlenwerte. | | | | |

Alle insgesamt zu berücksichtigenden Symptome werden in die erste Spalte aufgenommen. Alle Folge-Ereignisse einer Diagnose sind in der dieser Diagnose zugehörigen Spalte einzutragen, zusammen mit ihren $p(F_{i0j} \mid K_{i0} \sim)$

-Zahlenwerten, wobei identische Folge-Ereignisse, d.h. Ereignisse mit unterschiedlicher $F_{ij}$-Indizierung aber derselben Symptom-Zugehörigkeit, in einer Zeile stehen.

Der Zahlenwert zum Beispiel für $p(F_{26}|K_4 \sim)$ kann mühelos abgelesen werden als Null, wenn es am Kreuzungspunkt der $F_{26}$-Zeile und der $K_4$-Spalte keine Eintragung gibt, oder aber im Fall einer Eintragung als Zahlenwert, der für ein zu $K_4$ gehörendes Folge-Ereignis bereits ermittelt ist. Zum Beispiel für $p(F_{45}||K_4 \sim)$, wenn nämlich die beiden Folge-Ereignisse $F_{26}$ und $F_{45}$ zum selben Symptom in der ersten Spalte der Tabelle gehören.

Die Abb. 1 und die Tabelle 1 werden für das angefügte Berechnungsbeispiel ohne Änderungen verwendet. Dieses Berechnungsbeispiel kann als ein Rohbaugerüst eingesetzt werden, um Werkzeuge für Diagnoseentscheidungen auch außerhalb der Medizin zu erstellen.

Voraussetzungen

- Die Elemente in $\{K_1', K_2', K_3', K_4'\}$ sind stochastisch unabhängig.
- Die Elemente in $\{K_1', K_2', K_3', K_4'\}$ sind selbständige Ursachen (das bedeutet, daß die Inhibitoren der von den K'-Elementen wegführenden Kausalwege untereinander und gegenüber den K'-Elementen stochastisch unabhängig sind).
- $\{K_1', K_2', K_3', K_4'\}$ enthält **alle** Ereignisse, die Ursache sind für <u>mehr als ein</u> Element aus $\{F_{11}, F_{12}, F_{13}, F_{14}, F_{15}, F_{16}\}$.

Bemerkung zu den Voraussetzungen

Für zwei beliebige Elemente aus $\{F_{11}, F_{12}, F_{13}, F_{14}, F_{15}, F_{16}\}$ läßt sich **bedingte** stochastische Unabhängigkeit erreichen, falls die Bedingung sämtliche gemeinsamen Ursachen der beiden Ereignisse enthält. Ist das nicht der Fall, und ist z.B. $K_5'$ eine Ursache für mindestens zwei Elemente aus $\{F_{11}, F_{12}, F_{13}, F_{14}, F_{15}, F_{16}\}$, so wird das Nicht-Vorliegen von $K_5'$ angenommen und $\overline{K_5}$ in der Kausalstruktur und in den Berechnungen mitgeführt (wobei die gedankliche Mitführung genügt).

Berechnung von $x_1$

Als <u>W</u>ertungsumgebung von $K_1'$ (in kurzer Schreibweise:$W(K_1')$) wird ein Verbund von Ereignissen bezeichnet, der diejenigen Elemente der Kausalstruktur enthält, die Einfluß auf die Zutreffenswahrscheinlichkeit von $K_1$ besitzen. In der angegebenen Kausalstruktur besteht $W(K_1')$ aus den beiden Verbunden ($F_{11} F_{12} F_{13} F_{14} F_{15} F_{16}$) und ($K_2' K_5' K_4'$). Die Zutreffenswahrscheinlichkeit von $K_1$ unter der Bedingung der Wertungsumgebung von $K_1'$, also $p(K_1|W(K_1'))$ ist die gesuchte Unbekannte $x_1$. Historisch bedingt **schreibt man für $p(K_1|W(K_1'))$** auch kürzer $p(K_1|K_1')$. Entsprechendes gilt für $x_2$, $x_3$ und $x_4$. Damit folgt:

$$x_1 := p(K_1|W(K_1')) := p(K_1|K_1`) = p(K_1| F_{11} ... F_{16} K_2' K_3' K_4').$$

$$x_2 := p(K_2|W(K_2')) := p(K_2|K_2`) = p(K_2| F_{21} ... F_{26} K_1' K_3' K_4').$$

$$x_3 := p(K_3|W(K_3')) := p(K_3|K_3`) = p(K_3| F_{31} ... F_{36} K_2' K_1' K_4').$$

$$x_4 := p(K_4|W(K_4')) := p(K_4|K_4`) = p(K_4| F_{41} ... F_{46} K_2' K_3' K_1').$$

Für $x_1 := p(K_1| F_{11} ... F_{16} K_2' K_3' K_4')$ gilt:

$$x_1 :=$$

$$p(K_1 \mid F_{11}...F_{16}K_2'K_3'K_4') =$$

$$\frac{p(K_1 F_{11}...F_{16}K_2'K_3'K_4')}{p(K_1 F_{11}...F_{16}K_2'K_3'K_4') + p(\overline{K}_1 F_{11}...F_{16}K_2'K_3'K_4')} =$$

$$\frac{1}{1 + \dfrac{p(F_{11}...F_{16}\overline{K}_1 K_2'K_3'K_4')}{p(F_{11}...F_{16}K_1 K_2'K_3'K_4')}} =$$

$$\frac{1}{1 + \dfrac{p(F_{11}...F_{16} \mid \overline{K}_1 K_2'K_3'K_4')p(\overline{K}_1 K_2'K_3'K_4')}{p(F_{11}...F_{16} \mid K_1 K_2'K_3'K_4')p(K_1 K_2'K_3'K_4')}} =$$

$$\frac{1}{1 + \dfrac{p(F_{11}...F_{16} \mid \overline{K}_1 K_2'K_3'K_4')p(\overline{K}_1 \mid K_2'K_3'K_4')}{p(F_{11}...F_{16} \mid K_1 K_2'K_3'K_4')p(K_1 \mid K_2'K_3'K_4')}} = (wg.\,Unabhängigkeit\,der\,K)$$

$$\frac{1}{1 + \dfrac{p(F_{11}...F_{16} \mid \overline{K}_1 K_2'K_3'K_4')p(\overline{K}_1)}{p(F_{11}...F_{16} \mid K_1 K_2'K_3'K_4')p(K_1)}}.$$

Abkürzend: $Z_1 := p(F_{11}...F_{16}|\overline{K}_1 K_2'K_3'K_4')$, $N_1 := p(F_{11}...F_{16}|K_1 K_2'K_3'K_4')$.

Nach **<u>Linearer Interpolation</u>** folgt für $Z_1$ :

$$p(F_{11}...F_{16} \mid \overline{K}_1 K_2'K_3'K_4') =$$

$$p(F_{11}...F_{16} \mid \overline{K}_1 K_2 K_3 K_4) \cdot p(K_2 \mid K_2') \cdot p(K_3 \mid K_3') \cdot p(K_4 \mid K_4') +$$

$$p(F_{11}...F_{16} \mid \overline{K}_1 K_2 K_3 \overline{K}_4) \cdot p(K_2 \mid K_2') \cdot p(K_3 \mid K_3') \cdot p(\overline{K}_4 \mid K_4') +$$

$$p(F_{11}...F_{16} \mid \overline{K}_1 K_2 \overline{K}_3 K_4) \cdot p(K_2 \mid K_2') \cdot p(\overline{K}_3 \mid K_3') \cdot p(K_4 \mid K_4') +$$

$$p(F_{11}...F_{16} \mid \overline{K}_1 K_2 \overline{K}_3 \overline{K}_4) \cdot p(K_2 \mid K_2') \cdot p(\overline{K}_3 \mid K_3') \cdot p(\overline{K}_4 \mid K_4') +$$

$$p(F_{11}...F_{16} \mid \overline{K}_1 \overline{K}_2 K_3 K_4) \cdot p(\overline{K}_2 \mid K_2') \cdot p(K_3 \mid K_3') \cdot p(K_4 \mid K_4') +$$

$$p(F_{11}...F_{16} \mid \overline{K}_1 \overline{K}_2 K_3 \overline{K}_4) \cdot p(\overline{K}_2 \mid K_2') \cdot p(K_3 \mid K_3') \cdot p(\overline{K}_4 \mid K_4') +$$

$$p(F_{11}...F_{16} \mid \overline{K}_1 \overline{K}_2 \overline{K}_3 K_4) \cdot p(\overline{K}_2 \mid K_2') \cdot p(\overline{K}_3 \mid K_3') \cdot p(K_4 \mid K_4') +$$

$$p(F_{11}...F_{16} \mid \overline{K}_1 \overline{K}_2 \overline{K}_3 \overline{K}_4) \cdot p(\overline{K}_2 \mid K_2') \cdot p(\overline{K}_3 \mid K_3') \cdot p(\overline{K}_4 \mid K_4').$$

Ebenso verläuft die Lineare Interpolation bei $p(F_{11}...F_{16}|K_1 K_2'K_3'K_4')$, wobei lediglich $\overline{K}_1$ gegen $K_1$ getauscht wird.

Es wird eine vereinfachende Schreibweise eingeführt, als definierende Beispiele dienen $p(F_{11}|\sim) :=$ $p(F_{11}|\overline{K_1 K_2 K_3 K_4})$ und $p(F_{11}|K_1 \sim) := p(F_{11}|K_1 \overline{K_2 K_3 K_4})$, wobei $\sim$ einen Verbund bezeichnet, der mit Ausnahme des vor $\sim$ stehenden K-Elements alle konkurrierenden Diagnosen in negierter Form enthält.

Für die bedingten Wahrscheinlichkeiten, die in derartigen Interpolationen stehen, werden die Bezeichnungen $a_{ik}$ und $b_{ik}$ gewählt werden, $k := 0,..., 7$, und zwar $a_{ik}$ bei den Interpolationen der $Z_i$ und $b_{ik}$ bei den Interpolationen der $N_i$. so daß zum Beispiel der erste Faktor der obigen Gleichung ersetzt wird durch $a_{10}$ mit $a_{10} := p(F_{11}...F_{16} \mid \overline{K}_1 K_2 K_3 K_4)$.

Es folgt eine Faktorzerlegung in $a_{10} := p(F_{11} \mid \overline{K}_1 K_2 K_3 K_4) \cdot ... \cdot p(F_{16}|\overline{K}_1 K_2 K_3 K_4)$, die dadurch ermöglicht wird, daß der Verbund $(\overline{K}_1 K_2 K_3 K_4)$ alle Ursachen der$\{F_{11},...,F_{16}\}$ enthält, so daß die bedingte Unabhängigkeit der F-Elemente erreicht wird.

Die weitere Faktorzerlegung von Ausdrückender Form $p(F_{11}|\overline{K}_1K_2K_3K_4)$ in $p(F_{11}|K_1K_2K_3K_4) := [1 - P(\overline{F}_{11}|K_2 \sim) \cdot p(\overline{F}_{11}|K_3 \sim) \cdot p(\overline{F}_{11}|K_4 \sim)]$ wird dadurch ermöglicht, daß die $\{K_1, K_2, K_3, K_4\}$ als selbständige Ursachen von $F_{11}$ vorausgesetzt sind, so daß der "Faktorisierungssatz bei vollzähligen Ursachen" angewendet werden kann. Damit folgt:

$$
\begin{aligned}
&a_{10} := \\
&p(F_{11}...F_{16} \mid \overline{K}_1 K_2 K_3 K_4) = \\
&p(F_{11} \mid \overline{K}_1 K_2 K_3 K_4) \cdot ... \cdot p(F_{16} \mid \overline{K}_1 K_2 K_3 K_4) = \\
&\left[1 - p(\overline{F}_{11} \mid \overline{K}_1 K_2 \overline{K}_3 \overline{K}_4) \cdot p(\overline{F}_{11} \mid \overline{K}_1 \overline{K}_2 K_3 \overline{K}_4) \cdot p(\overline{F}_{11} \mid \overline{K}_1 \overline{K}_2 \overline{K}_3 K_4)\right] \cdot \\
&\quad \vdots \\
&\cdot \left[1 - p(\overline{F}_{16} \mid \overline{K}_1 K_2 \overline{K}_3 \overline{K}_4) \cdot p(\overline{F}_{16} \mid \overline{K}_1 \overline{K}_2 K_3 \overline{K}_4) \cdot p(\overline{F}_{16} \mid \overline{K}_1 \overline{K}_2 \overline{K}_3 K_4)\right] = \\
&\left[1 - p(\overline{F}_{11} \mid K_2 \sim) \cdot p(\overline{F}_{11} \mid K_3 \sim) \cdot p(\overline{F}_{11} \mid K_4 \sim)\right] \cdot \\
&\quad \vdots \\
&\cdot \left[1 - p(\overline{F}_{16} \mid K_2 \sim) \cdot p(\overline{F}_{16} \mid K_3 \sim) \cdot p(\overline{F}_{16} \mid K_4 \sim)\right]
\end{aligned}
$$

<u>Hinweis</u>

In den Voraussetzungen wurde lediglich gefordert, daß die Menge $\{K_1{}', K_2{}', K_3{}', K_4{}'\}$ diejenigen K'-Elemente enthält, die mehr als ein F-Element erzeugen. Demzufolge können außerhalb des Bedingungsverbunds, der aus Elementen der Menge $\{K_1, K_2, K_3, K_4\}$ gebildet ist (unter Umständen ergänzt durch negierte K-Elemente), auch Ursachen existieren, die ein einzelnes F-Element erzeugen.

Auf eine Wahrscheinlichkeit der Form $p(F_{11} \mid \overline{K}_1K_2K_3K_4)$ ist in einem solchen Fall die <u>Faktorzerlegung bei verdeckten Ursachen</u> anzuwenden, so daß sich ergibt:

$$
p(F_{11} \mid \overline{K}_1 K_2 K_3 K_4) = 1 - \frac{p(\overline{F}_{11} \mid \overline{K}_1 K_2 \overline{K}_3 \overline{K}_4) \cdot p(\overline{F}_{11} \mid \overline{K}_1 \overline{K}_2 K_3 \overline{K}_4) \cdot p(\overline{F}_{11} \mid \overline{K}_1 \overline{K}_2 \overline{K}_3 K_4)}{p(\overline{F}_{11} \mid \overline{K}_1 \overline{K}_2 \overline{K}_3 \overline{K}_4)^2} .
$$

Für praktische Belange wird $p(F_{11} \mid \sim) = 0$ gesetzt. Dies ist eine vorläufige Regelung, die jederzeit zur Verbesserung der Genauigkeit aufgehoben werden kann.

Weiter ist anzumerken, daß für ein beliebiges $F_{ij}$ und ein beliebiges $K_i$ stets $p(F_{ij} \mid K_i \sim) = 0$ gilt, falls $F_{ij}$ nicht $K_i$ als Ursache hat. So könnte z.B. in obiger Berechnung $p(F_{11} \mid K_1K_2K_3K_4)$ zu Null werden, falls $F_{11}$ weder von $K_2$, noch von $K_3$, noch von $K_4$ verursacht wird. Diese Möglichkeit ist jedoch mit der Forderung ausgeschlossen, daß jedes $F_{ij}$ von mindestens zwei K'-Elementen verursacht sein muß.

Die korrekte Reihenfolge der Gleichungen für die direkte Programmierung von $a_{10}$ ergibt:

$$p(F_{11} \mid \overline{K}_1 K_2 K_3 K_4) := \left[ 1 - p(\overline{F}_{11} \mid K_2 \sim) \cdot p(\overline{F}_{11} \mid K_3 \sim) \cdot p(\overline{F}_{11} \mid K_4 \sim) \right];$$

$$\vdots$$

$$p(F_{16} \mid \overline{K}_1 K_2 K_3 K_4) := \left[ 1 - p(\overline{F}_{16} \mid K_2 \sim) \cdot p(\overline{F}_{16} \mid K_3 \sim) \cdot p(\overline{F}_{16} \mid K_4 \sim) \right];$$

$$a_{10} :=$$

$$(f_{11} \cdot p(F_{11} \mid \overline{K}_1 K_2 K_3 K_4) + (1 - f_{11}) \cdot (1 - p(F_{11} \mid \overline{K}_1 K_2 K_3 K_4)) \cdot$$

$$\vdots$$

$$\cdot (f_{16} \cdot p(F_{16} \mid \overline{K}_1 K_2 K_3 K_4) + (1 - f_{16}) \cdot (1 - p(F_{16} \mid \overline{K}_1 K_2 K_3 K_4));$$

Als ein weiteres Beispiel wird abschließend $b_{10}$ angegeben, den ersten Faktor von $N_1$ nach Linearer Interpolation, d.h. $b_{10} := p(F_{11}...F_{16} \mid K_1 K_2 K_3 K_4)$, in der für die Programmierung erforderlichen Reihenfolge der Gleichungen:

$$p(F_{11} \mid K_1 K_2 K_3 K_4) := \left[ 1 - p(\overline{F}_{11} \mid K_1 \sim) \cdot p(\overline{F}_{11} \mid K_2 \sim) \cdot p(\overline{F}_{11} \mid K_3 \sim) \cdot p(\overline{F}_{11} \mid K_4 \sim) \right];$$

$$\vdots$$

$$p(F_{16} \mid K_1 K_2 K_3 K_4) := \left[ 1 - p(\overline{F}_{16} \mid K_1 \sim) \cdot p(\overline{F}_{16} \mid K_2 \sim) \cdot p(\overline{F}_{16} \mid K_3 \sim) \cdot p(\overline{F}_{16} \mid K_4 \sim) \right];$$

$$b_{10} :=$$

$$(f_{11} \cdot p(F_{11} \mid K_1 K_2 K_3 K_4) + (1 - f_{11}) \cdot (1 - p(F_{11} \mid K_1 K_2 K_3 K_4)) \cdot$$

$$\vdots$$

$$\cdot (f_{16} \cdot p(F_{16} \mid K_1 K_2 K_3 K_4) + (1 - f_{16}) \cdot (1 - p(F_{16} \mid K_1 K_2 K_3 K_4)).$$

Alle weiteren Einzelheiten ergeben sich aus den Patentansprüchen und insbesondere dem angefügten ausführlichen und vollständigen Beispiel, das auch als definierendes Beispiel gilt.

```
> `Beschreibung e) (Fortsetzung)`:
>
> `Beispiel`:
> `Abgearbeitet Tabelle 1`:
> `Zur Diagnosestellung werden S1 bis S4 als vorhanden angenommen`:
> `Start mit einem einzigen Klick auf das !!!-Symbol`;
```

$$\text{Start mit einem einzigen Klick auf das !!!-Symbol} \tag{1}$$

```
>
> S1 := 1; S2 := 1; S3 := 1; S4 := 1; S5 := 0; S6 := 0; S7 := 0; S8 := 0; S9 := 0;
```

$$S1 := 1$$
$$S2 := 1$$
$$S3 := 1$$
$$S4 := 1$$
$$S5 := 0$$
$$S6 := 0$$
$$S7 := 0$$
$$S8 := 0$$
$$S9 := 0 \tag{2}$$

```
>
> f11 := S1; f12 := S2; f13 := S3; f14 := S4; f15 := S5; f16 := S6;
```

$$f11 := 1$$
$$f12 := 1$$
$$f13 := 1$$
$$f14 := 1$$
$$f15 := 0$$
$$f16 := 0 \tag{3}$$

```
>
> p(F11`|`K1`~`) := 0.6; p(F11`|`K2`~`) := 0.6; p(F11`|`K3`~`) := 0; p(F11`|`K4`~`) := 0.5;
```

$$p(F11 \, `|` \, K1 \, `~`) := 0.6$$
$$p(F11 \, `|` \, K2 \, `~`) := 0.6$$
$$p(F11 \, `|` \, K3 \, `~`) := 0$$
$$p(F11 \, `|` \, K4 \, `~`) := 0.5 \tag{4}$$

```
>
> p(F12`|`K1`~`) := 0.5; p(F12`|`K2`~`) := 0.5; p(F12`|`K3`~`) := 0.3; p(F12`|`K4`~`) := 0.3;
```

$$p(F12 \, `|` \, K1 \, `~`) := 0.5$$
$$p(F12 \, `|` \, K2 \, `~`) := 0.5$$
$$p(F12 \, `|` \, K3 \, `~`) := 0.3$$
$$p(F12 \, `|` \, K4 \, `~`) := 0.3 \tag{5}$$

```
> p(F13`|`K1`~`) := 0.5; p(F13`|`K2`~`) := 0.5; p(F13`|`K3`~`) := 0; p(F13`|`K4`~`) := 0.3;
```

$$p(F13 \, `|` \, K1 \, `~`) := 0.5$$
$$p(F13 \, `|` \, K2 \, `~`) := 0.5$$
$$p(F13 \, `|` \, K3 \, `~`) := 0$$
$$p(F13 \, `|` \, K4 \, `~`) := 0.3 \tag{6}$$

```
> p(F14`|`K1`~`) := 0.6; p(F14`|`K2`~`) := 0; p(F14`|`K3`~`) := 0.2; p(F14`|`K4`~`) := 0.5;
```

$$p(F14 \, `|` \, K1 \, `~`) := 0.6$$

$$p(F14 \text{ '|' } K2 \text{ '~'}) := 0$$

$$p(F14 \text{ '|' } K3 \text{ '~'}) := 0.2$$

$$p(F14 \text{ '|' } K4 \text{ '~'}) := 0.5 \tag{7}$$

> $p(F15\text{'|'}K1\text{'~'}) := 0.9; p(F15\text{'|'}K2\text{'~'}) := 0; p(F15\text{'|'}K3\text{'~'}) := 0.5; p(F15\text{'|'}K4\text{'~'}) := 0;$

$$p(F15 \text{ '|' } K1 \text{ '~'}) := 0.9$$

$$p(F15 \text{ '|' } K2 \text{ '~'}) := 0$$

$$p(F15 \text{ '|' } K3 \text{ '~'}) := 0.5$$

$$p(F15 \text{ '|' } K4 \text{ '~'}) := 0 \tag{8}$$

> $p(F16\text{'|'}K1\text{'~'}) := 0.7; p(F16\text{'|'}K2\text{'~'}) := 0.4; p(F16\text{'|'}K3\text{'~'}) := 0.4; p(F16\text{'|'}K4\text{'~'}) := 0;$

$$p(F16 \text{ '|' } K1 \text{ '~'}) := 0.7$$

$$p(F16 \text{ '|' } K2 \text{ '~'}) := 0.4$$

$$p(F16 \text{ '|' } K3 \text{ '~'}) := 0.4$$

$$p(F16 \text{ '|' } K4 \text{ '~'}) := 0 \tag{9}$$

> $Digits := 2;$

$$Digits := 2 \tag{10}$$

> $p(F11\text{'|'}\overline{K1}\,K2\,K3\,K4) := 1 - (1 - p(F11\text{'|'}\underline{K2}\text{'~'})) \cdot (1 - p(F11\text{'|'}K3\text{'~'})) \cdot (1 - p(F11\text{'|'}K4\text{'~'}));$

$$p(F11 \text{ '|' } \overline{K1}\,K2\,K3\,K4) := 0.80 \tag{11}$$

>

> $p(F12\text{'|'}\overline{K1}\,K2\,K3\,K4) := 1 - (1 - p(F12\text{'|'}\underline{K2}\text{'~'})) \cdot (1 - p(F12\text{'|'}K3\text{'~'})) \cdot (1 - p(F12\text{'|'}K4\text{'~'}));$

$$p(F12 \text{ '|' } \overline{K1}\,K2\,K3\,K4) := 0.76 \tag{12}$$

>

> $p(F13\text{'|'}\overline{K1}\,K2\,K3\,K4) := 1 - (1 - p(F13\text{'|'}\underline{K2}\text{'~'})) \cdot (1 - p(F13\text{'|'}K3\text{'~'})) \cdot (1 - p(F13\text{'|'}K4\text{'~'}));$

$$p(F13 \text{ '|' } \overline{K1}\,K2\,K3\,K4) := 0.65 \tag{13}$$

>

> $p(F14\text{'|'}\overline{K1}\,K2\,K3\,K4) := 1 - (1 - p(F14\text{'|'}\underline{K2}\text{'~'})) \cdot (1 - p(F14\text{'|'}K3\text{'~'})) \cdot (1 - p(F14\text{'|'}K4\text{'~'}));$

$$p(F14 \text{ '|' } \overline{K1}\,K2\,K3\,K4) := 0.60 \tag{14}$$

>

> $p(F15\text{'|'}\overline{K1}\,K2\,K3\,K4) := 1 - (1 - p(F15\text{'|'}\underline{K2}\text{'~'})) \cdot (1 - p(F15\text{'|'}K3\text{'~'})) \cdot (1 - p(F15\text{'|'}K4\text{'~'}));$

$$p(F15 \text{ '|' } \overline{K1}\,K2\,K3\,K4) := 0.5 \tag{15}$$

>

> $p(F16\text{'|'}\overline{K1}\,K2\,K3\,K4) := 1 - (1 - p(F16\text{'|'}\underline{K2}\text{'~'})) \cdot (1 - p(F16\text{'|'}K3\text{'~'})) \cdot (1 - p(F16\text{'|'}K4\text{'~'}));$

$$p(F16 \text{ '|' } \overline{K1}\,K2\,K3\,K4) := 0.64 \tag{16}$$

>

> $a10 := (f11 \cdot p(F11\text{'|'}\overline{K1}\,K2\,K3\,K4) + (1 - f11) \cdot (1 - p(F11\text{'|'}\overline{K1}\,K2\,K3\,K4))) \cdot (f12 \cdot p(F12\text{'|'}\overline{K1}\,K2\,K3\,K4)$
> $+ (1 - f12) \cdot (1 - p(F12\text{'|'}\overline{K1}\,K2\,K3\,K4))) \cdot (f13 \cdot p(F13\text{'|'}\overline{K1}\,K2\,K3\,K4) + (1 - f13) \cdot (1 - p(F13\text{'|'}$
> $\overline{K1}\,K2\,K3\,K4))) \cdot (f14 \cdot p(F14\text{'|'}\overline{K1}\,K2\,K3\,K4) + (1 - f14) \cdot (1 - p(F14\text{'|'}\overline{K1}\,K2\,K3\,K4))) \cdot (f15 \cdot p(F15\text{'|'}$
> $\overline{K1}\,K2\,K3\,K4) + (1 - f15) \cdot (1 - p(F15\text{'|'}\overline{K1}\,K2\,K3\,K4))) \cdot (f16 \cdot p(F16\text{'|'}\overline{K1}\,K2\,K3\,K4) + (1 - f16) \cdot (1$
> $- p(F16\text{'|'}\overline{K1}\,K2\,K3\,K4)));$

$$a10 := 0.043 \tag{17}$$

> $p(F11\text{'|'}\overline{K1}\,K2\,K3\,\overline{K4}) := 1 - (1 - p(F11\text{'|'}\underline{K2}\text{'~'})) \cdot (1 - \underline{p}(F11\text{'|'}K3\text{'~'}));$

$$p(F11 \text{ '|' } \overline{K1}\,K2\,K3\,\overline{K4}) := 0.6 \tag{18}$$

>

> $p(F12\text{'|'}\overline{K1}\,K2\,K3\,\overline{K4}) := 1 - (1 - p(F12\text{'|'}\underline{K2}\text{'~'})) \cdot (1 - \underline{p}(F12\text{'|'}K3\text{'~'}));$

$$p(F12 \text{ '|' } \overline{K1}\,K2\,K3\,\overline{K4}) := 0.65 \tag{19}$$

>

$$> p\left(F13\urcorner\overline{K1}\,K2\,K3\,\overline{K4}\right) := 1 - \left(1 - p\left(F13\urcorner K2\urcorner\tilde{}\,\right)\right)\cdot\left(1 - \underline{p}\left(F13\urcorner K3\urcorner\tilde{}\,\right)\right);$$
$$p\left(F13\ \urcorner\ K1\ K2\ K3\ \overline{K4}\right) := 0.5 \tag{20}$$

$$> p\left(F14\urcorner\overline{K1}\,K2\,K3\,\overline{K4}\right) := 1 - \left(1 - p\left(F14\urcorner K2\urcorner\tilde{}\,\right)\right)\cdot\left(1 - \underline{p}\left(F14\urcorner K3\urcorner\tilde{}\,\right)\right);$$
$$p\left(F14\ \urcorner\ K1\ K2\ K3\ \overline{K4}\right) := 0.2 \tag{21}$$

$$> p\left(F15\urcorner\overline{K1}\,K2\,K3\,\overline{K4}\right) := 1 - \left(1 - p\left(F15\urcorner K2\urcorner\tilde{}\,\right)\right)\cdot\left(1 - \underline{p}\left(F15\urcorner K3\urcorner\tilde{}\,\right)\right);$$
$$p\left(F15\ \urcorner\ K1\ K2\ K3\ \overline{K4}\right) := 0.5 \tag{22}$$

$$> p\left(F16\urcorner\overline{K1}\,K2\,K3\,\overline{K4}\right) := 1 - \left(1 - p\left(F16\urcorner K2\urcorner\tilde{}\,\right)\right)\cdot\left(1 - \underline{p}\left(F16\urcorner K3\urcorner\tilde{}\,\right)\right);$$
$$p\left(F16\ \urcorner\ K1\ K2\ K3\ \overline{K4}\right) := 0.64 \tag{23}$$

$$> a11 := \left(f11\cdot p\left(F11\urcorner\overline{K1}\,K2\,K3\,\overline{K4}\right) + \left(1 - f11\right)\cdot\left(1 - p\left(F11\urcorner\overline{K1}\,K2\,K3\,\overline{K4}\right)\right)\right)\cdot\left(f12\cdot p\left(F12\urcorner\overline{K1}\,K2\,K3\,\overline{K4}\right)\right.$$
$$+ \left(1 - f12\right)\cdot\left(1 - p\left(F12\urcorner\overline{K1}\,K2\,K3\,\overline{K4}\right)\right)\cdot\left(f13\cdot p\left(F13\urcorner\overline{K1}\,K2\,K3\,\overline{K4}\right) + \left(1 - f13\right)\cdot\left(1 - p\left(F13\urcorner\right.\right.$$
$$\overline{K1}\,K2\,K3\,\overline{K4}\right)\right)\cdot\left(f14\cdot p\left(F14\urcorner\overline{K1}\,K2\,K3\,\overline{K4}\right) + \left(1 - f14\right)\cdot\left(1 - p\left(F14\urcorner\overline{K1}\,K2\,K3\,\overline{K4}\right)\right)\right)\cdot\left(f15\cdot p\left(F15\urcorner\right.\right.$$
$$\overline{K1}\,K2\,K3\,\overline{K4}\right) + \left(1 - f15\right)\cdot\left(1 - p\left(F15\urcorner\overline{K1}\,K2\,K3\,\overline{K4}\right)\right)\cdot\left(f16\cdot p\left(F16\urcorner\overline{K1}\,K2\,K3\,\overline{K4}\right) + \left(1 - f16\right)\cdot\left(1\right.$$
$$\left.- p\left(F16\urcorner\overline{K1}\,K2\,K3\,\overline{K4}\right)\right)\right);$$
$$a11 := 0.0072 \tag{24}$$

$$> p\left(F11\urcorner\overline{K1}\,K2\,\overline{K3}\,K4\right) := 1 - \left(1 - p\left(F11\urcorner K2\urcorner\tilde{}\,\right)\right)\cdot\left(1 - p\left(F11\urcorner K4\urcorner\tilde{}\,\right)\right);$$
$$p\left(F11\ \urcorner\ K1\ K2\ K3\ K4\right) := 0.80 \tag{25}$$

$$> p\left(F12\urcorner\overline{K1}\,K2\,\overline{K3}\,K4\right) := 1 - \left(1 - p\left(F12\urcorner K2\urcorner\tilde{}\,\right)\right)\cdot\left(1 - p\left(F12\urcorner K4\urcorner\tilde{}\,\right)\right);$$
$$p\left(F12\ \urcorner\ K1\ K2\ K3\ K4\right) := 0.65 \tag{26}$$

$$> p\left(F13\urcorner\overline{K1}\,K2\,\overline{K3}\,K4\right) := 1 - \left(1 - p\left(F13\urcorner K2\urcorner\tilde{}\,\right)\right)\cdot\left(1 - p\left(F13\urcorner K4\urcorner\tilde{}\,\right)\right);$$
$$p\left(F13\ \urcorner\ K1\ K2\ K3\ K4\right) := 0.65 \tag{27}$$

$$> p\left(F14\urcorner\overline{K1}\,K2\,\overline{K3}\,K4\right) := 1 - \left(1 - p\left(F14\urcorner K2\urcorner\tilde{}\,\right)\right)\cdot\left(1 - p\left(F14\urcorner K4\urcorner\tilde{}\,\right)\right);$$
$$p\left(F14\ \urcorner\ K1\ K2\ K3\ K4\right) := 0.5 \tag{28}$$

$$> p\left(F15\urcorner\overline{K1}\,K2\,\overline{K3}\,K4\right) := 1 - \left(1 - p\left(F15\urcorner K2\urcorner\tilde{}\,\right)\right)\cdot\left(1 - p\left(F15\urcorner K4\urcorner\tilde{}\,\right)\right);$$
$$p\left(F15\ \urcorner\ K1\ K2\ K3\ K4\right) := 0 \tag{29}$$

$$> p\left(F16\urcorner\overline{K1}\,K2\,\overline{K3}\,K4\right) := 1 - \left(1 - p\left(F16\urcorner K2\urcorner\tilde{}\,\right)\right)\cdot\left(1 - p\left(F16\urcorner K4\urcorner\tilde{}\,\right)\right);$$
$$p\left(F16\ \urcorner\ K1\ K2\ K3\ K4\right) := 0.4 \tag{30}$$

$$> a12 := \left(f11\cdot p\left(F11\urcorner\overline{K1}\,K2\,\overline{K3}\,K4\right) + \left(1 - f11\right)\cdot\left(1 - p\left(F11\urcorner\overline{K1}\,K2\,\overline{K3}\,K4\right)\right)\right)\cdot\left(f12\cdot p\left(F12\urcorner\overline{K1}\,K2\,\overline{K3}\,K4\right)\right.$$
$$+ \left(1 - f12\right)\cdot\left(1 - p\left(F12\urcorner\overline{K1}\,K2\,\overline{K3}\,K4\right)\right)\cdot\left(f13\cdot p\left(F13\urcorner\overline{K1}\,K2\,\overline{K3}\,K4\right) + \left(1 - f13\right)\cdot\left(1 - p\left(F13\urcorner\overline{K1}\,K2\right.\right.$$
$$\overline{K3}\,K4\right)\right)\cdot\left(f14\cdot p\left(F14\urcorner\overline{K1}\,K2\,\overline{K3}\,K4\right) + \left(1 - f14\right)\cdot\left(1 - p\left(F14\urcorner\overline{K1}\,K2\,\overline{K3}\,K4\right)\right)\right)\cdot\left(f15\cdot p\left(F15\urcorner\overline{K1}\,K2\right.\right.$$
$$\overline{K3}\,K4\right) + \left(1 - f15\right)\cdot\left(1 - p\left(F15\urcorner\overline{K1}\,K2\,\overline{K3}\,K4\right)\right)\cdot\left(f16\cdot p\left(F16\urcorner\overline{K1}\,K2\,\overline{K3}\,K4\right) + \left(1 - f16\right)\cdot\left(1 - p\left(F16\urcorner\right.\right.$$
$$\overline{K1}\,K2\,\overline{K3}\,K4\right)\right);$$
$$a12 := 0.10 \tag{31}$$

$$> p\left(F11\urcorner\overline{K1}\,K2\,\overline{K3}\,\overline{K4}\right) := p\left(F11\urcorner K2\urcorner\tilde{}\,\right);$$
$$p\left(F11\ \urcorner\ K1\ K2\ \overline{K3}\ \overline{K4}\right) := 0.6 \tag{32}$$

$$> p\left(F12\urcorner\overline{K1}\,K2\,\overline{K3}\,\overline{K4}\right) := p\left(F12\urcorner K2\urcorner\tilde{}\,\right);$$
$$\tag{33}$$

$$p\left(F12 \ \middle| \ \overline{K1}\ K2\ \overline{K3}\ \overline{K4}\right) := 0.5 \tag{33}$$

$$> p\left(F13\middle|\overline{K1}\ K2\overline{K3}K4\right) := p\left(F13\middle|K2^{\cdot}{\sim}^{\cdot}\right);$$
$$p\left(F13 \ \middle| \ \overline{K1}\ K2\ \overline{K3}\ \overline{K4}\right) := 0.5 \tag{34}$$

$$> p\left(F14\middle|\overline{K1}\ K2\overline{K3}\ \overline{K4}\right) := p\left(F14\middle|K2^{\cdot}{\sim}^{\cdot}\right);$$
$$p\left(F14 \ \middle| \ \overline{K1}\ K2\ \overline{K3}\ \overline{K4}\right) := 0 \tag{35}$$

$$> p\left(F15\middle|\overline{K1}\ K2\overline{K3}\overline{K4}\right) := p\left(F15\middle|K2^{\cdot}{\sim}^{\cdot}\right);$$
$$p\left(F15 \ \middle| \ \overline{K1}\ K2\ \overline{K3}\ \overline{K4}\right) := 0 \tag{36}$$

$$> p\left(F16\middle|\overline{K1}\ K2\overline{K3}K4\right) := p\left(F16\middle|K2^{\cdot}{\sim}^{\cdot}\right);$$
$$p\left(F16 \ \middle| \ \overline{K1}\ K2\ \overline{K3}\ \overline{K4}\right) := 0.4 \tag{37}$$

$$> a13 := \left(f11 \cdot p\left(F11\middle|\overline{K1}\ K2\overline{K3}\overline{K4}\right) + (1 - f11) \cdot \left(1 - p\left(F11\middle|\overline{K1}\ K2\overline{K3}\overline{K4}\right)\right)\right) \cdot \left(f12 \cdot p\left(F12\middle|\overline{K1}\ K2\overline{K3}\overline{K4}\right)\right.$$
$$+ (1 - f12) \cdot \left(1 - p\left(F12\middle|\overline{K1}\ K2\overline{K3}K4\right)\right)\right) \cdot \left(f13 \cdot p\left(F13\middle|\overline{K1}\ K2\overline{K3}K4\right) + (1 - f13) \cdot \left(1 - p\left(F13\middle|\overline{K1}\ K2\right.\right.\right.$$
$$\left.\left.\overline{K3}K4\right)\right)\right) \cdot \left(f14 \cdot p\left(F14\middle|\overline{K1}\ K2\overline{K3}\overline{K4}\right) + (1 - f14) \cdot \left(1 - p\left(F14\middle|\overline{K1}\ K2\overline{K3}\overline{K4}\right)\right)\right) \cdot \left(f15 \cdot p\left(F15\middle|\overline{K1}\ K2\overline{K3}\right.\right.$$
$$\left.\overline{K4}\right) + (1 - f15) \cdot \left(1 - p\left(F15\middle|\overline{K1}\ K2\overline{K3}K4\right)\right)\right) \cdot \left(f16 \cdot p\left(F16\middle|\overline{K1}\ K2\overline{K3}K4\right) + (1 - f16) \cdot \left(1 - p\left(F16\middle|\right.\right.\right.$$
$$\left.\left.\left.\overline{K1}\ K2\overline{K3}K4\right)\right)\right);$$
$$a13 := 0. \tag{38}$$

$$> p\left(F11\middle|\overline{K1}\ \overline{K2}K3K4\right) := 1 - \left(1 - p\left(F11\middle|K3^{\cdot}{\sim}^{\cdot}\right)\right) \cdot \left(1 - p\left(F11\middle|K4{\sim}^{\cdot}\right)\right);$$
$$p\left(F11 \ \middle| \ \overline{K1}\ \overline{K2}\ K3\ K4\right) := 0.5 \tag{39}$$

$$>$$
$$> p\left(F12\middle|\overline{K1}\ \overline{K2}K3K4\right) := 1 - \left(1 - p\left(F12\middle|K3^{\cdot}{\sim}^{\cdot}\right)\right) \cdot \left(1 - p\left(F12\middle|K4{\sim}^{\cdot}\right)\right);$$
$$p\left(F12 \ \middle| \ \overline{K1}\ \overline{K2}\ K3\ K4\right) := 0.51 \tag{40}$$

$$>$$
$$> p\left(F13\middle|\overline{K1}\ \overline{K2}K3K4\right) := 1 - \left(1 - p\left(F13\middle|K3^{\cdot}{\sim}^{\cdot}\right)\right) \cdot \left(1 - p\left(F13\middle|K4{\sim}^{\cdot}\right)\right);$$
$$p\left(F13 \ \middle| \ \overline{K1}\ \overline{K2}\ K3\ K4\right) := 0.3 \tag{41}$$

$$>$$
$$> p\left(F14\middle|\overline{K1}\ \overline{K2}K3K4\right) := 1 - \left(1 - p\left(F14\middle|K3^{\cdot}{\sim}^{\cdot}\right)\right) \cdot \left(1 - p\left(F14\middle|K4{\sim}^{\cdot}\right)\right);$$
$$p\left(F14 \ \middle| \ \overline{K1}\ \overline{K2}\ K3\ K4\right) := 0.60 \tag{42}$$

$$>$$
$$> p\left(F15\middle|\overline{K1}\ \overline{K2}K3K4\right) := 1 - \left(1 - p\left(F15\middle|K3^{\cdot}{\sim}^{\cdot}\right)\right) \cdot \left(1 - p\left(F15\middle|K4{\sim}^{\cdot}\right)\right);$$
$$p\left(F15 \ \middle| \ \overline{K1}\ \overline{K2}\ K3\ K4\right) := 0.5 \tag{43}$$

$$>$$
$$> p\left(F16\middle|\overline{K1}\ \overline{K2}K3K4\right) := 1 - \left(1 - p\left(F16\middle|K3^{\cdot}{\sim}^{\cdot}\right)\right) \cdot \left(1 - p\left(F16\middle|K4{\sim}^{\cdot}\right)\right);$$
$$p\left(F16 \ \middle| \ \overline{K1}\ \overline{K2}\ K3\ K4\right) := 0.4 \tag{44}$$

$$>$$
$$> a14 := \left(f11 \cdot p\left(F11\middle|\overline{K1}\ \overline{K2}K3K4\right) + (1 - f11) \cdot \left(1 - p\left(F11\middle|\overline{K1}\ \overline{K2}K3K4\right)\right)\right) \cdot \left(f12 \cdot p\left(F12\middle|\overline{K1}\ \overline{K2}K3K4\right)\right.$$
$$+ (1 - f12) \cdot \left(1 - p\left(F12\middle|\overline{K1}\ \overline{K2}K3K4\right)\right)\right) \cdot \left(f13 \cdot p\left(F13\middle|\overline{K1}\ \overline{K2}K3K4\right) + (1 - f13) \cdot \left(1 - p\left(F13\middle|\overline{K1}\right.\right.\right.$$
$$\left.\left.\overline{K2}K3K4\right)\right)\right) \cdot \left(f14 \cdot p\left(F14\middle|\overline{K1}\ \overline{K2}K3K4\right) + (1 - f14) \cdot \left(1 - p\left(F14\middle|\overline{K1}\ \overline{K2}K3K4\right)\right)\right) \cdot \left(f15 \cdot p\left(F15\middle|\overline{K1}\right.\right.$$
$$\left.\overline{K2}K3K4\right) + (1 - f15) \cdot \left(1 - p\left(F15\middle|\overline{K1}\ \overline{K2}K3K4\right)\right)\right) \cdot \left(f16 \cdot p\left(F16\middle|\overline{K1}\ \overline{K2}K3K4\right) + (1 - f16) \cdot \left(1\right.\right.$$
$$\left.\left.- p\left(F16\middle|\overline{K1}\ \overline{K2}K3K4\right)\right)\right);$$
$$a14 := 0.014 \tag{45}$$

$$> p\left(F11\middle|\overline{K1}\ \overline{K2}K3\overline{K4}\right) := p\left(F11\middle|K3^{\cdot}{\sim}^{\cdot}\right);$$
$$p\left(F11 \ \middle| \ \overline{K1}\ \overline{K2}\ K3\ \overline{K4}\right) := 0 \tag{46}$$

$$> p\left(F12\middle|\overline{K1}\ \overline{K2}K3\overline{K4}\right) := p\left(F12\middle|K3^{\cdot}{\sim}^{\cdot}\right);$$
$$p\left(F12 \ \middle| \ \overline{K1}\ \overline{K2}\ K3\ \overline{K4}\right) := 0.3 \tag{47}$$

$$> p\left(F13\middle|\overline{K1}\ \overline{K2}K3\overline{K4}\right) := p\left(F13\middle|K3^{\cdot}{\sim}^{\cdot}\right);$$
$$p\left(F13 \ \middle| \ \overline{K1}\ \overline{K2}\ K3\ \overline{K4}\right) := 0 \tag{48}$$

$$p\left(F14\text{`|`}\overline{K1}\ \overline{K2}\ K3\ \overline{K4}\right) := p\left(F14\text{`|`}K3\text{`}\sim\text{`}\right);$$
$$p\left(F14\text{`|`}\ \overline{K1}\ \overline{K2}\ K3\ \overline{K4}\right) := 0.2 \tag{49}$$

$$p\left(F15\text{`|`}\overline{K1}\ \overline{K2}\ K3\ \overline{K4}\right) := p\left(F15\text{`|`}K3\text{`}\sim\text{`}\right);$$
$$p\left(F15\text{`|`}\ \overline{K1}\ \overline{K2}\ K3\ \overline{K4}\right) := 0.5 \tag{50}$$

$$p\left(F16\text{`|`}\overline{K1}\ \overline{K2}\ K3\ \overline{K4}\right) := p\left(F16\text{`|`}K3\text{`}\sim\text{`}\right);$$
$$p\left(F16\text{`|`}\ \overline{K1}\ \overline{K2}\ K3\ \overline{K4}\right) := 0.4 \tag{51}$$

$$a15 := \left(f11 \cdot p\left(F11\text{`|`}\overline{K1}\ \overline{K2}\ K3\ \overline{K4}\right) + \left(1 - f11\right) \cdot \left(1 - p\left(F11\text{`|`}\overline{K1}\ \overline{K2}\ K3\ \overline{K4}\right)\right)\right) \cdot \left(f12 \cdot p\left(F12\text{`|`}\overline{K1}\ \overline{K2}\ K3\ \overline{K4}\right)\right.$$
$$+ \left(1 - f12\right) \cdot \left(1 - p\left(F12\text{`|`}\overline{K1}\ \overline{K2}\ K3\ \overline{K4}\right)\right)\right) \cdot \left(f13 \cdot p\left(F13\text{`|`}\overline{K1}\ \overline{K2}\ K3\ \overline{K4}\right) + \left(1 - f13\right) \cdot \left(1 - p\left(F13\text{`|`}\overline{K1}\ \overline{K2}\ K3\ \overline{K4}\right)\right)\right) \cdot \left(f14 \cdot p\left(F14\text{`|`}\overline{K1}\ \overline{K2}\ K3\ \overline{K4}\right) + \left(1 - f14\right) \cdot \left(1 - p\left(F14\text{`|`}\overline{K1}\ \overline{K2}\ K3\ \overline{K4}\right)\right)\right) \cdot \left(f15 \cdot p\left(F15\text{`|`}\overline{K1}\ \overline{K2}\ K3\ \overline{K4}\right) + \left(1 - f15\right) \cdot \left(1 - p\left(F15\text{`|`}\overline{K1}\ \overline{K2}\ K3\ \overline{K4}\right)\right)\right) \cdot \left(f16 \cdot p\left(F16\text{`|`}\overline{K1}\ \overline{K2}\ K3\ \overline{K4}\right) + \left(1 - f16\right) \cdot \left(1 - p\left(F16\text{`|`}\overline{K1}\ \overline{K2}\ K3\ \overline{K4}\right)\right)\right);$$
$$a15 := 0. \tag{52}$$

$$p\left(F11\text{`|`}\overline{K1}\ \overline{K2}\ \overline{K3}\ K4\right) := p\left(F11\text{`|`}K4\text{`}\sim\text{`}\right);$$
$$p\left(F11\text{`|`}\ \overline{K1}\ \overline{K2}\ \overline{K3}\ K4\right) := 0.5 \tag{53}$$

$$p\left(F12\text{`|`}\overline{K1}\ \overline{K2}\ \overline{K3}\ K4\right) := p\left(F12\text{`|`}K4\text{`}\sim\text{`}\right);$$
$$p\left(F12\text{`|`}\ \overline{K1}\ \overline{K2}\ \overline{K3}\ K4\right) := 0.3 \tag{54}$$

$$p\left(F13\text{`|`}\overline{K1}\ \overline{K2}\ \overline{K3}\ K4\right) := p\left(F13\text{`|`}K4\text{`}\sim\text{`}\right);$$
$$p\left(F13\text{`|`}\ \overline{K1}\ \overline{K2}\ \overline{K3}\ K4\right) := 0.3 \tag{55}$$

$$p\left(F14\text{`|`}\overline{K1}\ \overline{K2}\ \overline{K3}\ K4\right) := p\left(F14\text{`|`}K4\text{`}\sim\text{`}\right);$$
$$p\left(F14\text{`|`}\ \overline{K1}\ \overline{K2}\ \overline{K3}\ K4\right) := 0.5 \tag{56}$$

$$p\left(F15\text{`|`}\overline{K1}\ \overline{K2}\ \overline{K3}\ K4\right) := p\left(F15\text{`|`}K4\text{`}\sim\text{`}\right);$$
$$p\left(F15\text{`|`}\ \overline{K1}\ \overline{K2}\ \overline{K3}\ K4\right) := 0 \tag{57}$$

$$p\left(F16\text{`|`}\overline{K1}\ \overline{K2}\ \overline{K3}\ K4\right) := p\left(F16\text{`|`}K4\text{`}\sim\text{`}\right);$$
$$p\left(F16\text{`|`}\ \overline{K1}\ \overline{K2}\ \overline{K3}\ K4\right) := 0 \tag{58}$$

$$a16 := \left(f11 \cdot p\left(F11\text{`|`}\overline{K1}\ \overline{K2}\ \overline{K3}\ K4\right) + \left(1 - f11\right) \cdot \left(1 - p\left(F11\text{`|`}\overline{K1}\ \overline{K2}\ \overline{K3}\ K4\right)\right)\right) \cdot \left(f12 \cdot p\left(F12\text{`|`}\overline{K1}\ \overline{K2}\ \overline{K3}\ K4\right)\right.$$
$$+ \left(1 - f12\right) \cdot \left(1 - p\left(F12\text{`|`}\overline{K1}\ \overline{K2}\ \overline{K3}\ K4\right)\right)\right) \cdot \left(f13 \cdot p\left(F13\text{`|`}\overline{K1}\ \overline{K2}\ \overline{K3}\ K4\right) + \left(1 - f13\right) \cdot \left(1 - p\left(F13\text{`|`}\overline{K1}\ \overline{K2}\ \overline{K3}\ K4\right)\right)\right) \cdot \left(f14 \cdot p\left(F14\text{`|`}\overline{K1}\ \overline{K2}\ \overline{K3}\ K4\right) + \left(1 - f14\right) \cdot \left(1 - p\left(F14\text{`|`}\overline{K1}\ \overline{K2}\ \overline{K3}\ K4\right)\right)\right) \cdot \left(f15 \cdot p\left(F15\text{`|`}\overline{K1}\ \overline{K2}\ \overline{K3}\ K4\right) + \left(1 - f15\right) \cdot \left(1 - p\left(F15\text{`|`}\overline{K1}\ \overline{K2}\ \overline{K3}\ K4\right)\right)\right) \cdot \left(f16 \cdot p\left(F16\text{`|`}\overline{K1}\ \overline{K2}\ \overline{K3}\ K4\right) + \left(1 - f16\right) \cdot \left(1 - p\left(F16\text{`|`}\overline{K1}\ \overline{K2}\ \overline{K3}\ K4\right)\right)\right);$$
$$a16 := 0.022 \tag{59}$$

$$a17 := 0;$$
$$a17 := 0 \tag{60}$$

$$p\left(F11\text{`|`}K1\,K2\,K3\,K4\right) := 1 - \left(1 - p\left(F11\text{`|`}K1\text{`}\sim\text{`}\right)\right) \cdot \left(1 - p\left(F11\text{`|`}K2\text{`}\sim\text{`}\right)\right) \cdot \left(1 - p\left(F11\text{`|`}K3\text{`}\sim\text{`}\right)\right) \cdot \left(1 - p\left(F11\text{`|`}K4\text{`}\sim\text{`}\right)\right);$$
$$p\left(F11\text{`|`}\ K1\,K2\,K3\,K4\right) := 0.92 \tag{61}$$

$$p\left(F12\text{`|`}K1\,K2\,K3\,K4\right) := 1 - \left(1 - p\left(F12\text{`|`}K1\text{`}\sim\text{`}\right)\right) \cdot \left(1 - p\left(F12\text{`|`}K2\text{`}\sim\text{`}\right)\right) \cdot \left(1 - p\left(F12\text{`|`}K3\text{`}\sim\text{`}\right)\right) \cdot \left(1 - p\left(F12\text{`|`}K4\text{`}\sim\text{`}\right)\right);$$
$$p\left(F12\text{`|`}\ K1\,K2\,K3\,K4\right) := 0.87 \tag{62}$$

$$p\left(F13\text{`|`}K1\,K2\,K3\,K4\right) := 1 - \left(1 - p\left(F13\text{`|`}K1\text{`}\sim\text{`}\right)\right) \cdot \left(1 - p\left(F13\text{`|`}K2\text{`}\sim\text{`}\right)\right) \cdot \left(1 - p\left(F13\text{`|`}K3\text{`}\sim\text{`}\right)\right) \cdot \left(1 - p\left(F13\text{`|`}K4\text{`}\sim\text{`}\right)\right);$$
$$p\left(F13\text{`|`}\ K1\,K2\,K3\,K4\right) := 0.82 \tag{63}$$

$$p\left(F14\text{`|`}K1\,K2\,K3\,K4\right) := 1 - \left(1 - p\left(F14\text{`|`}K1\text{`}\sim\text{`}\right)\right) \cdot \left(1 - p\left(F14\text{`|`}K2\text{`}\sim\text{`}\right)\right) \cdot \left(1 - p\left(F14\text{`|`}K3\text{`}\sim\text{`}\right)\right) \cdot \left(1 - p\left(F14\text{`|`}K4\text{`}\sim\text{`}\right)\right);$$
$$p\left(F14\text{`|`}\ K1\,K2\,K3\,K4\right) := 0.84 \tag{64}$$

> $p(F15\backprime|\backprime K1\,K2\,K3\,K4) := 1 - (1 - p(F15\backprime|\backprime K1\backprime{\sim}\backprime)) \cdot (1 - p(F15\backprime|\backprime K2\backprime{\sim}\backprime)) \cdot (1 - p(F15\backprime|\backprime K3\backprime{\sim}\backprime)) \cdot (1 - p(F15\backprime|\backprime K4\backprime{\sim}\backprime));$

$$p(F15\ \backprime|\backprime\ K1\ K2\ K3\ K4) := 0.95 \tag{65}$$

> $p(F16\backprime|\backprime K1\,K2\,K3\,K4) := 1 - (1 - p(F16\backprime|\backprime K1\backprime{\sim}\backprime)) \cdot (1 - p(F16\backprime|\backprime K2\backprime{\sim}\backprime)) \cdot (1 - p(F16\backprime|\backprime K3\backprime{\sim}\backprime)) \cdot (1 - p(F16\backprime|\backprime K4\backprime{\sim}\backprime));$

$$p(F16\ \backprime|\backprime\ K1\ K2\ K3\ K4) := 0.89 \tag{66}$$

> $b10 := (fl1 \cdot p(F11\backprime|\backprime K1\,K2\,K3\,K4) + (1 - fl1) \cdot (1 - p(F11\backprime|\backprime K1\,K2\,K3\,K4))) \cdot (fl2 \cdot p(F12\backprime|\backprime K1\,K2\,K3\,K4) + (1 - fl2) \cdot (1 - p(F12\backprime|\backprime K1\,K2\,K3\,K4))) \cdot (fl3 \cdot p(F13\backprime|\backprime K1\,K2\,K3\,K4) + (1 - fl3) \cdot (1 - p(F13\backprime|\backprime K1\,K2\,K3\,K4))) \cdot (fl4 \cdot p(F14\backprime|\backprime K1\,K2\,K3\,K4) + (1 - fl4) \cdot (1 - p(F14\backprime|\backprime K1\,K2\,K3\,K4))) \cdot (fl5 \cdot p(F15\backprime|\backprime K1\,K2\,K3\,K4) + (1 - fl5) \cdot (1 - p(F15\backprime|\backprime K1\,K2\,K3\,K4))) \cdot (fl6 \cdot p(F16\backprime|\backprime K1\,K2\,K3\,K4) + (1 - fl6) \cdot (1 - p(F16\backprime|\backprime K1\,K2\,K3\,K4)));$

$$b10 := 0.0031 \tag{67}$$

> $p(F11\backprime|\backprime K1\,K2\,K3\,\overline{K4}) := 1 - (1 - p(F11\backprime|\backprime K1\backprime{\sim}\backprime)) \cdot (1 - p(F11\backprime|\backprime K2\backprime{\sim}\backprime)) \cdot (1 - p(F11\backprime|\backprime K3\backprime{\sim}\backprime));$

$$p(F11\ \backprime|\backprime\ K1\ K2\ K3\ \overline{K4}) := 0.84 \tag{68}$$

> $p(F12\backprime|\backprime K1\,K2\,K3\,\overline{K4}) := 1 - (1 - p(F12\backprime|\backprime K1\backprime{\sim}\backprime)) \cdot (1 - p(F12\backprime|\backprime K2\backprime{\sim}\backprime)) \cdot (1 - p(F12\backprime|\backprime K3\backprime{\sim}\backprime));$

$$p(F12\ \backprime|\backprime\ K1\ K2\ K3\ \overline{K4}) := 0.82 \tag{69}$$

> $p(F13\backprime|\backprime K1\,K2\,K3\,\overline{K4}) := 1 - (1 - p(F13\backprime|\backprime K1\backprime{\sim}\backprime)) \cdot (1 - p(F13\backprime|\backprime K2\backprime{\sim}\backprime)) \cdot (1 - p(F13\backprime|\backprime K3\backprime{\sim}\backprime));$

$$p(F13\ \backprime|\backprime\ K1\ K2\ K3\ \overline{K4}) := 0.75 \tag{70}$$

> $p(F14\backprime|\backprime K1\,K2\,K3\,\overline{K4}) := 1 - (1 - p(F14\backprime|\backprime K1\backprime{\sim}\backprime)) \cdot (1 - p(F14\backprime|\backprime K2\backprime{\sim}\backprime)) \cdot (1 - p(F14\backprime|\backprime K3\backprime{\sim}\backprime));$

$$p(F14\ \backprime|\backprime\ K1\ K2\ K3\ \overline{K4}) := 0.68 \tag{71}$$

> $p(F15\backprime|\backprime K1\,K2\,K3\,\overline{K4}) := 1 - (1 - p(F15\backprime|\backprime K1\backprime{\sim}\backprime)) \cdot (1 - p(F15\backprime|\backprime K2\backprime{\sim}\backprime)) \cdot (1 - p(F15\backprime|\backprime K3\backprime{\sim}\backprime));$

$$p(F15\ \backprime|\backprime\ K1\ K2\ K3\ \overline{K4}) := 0.95 \tag{72}$$

> $p(F16\backprime|\backprime K1\,K2\,K3\,\overline{K4}) := 1 - (1 - p(F16\backprime|\backprime K1\backprime{\sim}\backprime)) \cdot (1 - p(F16\backprime|\backprime K2\backprime{\sim}\backprime)) \cdot (1 - p(F16\backprime|\backprime K3\backprime{\sim}\backprime));$

$$p(F16\ \backprime|\backprime\ K1\ K2\ K3\ \overline{K4}) := 0.89 \tag{73}$$

> $b11 := (fl1 \cdot p(F11\backprime|\backprime K1\,K2\,K3\,\overline{K4}) + (1 - fl1) \cdot (1 - p(F11\backprime|\backprime K1\,K2\,K3\,\overline{K4}))) \cdot (fl2 \cdot p(F12\backprime|\backprime K1\,K2\,K3\,\overline{K4}) + (1 - fl2) \cdot (1 - p(F12\backprime|\backprime K1\,K2\,K3\,\overline{K4}))) \cdot (fl3 \cdot p(F13\backprime|\backprime K1\,K2\,K3\,\overline{K4}) + (1 - fl3) \cdot (1 - p(F13\backprime|\backprime K1\,K2\,K3\,\overline{K4}))) \cdot (fl4 \cdot p(F14\backprime|\backprime K1\,K2\,K3\,\overline{K4}) + (1 - fl4) \cdot (1 - p(F14\backprime|\backprime K1\,K2\,K3\,\overline{K4}))) \cdot (fl5 \cdot p(F15\backprime|\backprime K1\,K2\,K3\,\overline{K4}) + (1 - fl5) \cdot (1 - p(F15\backprime|\backprime K1\,K2\,K3\,\overline{K4}))) \cdot (fl6 \cdot p(F16\backprime|\backprime K1\,K2\,K3\,\overline{K4}) + (1 - fl6) \cdot (1 - p(F16\backprime|\backprime K1\,K2\,K3\,\overline{K4})));$

$$b11 := 0.0020 \tag{74}$$

> $p(F11\backprime|\backprime K1\,K2\,\overline{K3}\,K4) := 1 - (1 - p(F11\backprime|\backprime K1\backprime{\sim}\backprime)) \cdot (1 - p(F11\backprime|\backprime K2\backprime{\sim}\backprime)) \cdot (1 - p(F11\backprime|\backprime K4\backprime{\sim}\backprime));$

$$p(F11\ \backprime|\backprime\ K1\ K2\ \overline{K3}\ K4) := 0.92 \tag{75}$$

> $p(F12\backprime|\backprime K1\,K2\,\overline{K3}\,K4) := 1 - (1 - p(F12\backprime|\backprime K1\backprime{\sim}\backprime)) \cdot (1 - p(F12\backprime|\backprime K2\backprime{\sim}\backprime)) \cdot (1 - p(F12\backprime|\backprime K4\backprime{\sim}\backprime));$

$$p(F12\ \backprime|\backprime\ K1\ K2\ \overline{K3}\ K4) := 0.82 \tag{76}$$

> $p(F13\backprime|\backprime K1\,K2\,\overline{K3}\,K4) := 1 - (1 - p(F13\backprime|\backprime K1\backprime{\sim}\backprime)) \cdot (1 - p(F13\backprime|\backprime K2\backprime{\sim}\backprime)) \cdot (1 - p(F13\backprime|\backprime K4\backprime{\sim}\backprime));$

$$p(F13\ \backprime|\backprime\ K1\ K2\ \overline{K3}\ K4) := 0.82 \tag{77}$$

> $p(F14`|`K1\,K2\,\overline{K3}\,K4) := 1 - (1 - p(F14`|`K1`\sim`)) \cdot (1 - p(F14`|`K2`\sim`)) \cdot (1 - p(F14`|`K4`\sim`));$
$$p(F14\,`|`\,K1\,K2\,K3\,K4) := 0.80 \qquad (78)$$

> $p(F15`|`K1\,K2\,\overline{K3}\,K4) := 1 - (1 - p(F15`|`K1`\sim`)) \cdot (1 - p(F15`|`K2`\sim`)) \cdot (1 - p(F15`|`K4`\sim`));$
$$p(F15\,`|`\,K1\,K2\,K3\,K4) := 0.9 \qquad (79)$$

> $p(F16`|`K1\,K2\,\overline{K3}\,K4) := 1 - (1 - p(F16`|`K1`\sim`)) \cdot (1 - p(F16`|`K2`\sim`)) \cdot (1 - p(F16`|`K4`\sim`));$
$$p(F16\,`|`\,K1\,K2\,K3\,K4) := 0.82 \qquad (80)$$

> $b12 := (f11 \cdot p(F11`|`K1\,K2\,\overline{K3}\,K4) + (1 - f11) \cdot (1 - p(F11`|`K1\,K2\,\overline{K3}\,K4))) \cdot (f12 \cdot p(F12`|`K1\,K2\,\overline{K3}\,K4) + (1 - f12) \cdot (1 - p(F12`|`K1\,K2\,\overline{K3}\,K4))) \cdot (f13 \cdot p(F13`|`K1\,K2\,\overline{K3}\,K4) + (1 - f13) \cdot (1 - p(F13`|`K1\,K2\,\overline{K3}\,K4))) \cdot (f14 \cdot p(F14`|`K1\,K2\,\overline{K3}\,K4) + (1 - f14) \cdot (1 - p(F14`|`K1\,K2\,\overline{K3}\,K4))) \cdot (f15 \cdot p(F15`|`K1\,K2\,\overline{K3}\,K4) + (1 - f15) \cdot (1 - p(F15`|`K1\,K2\,\overline{K3}\,K4))) \cdot (f16 \cdot p(F16`|`K1\,K2\,\overline{K3}\,K4) + (1 - f16) \cdot (1 - p(F16`|`K1\,K2\,\overline{K3}\,K4)));$
$$b12 := 0.0090 \qquad (81)$$

> $p(F11`|`K1\,K2\,\overline{K3}\,\overline{K4}) := 1 - (1 - p(F11`|`K1`\sim`)) \cdot (1 - p(F11`|`K2`\sim`));$
$$p(F11\,`|`\,K1\,K2\,K3\,\overline{K4}) := 0.84 \qquad (82)$$

> $p(F12`|`K1\,K2\,\overline{K3}\,\overline{K4}) := 1 - (1 - p(F12`|`K1`\sim`)) \cdot (1 - p(F12`|`K2`\sim`));$
$$p(F12\,`|`\,K1\,K2\,K3\,\overline{K4}) := 0.75 \qquad (83)$$

> $p(F13`|`K1\,K2\,\overline{K3}\,\overline{K4}) := 1 - (1 - p(F13`|`K1`\sim`)) \cdot (1 - p(F13`|`K2`\sim`));$
$$p(F13\,`|`\,K1\,K2\,K3\,\overline{K4}) := 0.75 \qquad (84)$$

> $p(F14`|`K1\,K2\,\overline{K3}\,\overline{K4}) := 1 - (1 - p(F14`|`K1`\sim`)) \cdot (1 - p(F14`|`K2`\sim`));$
$$p(F14\,`|`\,K1\,K2\,K3\,\overline{K4}) := 0.6 \qquad (85)$$

> $p(F15`|`K1\,K2\,\overline{K3}\,\overline{K4}) := 1 - (1 - p(F15`|`K1`\sim`)) \cdot (1 - p(F15`|`K2`\sim`));$
$$p(F15\,`|`\,K1\,K2\,K3\,\overline{K4}) := 0.9 \qquad (86)$$

> $p(F16`|`K1\,K2\,\overline{K3}\,\overline{K4}) := 1 - (1 - p(F16`|`K1`\sim`)) \cdot (1 - p(F16`|`K2`\sim`));$
$$p(F16\,`|`\,K1\,K2\,K3\,\overline{K4}) := 0.82 \qquad (87)$$

> $b13 := (f11 \cdot p(F11`|`K1\,K2\,\overline{K3}\,\overline{K4}) + (1 - f11) \cdot (1 - p(F11`|`K1\,K2\,\overline{K3}\,\overline{K4}))) \cdot (f12 \cdot p(F12`|`K1\,K2\,\overline{K3}\,\overline{K4}) + (1 - f12) \cdot (1 - p(F12`|`K1\,K2\,\overline{K3}\,\overline{K4}))) \cdot (f13 \cdot p(F13`|`K1\,K2\,\overline{K3}\,\overline{K4}) + (1 - f13) \cdot (1 - p(F13`|`K1\,K2\,\overline{K3}\,\overline{K4}))) \cdot (f14 \cdot p(F14`|`K1\,K2\,\overline{K3}\,\overline{K4}) + (1 - f14) \cdot (1 - p(F14`|`K1\,K2\,\overline{K3}\,\overline{K4}))) \cdot (f15 \cdot p(F15`|`K1\,K2\,\overline{K3}\,\overline{K4}) + (1 - f15) \cdot (1 - p(F15`|`K1\,K2\,\overline{K3}\,\overline{K4}))) \cdot (f16 \cdot p(F16`|`K1\,K2\,\overline{K3}\,\overline{K4}) + (1 - f16) \cdot (1 - p(F16`|`K1\,K2\,\overline{K3}\,\overline{K4})));$
$$b13 := 0.0050 \qquad (88)$$

> $p(F11`|`K1\,\overline{K2}\,K3\,K4) := 1 - (1 - p(F11`|`K1`\sim`)) \cdot (1 - p(F11`|`K3`\sim`)) \cdot (1 - p(F11`|`K4`\sim`));$
$$p(F11\,`|`\,K1\,K2\,K3\,K4) := 0.80 \qquad (89)$$

> $p(F12`|`K1\,\overline{K2}\,K3\,K4) := 1 - (1 - p(F12`|`K1`\sim`)) \cdot (1 - p(F12`|`K3`\sim`)) \cdot (1 - p(F12`|`K4`\sim`));$
$$p(F12\,`|`\,K1\,K2\,K3\,K4) := 0.76 \qquad (90)$$

> $p(F13`|`K1\,\overline{K2}\,K3\,K4) := 1 - (1 - p(F13`|`K1`\sim`)) \cdot (1 - p(F13`|`K3`\sim`)) \cdot (1 - p(F13`|`K4`\sim`));$
$$p(F13\,`|`\,K1\,K2\,K3\,K4) := 0.65 \qquad (91)$$

> $p(F14`|`K1\,\overline{K2}\,K3\,K4) := 1 - (1 - p(F14`|`K1`\sim`)) \cdot (1 - p(F14`|`K3`\sim`)) \cdot (1 - p(F14`|`K4`\sim`));$
$$p(F14\,`|`\,K1\,K2\,K3\,K4) := 0.84 \qquad (92)$$

> $p(F15`|`K1\,\overline{K2}\,K3\,K4) := 1 - (1 - p(F15`|`K1`\sim`)) \cdot (1 - p(F15`|`K3`\sim`)) \cdot (1 - p(F15`|`K4`\sim`));$
$$(93)$$

$$p(F15 \text{ 'I' } K1 \overline{K2} K3 K4) := 0.95 \tag{93}$$

$$> p(F16\text{'I'}K1\overline{K2}K3K4) := 1 - (1 - p(F16\text{'I'}K1\text{'}\sim\text{'})) \cdot (1 - p(F16\text{'I'}K3\text{'}\sim\text{'})) \cdot (1 - p(F16\text{'I'}K4\text{'}\sim\text{'}));$$
$$p(F16 \text{ 'I' } K1 \ K2 \ K3 \ K4) := 0.82 \tag{94}$$

$$> b14 := (f11 \cdot p(F11\text{'I'}K1\overline{K2}K3K4) + (1 - f11) \cdot (1 - p(F11\text{'I'}K1\overline{K2}K3K4))) \cdot (f12 \cdot p(F12\text{'I'}K1\overline{K2}K3K4) + (1 - f12) \cdot (1 - p(F12\text{'I'}K1\overline{K2}K3K4))) \cdot (f13 \cdot p(F13\text{'I'}K1\overline{K2}K3K4) + (1 - f13) \cdot (1 - p(F13\text{'I'}K1\overline{K2}K3K4))) \cdot (f14 \cdot p(F14\text{'I'}K1\overline{K2}K3K4) + (1 - f14) \cdot (1 - p(F14\text{'I'}K1\overline{K2}K3K4))) \cdot (f15 \cdot p(F15\text{'I'}K1\overline{K2}K3K4) + (1 - f15) \cdot (1 - p(F15\text{'I'}K1\overline{K2}K3K4))) \cdot (f16 \cdot p(F16\text{'I'}K1\overline{K2}K3K4) + (1 - f16) \cdot (1 - p(F16\text{'I'}K1\overline{K2}K3K4)));$$

$$b14 := 0.0031 \tag{95}$$

$$> p(F11\text{'I'}K1\overline{K2}K3\overline{K4}) := 1 - (1 - p(F11\text{'I'}K1\text{'}\sim\text{'})) \cdot (1 - p(F11\text{'I'}K3\text{'}\sim\text{'}));$$
$$p(F11 \text{ 'I' } K1 \ K2 \ K3 \ \overline{K4}) := 0.6 \tag{96}$$

$$> p(F12\text{'I'}K1\overline{K2}K3\overline{K4}) := 1 - (1 - p(F12\text{'I'}K1\text{'}\sim\text{'})) \cdot (1 - p(F12\text{'I'}K3\text{'}\sim\text{'}));$$
$$p(F12 \text{ 'I' } K1 \ K2 \ K3 \ \overline{K4}) := 0.65 \tag{97}$$

$$> p(F13\text{'I'}K1\overline{K2}K3\overline{K4}) := 1 - (1 - p(F13\text{'I'}K1\text{'}\sim\text{'})) \cdot (1 - p(F13\text{'I'}K3\text{'}\sim\text{'}));$$
$$p(F13 \text{ 'I' } K1 \ K2 \ K3 \ \overline{K4}) := 0.5 \tag{98}$$

$$> p(F14\text{'I'}K1\overline{K2}K3\overline{K4}) := 1 - (1 - p(F14\text{'I'}K1\text{'}\sim\text{'})) \cdot (1 - p(F14\text{'I'}K3\text{'}\sim\text{'}));$$
$$p(F14 \text{ 'I' } K1 \ K2 \ K3 \ \overline{K4}) := 0.68 \tag{99}$$

$$> p(F15\text{'I'}K1\overline{K2}K3\overline{K4}) := 1 - (1 - p(F15\text{'I'}K1\text{'}\sim\text{'})) \cdot (1 - p(F15\text{'I'}K3\text{'}\sim\text{'}));$$
$$p(F15 \text{ 'I' } K1 \ K2 \ K3 \ \overline{K4}) := 0.95 \tag{100}$$

$$> p(F16\text{'I'}K1\overline{K2}K3\overline{K4}) := 1 - (1 - p(F16\text{'I'}K1\text{'}\sim\text{'})) \cdot (1 - p(F16\text{'I'}K3\text{'}\sim\text{'}));$$
$$p(F16 \text{ 'I' } K1 \ K2 \ K3 \ \overline{K4}) := 0.82 \tag{101}$$

$$> b15 := (f11 \cdot p(F11\text{'I'}K1\overline{K2}K3\overline{K4}) + (1 - f11) \cdot (1 - p(F11\text{'I'}K1\overline{K2}K3\overline{K4}))) \cdot (f12 \cdot p(F12\text{'I'}K1\overline{K2}K3\overline{K4}) + (1 - f12) \cdot (1 - p(F12\text{'I'}K1\overline{K2}K3\overline{K4}))) \cdot (f13 \cdot p(F13\text{'I'}K1\overline{K2}K3\overline{K4}) + (1 - f13) \cdot (1 - p(F13\text{'I'}K1\overline{K2}K3\overline{K4}))) \cdot (f14 \cdot p(F14\text{'I'}K1\overline{K2}K3\overline{K4}) + (1 - f14) \cdot (1 - p(F14\text{'I'}K1\overline{K2}K3\overline{K4}))) \cdot (f15 \cdot p(F15\text{'I'}K1\overline{K2}K3\overline{K4}) + (1 - f15) \cdot (1 - p(F15\text{'I'}K1\overline{K2}K3\overline{K4}))) \cdot (f16 \cdot p(F16\text{'I'}K1\overline{K2}K3\overline{K4}) + (1 - f16) \cdot (1 - p(F16\text{'I'}K1\overline{K2}K3\overline{K4})));$$

$$b15 := 0.0013 \tag{102}$$

$$> p(F11\text{'I'}K1\overline{K2} \ \overline{K3}K4) := 1 - (1 - p(F11\text{'I'}K1\text{'}\sim\text{'})) \cdot (1 - p(F11\text{'I'}K4\text{'}\sim\text{'}));$$
$$p(F11 \text{ 'I' } K1 \ K2 \ \overline{K3} \ K4) := 0.80 \tag{103}$$

$$> p(F12\text{'I'}K1\overline{K2} \ \overline{K3}K4) := 1 - (1 - p(F12\text{'I'}K1\text{'}\sim\text{'})) \cdot (1 - p(F12\text{'I'}K4\text{'}\sim\text{'}));$$
$$p(F12 \text{ 'I' } K1 \ K2 \ K3 \ K4) := 0.65 \tag{104}$$

$$> p(F13\text{'I'}K1\overline{K2} \ \overline{K3}K4) := 1 - (1 - p(F13\text{'I'}K1\text{'}\sim\text{'})) \cdot (1 - p(F13\text{'I'}K4\text{'}\sim\text{'}));$$
$$p(F13 \text{ 'I' } K1 \ K2 \ K3 \ K4) := 0.65 \tag{105}$$

$$> p(F14\text{'I'}K1\overline{K2} \ \overline{K3}K4) := 1 - (1 - p(F14\text{'I'}K1\text{'}\sim\text{'})) \cdot (1 - p(F14\text{'I'}K4\text{'}\sim\text{'}));$$
$$p(F14 \text{ 'I' } K1 \ K2 \ K3 \ K4) := 0.80 \tag{106}$$

$$> p(F15\text{'I'}K1\overline{K2} \ \overline{K3}K4) := 1 - (1 - p(F15\text{'I'}K1\text{'}\sim\text{'})) \cdot (1 - p(F15\text{'I'}K4\text{'}\sim\text{'}));$$
$$p(F15 \text{ 'I' } K1 \ K2 \ K3 \ K4) := 0.9 \tag{107}$$

$$> p(F16\text{'I'}K1\overline{K2} \ \overline{K3}K4) := 1 - (1 - p(F16\text{'I'}K1\text{'}\sim\text{'})) \cdot (1 - p(F16\text{'I'}K4\text{'}\sim\text{'}));$$
$$p(F16 \text{ 'I' } K1 \ K2 \ K3 \ K4) := 0.7 \tag{108}$$

$$> b16 := (f11 \cdot p(F11\text{'I'}K1\overline{K2}\overline{K3}K4) + (1 - f11) \cdot (1 - p(F11\text{'I'}K1\overline{K2}\overline{K3}K4))) \cdot (f12 \cdot p(F12\text{'I'}K1\overline{K2}\overline{K3}K4) + (1 - f12) \cdot (1 - p(F12\text{'I'}K1\overline{K2}\overline{K3}K4))) \cdot (f13 \cdot p(F13\text{'I'}K1\overline{K2}\overline{K3}K4) + (1 - f13) \cdot (1 - p(F13\text{'I'}K1\overline{K2}\overline{K3}K4))) \cdot (f14 \cdot p(F14\text{'I'}K1\overline{K2}\overline{K3}K4) + (1 - f14) \cdot (1 - p(F14\text{'I'}K1\overline{K2}\overline{K3}K4))) \cdot (f15 \cdot p(F15\text{'I'}K1\overline{K2}\overline{K3}K4) + (1 - f15) \cdot (1 - p(F15\text{'I'}K1\overline{K2}\overline{K3}K4))) \cdot (f16 \cdot p(F16\text{'I'}K1\overline{K2}\overline{K3}K4) + (1 - f16) \cdot (1 - p(F16\text{'I'}K1\overline{K2}\overline{K3}K4)));$$

$$b16 := 0.0081 \tag{109}$$

$$> p(F11\text{'I'}K1\overline{K2} \ \overline{K3} \ \overline{K4}) := p(F11\text{'I'}K1\text{'}\sim\text{'});$$

$$p(F11 \gamma K1 \overline{K2}\,\overline{K3}\,\overline{K4}) := 0.6 \tag{110}$$

> $p(F12\gamma K1 \overline{K2}\,\overline{K3}\,\overline{K4}) := p(F12\gamma K1\textasciitilde);$
$$p(F12 \gamma K1 \overline{K2}\,\overline{K3}\,\overline{K4}) := 0.5 \tag{111}$$

> $p(F13\gamma K1 \overline{K2}\,\overline{K3}\,K4) := p(F13\gamma K1\textasciitilde);$
$$p(F13 \gamma K1 \overline{K2}\,\overline{K3}\,\overline{K4}) := 0.5 \tag{112}$$

> $p(F14\gamma K1 \overline{K2}\,\overline{K3}\,K4) := p(F14\gamma K1\textasciitilde);$
$$p(F14 \gamma K1 \overline{K2}\,\overline{K3}\,\overline{K4}) := 0.6 \tag{113}$$

> $p(F15\gamma K1 \overline{K2}\,\overline{K3}\,K4) := p(F15\gamma K1\textasciitilde);$
$$p(F15 \gamma K1 \overline{K2}\,\overline{K3}\,\overline{K4}) := 0.9 \tag{114}$$

> $p(F16\gamma K1 \overline{K2}\,\overline{K3}\,K4) := p(F16\gamma K1\textasciitilde);$
$$p(F16 \gamma K1 \overline{K2}\,\overline{K3}\,\overline{K4}) := 0.7 \tag{115}$$

> $b17 := (f11 \cdot p(F11\gamma K1 \overline{K2\,K3\,K4}) + (1 - f11) \cdot (1 - p(F11\gamma K1\overline{K2\,K3\,K4}))) \cdot (f12 \cdot p(F12\gamma K1 \overline{K2\,K3\,K4}) + (1 - f12) \cdot (1 - p(F12\gamma K1\overline{K2\,K3\,K4}))) \cdot (f13 \cdot p(F13\gamma K1\overline{K2\,K3\,K4}) + (1 - f13) \cdot (1 - p(F13\gamma K1\overline{K2\,K3\,K4}))) \cdot (f14 \cdot p(F14\gamma K1 \overline{K2\,K3\,K4}) + (1 - f14) \cdot (1 - p(F14\gamma K1\overline{K2\,K3\,K4}))) \cdot (f15 \cdot p(F15\gamma K1\overline{K2\,K3\,K4}) + (1 - f15) \cdot (1 - p(F15\gamma K1\overline{K2\,K3\,K4}))) \cdot (f16 \cdot p(F16\gamma K1\overline{K2\,K3\,K4}) + (1 - f16) \cdot (1 - p(F16\gamma K1\overline{K2\,K3\,K4})));$

$$b17 := 0.0027 \tag{116}$$

> $c1 := 0.25;$
$$c1 := 0.25 \tag{117}$$

> `Eingefügte Nullen für bessere Formatierung`:

> $eq1 := x1 = 1 \Big/ \Big(1 + (a10 \cdot x2 \cdot x3 \cdot x4 + a11 \cdot x2 \cdot x3 \cdot (1 - x4) + a12 \cdot x2 \cdot (1 - x3) \cdot x4 + a13 \cdot x2 \cdot (1 - x3) \cdot (1 - x4)$

$$+ 0 + 0 + 0 + 0 + 0 + 0 + 0 + 0 + 0 + a14 \cdot (1 - x2) \cdot x3 \cdot x4 + a15 \cdot (1 - x2) \cdot x3 \cdot (1 - x4) + a16 \cdot (1$$

$$- x2) \cdot (1 - x3) \cdot x4 + a17 \cdot (1 - x2) \cdot (1 - x3) \cdot (1 - x4)) \Big/ (b10 \cdot x2 \cdot x3 \cdot x4 + b11 \cdot x2 \cdot x3 \cdot (1 - x4) + b12 \cdot x2$$

$$\cdot (1 - x3) \cdot x4 + b13 \cdot x2 \cdot (1 - x3) \cdot (1 - x4) + b14 \cdot (1 - x2) \cdot x3 \cdot x4 + b15 \cdot (1 - x2) \cdot x3 \cdot (1 - x4) + b16 \cdot (1$$

$$- x2) \cdot (1 - x3) \cdot x4 + b17 \cdot (1 - x2) \cdot (1 - x3) \cdot (1 - x4)) \cdot \left(\frac{(1 - c1)}{c1}\right)\Big);$$

$$eq1 := x1 = 1 \Big/ (1 + (3.0 \,(0.043\, x2\, x3\, x4 + 0.0072\, x2\, x3\, (1 - x4) + 0.10\, x2\, (1 - x3)\, x4 \tag{118}$$

$$+ 0.014\, (1 - x2)\, x3\, x4 + 0.022\, (1 - x2)\, (1 - x3)\, x4)) \Big/ (0.0031\, x2\, x3\, x4$$

$$+ 0.0020\, x2\, x3\, (1 - x4) + 0.0090\, x2\, (1 - x3)\, x4 + 0.0050\, x2\, (1 - x3)\, (1 - x4)$$

$$+ 0.0031\, (1 - x2)\, x3\, x4 + 0.0013\, (1 - x2)\, x3\, (1 - x4) + 0.0081\, (1 - x2)\, (1 - x3)\, x4$$

$$+ 0.0027\, (1 - x2)\, (1 - x3)\, (1 - x4)))$$

>
> $f21 := S1; f22 := S2; f23 := S3; f24 := S6; f25 := S7; f26 := S8;$
$$f21 := 1$$
$$f22 := 1$$
$$f23 := 1$$
$$f24 := 0$$
$$f25 := 0$$
$$f26 := 0 \tag{119}$$

>
> $p(F21\gamma K1\textasciitilde) := 0.6; p(F21\gamma K2\textasciitilde) := 0.6; p(F21\gamma K3\textasciitilde) := 0; p(F21\gamma K4\textasciitilde) := 0.5;$

$$p(F21 \text{ '} | \text{' } K1 \text{ '} \sim \text{'}) := 0.6$$

$$p(F21 \text{ '} | \text{' } K2 \text{ '} \sim \text{'}) := 0.6$$

$$p(F21 \text{ '} | \text{' } K3 \text{ '} \sim \text{'}) := 0$$

$$p(F21 \text{ '} | \text{' } K4 \text{ '} \sim \text{'}) := 0.5 \tag{120}$$

$$p(F22 \text{'} | \text{'} K1 \text{'} \sim \text{'}) := 0.5; p(F22 \text{'} | \text{'} K2 \text{'} \sim \text{'}) := 0.5; p(F22 \text{'} | \text{'} K3 \text{'} \sim \text{'}) := 0.3; p(F22 \text{'} | \text{'} K4 \text{'} \sim \text{'}) := 0.3;$$

$$p(F22 \text{ '} | \text{' } K1 \text{ '} \sim \text{'}) := 0.5$$

$$p(F22 \text{ '} | \text{' } K2 \text{ '} \sim \text{'}) := 0.5$$

$$p(F22 \text{ '} | \text{' } K3 \text{ '} \sim \text{'}) := 0.3$$

$$p(F22 \text{ '} | \text{' } K4 \text{ '} \sim \text{'}) := 0.3 \tag{121}$$

$$p(F23 \text{'} | \text{'} K1 \text{'} \sim \text{'}) := 0.5; p(F23 \text{'} | \text{'} K2 \text{'} \sim \text{'}) := 0.5; p(F23 \text{'} | \text{'} K3 \text{'} \sim \text{'}) := 0; p(F23 \text{'} | \text{'} K4 \text{'} \sim \text{'}) := 0.3;$$

$$p(F23 \text{ '} | \text{' } K1 \text{ '} \sim \text{'}) := 0.5$$

$$p(F23 \text{ '} | \text{' } K2 \text{ '} \sim \text{'}) := 0.5$$

$$p(F23 \text{ '} | \text{' } K3 \text{ '} \sim \text{'}) := 0$$

$$p(F23 \text{ '} | \text{' } K4 \text{ '} \sim \text{'}) := 0.3 \tag{122}$$

$$p(F24 \text{'} | \text{'} K1 \text{'} \sim \text{'}) := 0.7; p(F24 \text{'} | \text{'} K2 \text{'} \sim \text{'}) := 0.4; p(F24 \text{'} | \text{'} K3 \text{'} \sim \text{'}) := 0.4; p(F24 \text{'} | \text{'} K4 \text{'} \sim \text{'}) := 0;$$

$$p(F24 \text{ '} | \text{' } K1 \text{ '} \sim \text{'}) := 0.7$$

$$p(F24 \text{ '} | \text{' } K2 \text{ '} \sim \text{'}) := 0.4$$

$$p(F24 \text{ '} | \text{' } K3 \text{ '} \sim \text{'}) := 0.4$$

$$p(F24 \text{ '} | \text{' } K4 \text{ '} \sim \text{'}) := 0 \tag{123}$$

$$p(F25 \text{'} | \text{'} K1 \text{'} \sim \text{'}) := 0; p(F25 \text{'} | \text{'} K2 \text{'} \sim \text{'}) := 0.6; p(F25 \text{'} | \text{'} K3 \text{'} \sim \text{'}) := 0.6; p(F25 \text{'} | \text{'} K4 \text{'} \sim \text{'}) := 0;$$

$$p(F25 \text{ '} | \text{' } K1 \text{ '} \sim \text{'}) := 0$$

$$p(F25 \text{ '} | \text{' } K2 \text{ '} \sim \text{'}) := 0.6$$

$$p(F25 \text{ '} | \text{' } K3 \text{ '} \sim \text{'}) := 0.6$$

$$p(F25 \text{ '} | \text{' } K4 \text{ '} \sim \text{'}) := 0 \tag{124}$$

$$p(F26 \text{'} | \text{'} K1 \text{'} \sim \text{'}) := 0; p(F26 \text{'} | \text{'} K2 \text{'} \sim \text{'}) := 0.7; p(F26 \text{'} | \text{'} K3 \text{'} \sim \text{'}) := 0; p(F26 \text{'} | \text{'} K4 \text{'} \sim \text{'}) := 0.2;$$

$$p(F26 \text{ '} | \text{' } K1 \text{ '} \sim \text{'}) := 0$$

$$p(F26 \text{ '} | \text{' } K2 \text{ '} \sim \text{'}) := 0.7$$

$$p(F26 \text{ '} | \text{' } K3 \text{ '} \sim \text{'}) := 0$$

$$p(F26 \text{ '} | \text{' } K4 \text{ '} \sim \text{'}) := 0.2 \tag{125}$$

$$p(F21 \text{'} | \text{'} \overline{K2} K1 K3 K4) := 1 - (1 - p(F21 \text{'} | \text{'} \underline{K1} \sim \text{'})) \cdot (1 - p(F21 \text{'} | \text{'} K3 \text{'} \sim \text{'})) \cdot (1 - p(F21 \text{'} | \text{'} K4 \text{'} \sim \text{'}));$$
$$p(F21 \text{'} | \text{'} K2 K1 K3 K4) := 0.80 \tag{126}$$

$$p(F22 \text{'} | \text{'} \overline{K2} K1 K3 K4) := 1 - (1 - p(F22 \text{'} | \text{'} \underline{K1} \sim \text{'})) \cdot (1 - p(F22 \text{'} | \text{'} K3 \text{'} \sim \text{'})) \cdot (1 - p(F22 \text{'} | \text{'} K4 \text{'} \sim \text{'}));$$
$$p(F22 \text{'} | \text{'} K2 K1 K3 K4) := 0.76 \tag{127}$$

$$p(F23 \text{'} | \text{'} \overline{K2} K1 K3 K4) := 1 - (1 - p(F23 \text{'} | \text{'} \underline{K1} \sim \text{'})) \cdot (1 - p(F23 \text{'} | \text{'} K3 \text{'} \sim \text{'})) \cdot (1 - p(F23 \text{'} | \text{'} K4 \text{'} \sim \text{'}));$$
$$p(F23 \text{'} | \text{'} K2 K1 K3 K4) := 0.65 \tag{128}$$

$$p(F24 \text{'} | \text{'} \overline{K2} K1 K3 K4) := 1 - (1 - p(F24 \text{'} | \text{'} \underline{K1} \sim \text{'})) \cdot (1 - p(F24 \text{'} | \text{'} K3 \text{'} \sim \text{'})) \cdot (1 - p(F24 \text{'} | \text{'} K4 \text{'} \sim \text{'}));$$
$$p(F24 \text{'} | \text{'} K2 K1 K3 K4) := 0.82 \tag{129}$$

$$p(F25 \text{'} | \text{'} \overline{K2} K1 K3 K4) := 1 - (1 - p(F25 \text{'} | \text{'} \underline{K1} \sim \text{'})) \cdot (1 - p(F25 \text{'} | \text{'} K3 \text{'} \sim \text{'})) \cdot (1 - p(F25 \text{'} | \text{'} K4 \text{'} \sim \text{'}));$$
$$p(F25 \text{'} | \text{'} K2 K1 K3 K4) := 0.6 \tag{130}$$

$$p(F26 \text{'} | \text{'} \overline{K2} K1 K3 K4) := 1 - (1 - p(F26 \text{'} | \text{'} \underline{K1} \sim \text{'})) \cdot (1 - p(F26 \text{'} | \text{'} K3 \text{'} \sim \text{'})) \cdot (1 - p(F26 \text{'} | \text{'} K4 \text{'} \sim \text{'}));$$
$$p(F26 \text{'} | \text{'} K2 K1 K3 K4) := 0.2 \tag{131}$$

$$a20 := (f21 \cdot p(F21 \text{'} | \text{'} \overline{K2} K1 K3 K4) + (1 - f21) \cdot (1 - p(F21 \text{'} | \text{'} \overline{K2} K1 K3 K4))) \cdot (f22 \cdot p(F22 \text{'} | \text{'} \overline{K2} K1 K3 K4)$$
$$+ (1 - f22) \cdot (1 - p(F22 \text{'} | \text{'} \overline{K2} K1 K3 K4))) \cdot (f23 \cdot p(F23 \text{'} | \text{'} \overline{K2} K1 K3 K4) + (1 - f23) \cdot (1 - p(F23 \text{'} | \text{'}$$
$$\overline{K2} K1 K3 K4))) \cdot (f24 \cdot p(F24 \text{'} | \text{'} \overline{K2} K1 K3 K4) + (1 - f24) \cdot (1 - p(F24 \text{'} | \text{'} \overline{K2} K1 K3 K4))) \cdot (f25 \cdot p(F25 \text{'} | \text{'}$$

$\overline{K2}\ K1\,K3\,\underline{K4}) + (1-f25)\cdot(1-p(F25\text{'}\text{'}\overline{K2}\ K1\,K3\,K4).))\cdot(f26\cdot p(F26\text{'}\text{'}\overline{K2}\ K1\cdot K3\,K4) + (1-f26)\cdot(1-p(F26\text{'}\text{'}\overline{K2}\ K1\,K3\,K4)));$

$$a20 := 0.023 \qquad (132)$$

$> a20 := (f21\cdot p(F21\text{'}\text{'}\overline{K2}\ K1\,K3\,\underline{K4}) + (1-f21)\cdot(1-p(F21\text{'}\text{'}\overline{K2\ K1}\,K3\,K4)))\cdot(f22\cdot p(F22\text{'}\text{'}\overline{K2}\ K1\,K3\,K4)$
$\underline{+(1-f22)}\cdot(1-p(F22\text{'}\text{'}\overline{K2}\ \underline{K1}\,K3\,K4)))\cdot(f23\cdot p(F23\text{'}\text{'}\overline{K2}\ K1\,K3\,K4)\,\underline{+(1-f23)}\cdot(1-p(F23\text{'}\text{'}$
$\overline{K2}\ K1\,K3\,K4)))\cdot(f24\cdot p(F24\text{'}\text{'}\overline{K2}\ K1\,K3\,\underline{K4}) + (1-f24)\cdot(1-p(F24\text{'}\text{'}\overline{K2}\ K1\,K3\,K4)))\cdot(f25\cdot p(F25\text{'}\text{'}$
$\overline{K2}\ K1\,K3\,\underline{K4}) + (1-f25)\cdot(1-p(F25\text{'}\text{'}\overline{K2}\ K1\,K3\,K4)))\cdot(f26\cdot p(F26\text{'}\text{'}\overline{K2}\ K1\,K3\,K4) + (1-f26)\cdot(1$
$-p(F26\text{'}\text{'}\overline{K2}\ K1\,K3\,K4)));$

$$a20 := 0.023 \qquad (133)$$

$> p(F21\text{'}\text{'}\overline{K2}\,K1\,K3\,\overline{K4}) := 1 - (1-p(F21\text{'}\text{'}\underline{K1}\,\text{~})))\cdot(1-\underline{p}(F21\text{'}\text{'}K3\text{~}));$
$$p(F21\ \text{'}\text{'}\ K2\ K1\ K3\ \overline{K4}) := 0.6 \qquad (134)$$

$> p(F22\text{'}\text{'}\overline{K2}\,K1\,K3\,\overline{K4}) := 1 - (1-p(F22\text{'}\text{'}\underline{K1}\,\text{~})))\cdot(1-\underline{p}(F22\text{'}\text{'}K3\text{~}));$
$$p(F22\ \text{'}\text{'}\ K2\ K1\ K3\ \overline{K4}) := 0.65 \qquad (135)$$

$> p(F23\text{'}\text{'}\overline{K2}\,K1\,K3\,\overline{K4}) := 1 - (1-p(F23\text{'}\text{'}\underline{K1}\,\text{~})))\cdot(1-\underline{p}(F23\text{'}\text{'}K3\text{~}));$
$$p(F23\ \text{'}\text{'}\ K2\ K1\ K3\ \overline{K4}) := 0.5 \qquad (136)$$

$> p(F24\text{'}\text{'}\overline{K2}\,K1\,K3\,\overline{K4}) := 1 - (1-p(F24\text{'}\text{'}\underline{K1}\,\text{~})))\cdot(1-\underline{p}(F24\text{'}\text{'}K3\text{~}));$
$$p(F24\ \text{'}\text{'}\ K2\ K1\ K3\ \overline{K4}) := 0.82 \qquad (137)$$

$> p(F25\text{'}\text{'}\overline{K2}\,K1\cdot K3\,\overline{K4}) := 1 - (1-p(F25\text{'}\text{'}\underline{K1}\,\text{~})))\cdot(1-\underline{p}(F25\text{'}\text{'}K3\text{~}));$
$$p(F25\ \text{'}\text{'}\ K2\ K1\ K3\ \overline{K4}) := 0.6 \qquad (138)$$

$> p(F26\text{'}\text{'}\overline{K2}\,K1\,K3\,\overline{K4}) := 1 - (1-p(F26\text{'}\text{'}\underline{K1}\,\text{~})))\cdot(1-\underline{p}(F26\text{'}\text{'}K3\text{~}));$
$$p(F26\ \text{'}\text{'}\ K2\ K1\ K3\ \overline{K4}) := 0 \qquad (139)$$

$> a21 := (f21\cdot p(F21\text{'}\text{'}\overline{K2}\ K1\,K3\,\overline{K4}) + (1-f21)\cdot(1-p(F21\text{'}\text{'}\overline{K2}\ K1\,K3\,\overline{K4})))\cdot(f22\cdot p(F22\text{'}\text{'}\overline{K2}\ K1\,K3\,\overline{K4})$
$\underline{+(1-f22)}\cdot(1-p(F22\text{'}\text{'}\overline{K2}\ K1\,K3\,\overline{K4})))\cdot(f23\cdot p(F23\text{'}\text{'}\overline{K2}\ K1\,K3\,\overline{K4})\,\underline{+(1-f23)}\cdot(1-p(F23\text{'}\text{'}$
$\overline{K2}\ K1\,K3\,\overline{K4})))\cdot(f24\cdot p(F24\text{'}\text{'}\overline{K2}\ K1\,K3\,\overline{K4}) + (1-f24)\cdot(1-p(F24\text{'}\text{'}\overline{K2}\ K1\,K3\,\overline{K4})))\cdot(f25\cdot p(F25\text{'}\text{'}$
$\overline{K2}\ K1\,K3\,\overline{K4}) + (1-f25)\cdot(1-p(F25\text{'}\text{'}\overline{K2}\ K1\,K3\,\overline{K4})))\cdot(f26\cdot p(F26\text{'}\text{'}\overline{K2}\ K1\,K3\,\overline{K4}) + (1-f26)\cdot(1$
$-p(F26\text{'}\text{'}\overline{K2}\ K1\,K3\,\overline{K4})));$

$$a21 := 0.014 \qquad (140)$$

$> p(F21\text{'}\text{'}\overline{K2}\,K1\,\overline{K3}\,K4) := 1 - (1-p(F21\text{'}\text{'}\underline{K1}\,\text{~})))\cdot\underline{(1}-p(F21\text{'}\text{'}K4\text{~}));$
$$p(F21\ \text{'}\text{'}\ K2\ K1\ K3\ K4) := 0.80 \qquad (141)$$

$> p(F22\text{'}\text{'}\overline{K2}\,K1\,\overline{K3}\,K4) := 1 - (1-p(F22\text{'}\text{'}\underline{K1}\,\text{~})))\cdot\underline{(1}-p(F22\text{'}\text{'}K4\text{~}));$
$$p(F22\ \text{'}\text{'}\ K2\ K1\ K3\ K4) := 0.65 \qquad (142)$$

$> p(F23\text{'}\text{'}\overline{K2}\,K1\,\overline{K3}\,K4) := 1 - (1-p(F23\text{'}\text{'}\underline{K1}\,\text{~})))\cdot\underline{(1}-p(F23\text{'}\text{'}K4\text{~}));$
$$p(F23\ \text{'}\text{'}\ K2\ K1\ K3\ K4) := 0.65 \qquad (143)$$

$> p(F24\text{'}\text{'}\overline{K2}\,K1\,\overline{K3}\,K4) := 1 - (1-p(F24\text{'}\text{'}\underline{K1}\,\text{~})))\cdot\underline{(1}-p(F24\text{'}\text{'}K4\text{~}));$
$$p(F24\ \text{'}\text{'}\ K2\ K1\ K3\ K4) := 0.7 \qquad (144)$$

$> p(F25\text{'}\text{'}\overline{K2}\,K1\,\overline{K3}\,K4) := 1 - (1-p(F25\text{'}\text{'}\underline{K1}\,\text{~})))\cdot(1-p(F25\text{'}\text{'}K4\text{~}));$
$$p(F25\ \text{'}\text{'}\ K2\ K1\ K3\ K4) := 0 \qquad (145)$$

$> p(F26\text{'}\text{'}\overline{K2}\,K1\,\overline{K3}\,K4) := 1 - (1-p(F26\text{'}\text{'}\underline{K1}\,\text{~})))\cdot\underline{(1}-p(F26\text{'}\text{'}K4\text{~}));$
$$p(F26\ \text{'}\text{'}\ K2\ K1\ K3\ K4) := 0.2 \qquad (146)$$

$> a22 := (f21\cdot p(F21\text{'}\text{'}\overline{K2}\ K1\,\overline{K3}\,K4) + (1-f21)\cdot(1-p(F21\text{'}\text{'}\overline{K2}\ K1\,\overline{K3}\,K4)))\cdot(f22\cdot p(F22\text{'}\text{'}\overline{K2}\ K1\,\overline{K3}\,K4)$
$\underline{+(1-f22)}\cdot(1-p(F22\text{'}\text{'}\overline{K2}\ K1\,\overline{K3}\,K4)))\cdot(f23\cdot p(F23\text{'}\text{'}\overline{K2}\ K1\,\overline{K3}\,K4)\,\underline{+(1-f23)}\cdot(1-p(F23\text{'}\text{'}\overline{K2}\ K1$
$\overline{K3}\,K4)))\cdot(f24\cdot p(F24\text{'}\text{'}\overline{K2}\ K1\,\overline{K3}\,K4) + (1-f24)\cdot(1-p(F24\text{'}\text{'}\overline{K2}\ K1\,\overline{K3}\,K4)))\cdot(f25\cdot p(F25\text{'}\text{'}\overline{K2}\ K1$
$\overline{K3}\,K4)\,\underline{+(1-f25)}\cdot(1-p(F25\text{'}\text{'}\overline{K2}\ K1\,\overline{K3}\,K4)))\cdot(f26\cdot p(F26\text{'}\text{'}\overline{K2}\ K1\,\overline{K3}\,K4) + (1-f26)\cdot(1-p(F26\text{'}\text{'}$
$\overline{K2}\ K1\,\overline{K3}\,K4)));$

$$a22 := 0.080 \qquad (147)$$

$> p(F21\text{'}\text{'}\overline{K2}\,K1\,\overline{K3}\,\overline{K4}) := p(F21\text{'}\text{'}K1\text{~});$

$$(148)$$

$$p\left(F21\,`|`\,\overline{K2}\;K1\;\overline{K3}\;\overline{K4}\right) := 0.6 \tag{148}$$

$> p\left(F22`|`\overline{K2}K1\overline{K3}\overline{K4}\right) := p\left(F22`|`K1`\sim`\right);$
$$p\left(F22\,`|`\,\overline{K2}\;K1\;\overline{K3}\;\overline{K4}\right) := 0.5 \tag{149}$$

$> p\left(F23`|`\overline{K2}K1\overline{K3}\overline{K4}\right) := p\left(F23`|`K1`\sim`\right);$
$$p\left(F23\,`|`\,\overline{K2}\;K1\;\overline{K3}\;\overline{K4}\right) := 0.5 \tag{150}$$

$> p\left(F24`|`\overline{K2}K1\overline{K3}\overline{K4}\right) := p\left(\overline{F24}`|`K1`\sim`\right);$
$$p\left(F24\,`|`\,\overline{K2}\;K1\;\overline{K3}\;\overline{K4}\right) := 0.7 \tag{151}$$

$> p\left(F25`|`\overline{K2}K1\overline{K3}\overline{K4}\right) := p\left(F25`|`K1`\sim`\right);$
$$p\left(F25\,`|`\,\overline{K2}\;K1\;\overline{K3}\;\overline{K4}\right) := 0 \tag{152}$$

$> p\left(F26`|`\overline{K2}K1\overline{K3}\overline{K4}\right) := p\left(F26`|`K1`\sim`\right);$
$$p\left(F26\,`|`\,\overline{K2}\;K1\;\overline{K3}\;\overline{K4}\right) := 0 \tag{153}$$

$> a23 := \left(f21 \cdot p\left(F21`|`\overline{K2}\;K1\overline{K3}\overline{K4}\right) + (1-f21) \cdot \left(1 - p\left(F21`|`\overline{K2}\;K1\overline{K3}\overline{K4}\right)\right)\right) \cdot \left(f22 \cdot p\left(F22`|`\overline{K2}\;K1\overline{K3}\overline{K4}\right) + (1-f22) \cdot \left(1 - p\left(F22`|`\overline{K2}\;K1\overline{K3}\overline{K4}\right)\right)\right) \cdot \left(f23 \cdot p\left(F23`|`\overline{K2}\;K1\overline{K3}\overline{K4}\right) + (1-f23) \cdot \left(1 - p\left(F23`|`\overline{K2}\;K1\overline{K3}\overline{K4}\right)\right)\right) \cdot \left(f24 \cdot p\left(F24`|`\overline{K2}\;K1\overline{K3}\overline{K4}\right) + (1-f24) \cdot \left(1 - p\left(F24`|`\overline{K2}\;K1\overline{K3}\overline{K4}\right)\right)\right) \cdot \left(f25 \cdot p\left(F25`|`\overline{K2}\;K1\overline{K3}\overline{K4}\right) + (1-f25) \cdot \left(1 - p\left(F25`|`\overline{K2}\;K1\overline{K3}\overline{K4}\right)\right)\right) \cdot \left(f26 \cdot p\left(F26`|`\overline{K2}\;K1\overline{K3}\overline{K4}\right) + (1-f26) \cdot \left(1 - p\left(F26`|`\overline{K2}\;K1\overline{K3}\overline{K4}\right)\right)\right);$
$$a23 := 0.045 \tag{154}$$

$> p\left(F21`|`\overline{K2}\overline{K1}\;K3\;K4\right) := 1 - \left(1 - p\left(F21`|`K3`\sim`\right)\right) \cdot \left(1 - p\left(F21`|`K4`\sim`\right)\right);$
$$p\left(F21\,`|`\,\overline{K2}\;\overline{K1}\;K3\;K4\right) := 0.5 \tag{155}$$

$> p\left(F22`|`\overline{K2}\overline{K1}\;K3\;K4\right) := 1 - \left(1 - p\left(F22`|`K3`\sim`\right)\right) \cdot \left(1 - p\left(F22`|`K4`\sim`\right)\right);$
$$p\left(F22\,`|`\,\overline{K2}\;\overline{K1}\;K3\;K4\right) := 0.51 \tag{156}$$

$> p\left(F23`|`\overline{K2}\overline{K1}\;K3\;K4\right) := 1 - \left(1 - p\left(F23`|`K3`\sim`\right)\right) \cdot \left(1 - p\left(F23`|`K4`\sim`\right)\right);$
$$p\left(F23\,`|`\,\overline{K2}\;\overline{K1}\;K3\;K4\right) := 0.3 \tag{157}$$

$> p\left(F24`|`\overline{K2}\overline{K1}\;K3\;K4\right) := 1 - \left(1 - p\left(F24`|`K3`\sim`\right)\right) \cdot \left(1 - p\left(F24`|`K4`\sim`\right)\right);$
$$p\left(F24\,`|`\,\overline{K2}\;\overline{K1}\;K3\;K4\right) := 0.4 \tag{158}$$

$> p\left(F25`|`\overline{K2}\overline{K1}\;K3\;K4\right) := 1 - \left(1 - p\left(F25`|`K3`\sim`\right)\right) \cdot \left(1 - p\left(F25`|`K4`\sim`\right)\right);$
$$p\left(F25\,`|`\,\overline{K2}\;\overline{K1}\;K3\;K4\right) := 0.6 \tag{159}$$

$> p\left(F26`|`\overline{K2}\overline{K1}\;K3\;K4\right) := 1 - \left(1 - p\left(F26`|`K3`\sim`\right)\right) \cdot \left(1 - p\left(F26`|`K4`\sim`\right)\right);$
$$p\left(F26\,`|`\,\overline{K2}\;\overline{K1}\;K3\;K4\right) := 0.2 \tag{160}$$

$> a24 := \left(f21 \cdot p\left(F21`|`\overline{K2}\;\overline{K1}\;K3\;K4\right) + (1-f21) \cdot \left(1 - p\left(F21`|`\overline{K2}\;\overline{K1}\;K3\;K4\right)\right)\right) \cdot \left(f22 \cdot p\left(F22`|`\overline{K2}\;\overline{K1}\;K3\;K4\right) + (1-f22) \cdot \left(1 - p\left(F22`|`\overline{K2}\;\overline{K1}\;K3\;K4\right)\right)\right) \cdot \left(f23 \cdot p\left(F23`|`\overline{K2}\;\overline{K1}\;K3\;K4\right) + (1-f23) \cdot \left(1 - p\left(F23`|`\overline{K2}\;\overline{K1}\;K3\;K4\right)\right)\right) \cdot \left(f24 \cdot p\left(F24`|`\overline{K2}\;\overline{K1}\;K3\;K4\right) + (1-f24) \cdot \left(1 - p\left(F24`|`\overline{K2}\;\overline{K1}\;K3\;K4\right)\right)\right) \cdot \left(f25 \cdot p\left(F25`|`\overline{K2}\;\overline{K1}\;K3\;K4\right) + (1-f25) \cdot \left(1 - p\left(F25`|`\overline{K2}\;\overline{K1}\;K3\;K4\right)\right)\right) \cdot \left(f26 \cdot p\left(F26`|`\overline{K2}\;\overline{K1}\;K3\;K4\right) + (1-f26) \cdot \left(1 - p\left(F26`|`\overline{K2}\;\overline{K1}\;K3\;K4\right)\right)\right);$
$$a24 := 0.015 \tag{161}$$

$> p\left(F21`|`\overline{K2}\overline{K1}\;K3\;\overline{K4}\right) := p\left(F21`|`K3`\sim`\right);$
$$p\left(F21\,`|`\,\overline{K2}\;\overline{K1}\;K3\;\overline{K4}\right) := 0 \tag{162}$$

$> p\left(F22`|`\overline{K2}\overline{K1}\;K3\;\overline{K4}\right) := p\left(F21`|`K3`\sim`\right);$
$$p\left(F22\,`|`\,\overline{K2}\;\overline{K1}\;K3\;\overline{K4}\right) := 0 \tag{163}$$

$> p\left(F23`|`\overline{K2}\overline{K1}\;K3\;\overline{K4}\right) := p\left(F23`|`K3`\sim`\right);$
$$p\left(F23\,`|`\,\overline{K2}\;\overline{K1}\;K3\;\overline{K4}\right) := 0 \tag{164}$$

$> p\left(F24`|`\overline{K2}\overline{K1}\;K3\;\overline{K4}\right) := p\left(F24`|`K3`\sim`\right);$
$$p\left(F24\,`|`\,\overline{K2}\;\overline{K1}\;K3\;\overline{K4}\right) := 0.4 \tag{165}$$

$> p\left(F25`|`\overline{K2}\overline{K1}\;K3\;\overline{K4}\right) := p\left(F25`|`K3`\sim`\right);$
$$p\left(F25\,`|`\,\overline{K2}\;\overline{K1}\;K3\;\overline{K4}\right) := 0.6 \tag{166}$$

> $p(F26\,\text{'l'}\,\overline{K2}\,\overline{K1}\,K3\,\overline{K4}) := p(F26\,\text{'l'}\,K3\text{'}\sim\text{'});$

$$p(F26\,\text{'l'}\,\overline{K2}\,\overline{K1}\,K3\,\overline{K4}) := 0 \tag{167}$$

> $a25 := (f21 \cdot p(F21\,\text{'l'}\,\overline{K2}\,\overline{K1}\,K3\,\overline{K4}) + (1 - f21) \cdot (1 - p(F21\,\text{'l'}\,\overline{K2}\,\overline{K1}\,K3\,\overline{K4}))) \cdot (f22 \cdot p(F22\,\text{'l'}\,\overline{K2}\,\overline{K1}\,K3\,\overline{K4})$
$+ (1 - f22) \cdot (1 - p(F22\,\text{'l'}\,\overline{K2}\,\overline{K1}\,K3\,\overline{K4}))) \cdot (f23 \cdot p(F23\,\text{'l'}\,\overline{K2}\,\overline{K1}\,K3\,\overline{K4}) + (1 - f23) \cdot (1 - p(F23\,\text{'l'}\,\overline{K2}$
$\overline{K1}\,K3\,\overline{K4}))) \cdot (f24 \cdot p(F24\,\text{'l'}\,\overline{K2}\,\overline{K1}\,K3\,\overline{K4}) + (1 - f24) \cdot (1 - p(F24\,\text{'l'}\,\overline{K2}\,\overline{K1}\,K3\,\overline{K4}))) \cdot (f25 \cdot p(F25\,\text{'l'}\,\overline{K2}$
$\overline{K1}\,K3\,\overline{K4}) + (1 - f25) \cdot (1 - p(F25\,\text{'l'}\,\overline{K2}\,\overline{K1}\,K3\,\overline{K4}))) \cdot (f26 \cdot p(F26\,\text{'l'}\,\overline{K2}\,\overline{K1}\,K3\,\overline{K4}) + (1 - f26) \cdot (1$
$- p(F26\,\text{'l'}\,\overline{K2}\,\overline{K1}\,K3\,\overline{K4})));$

$$a25 := 0. \tag{168}$$

> $p(F21\,\text{'l'}\,\overline{K2}\,\overline{K1}\,\overline{K3}\,K4) := p(F21\,\text{'l'}\,K4\text{'}\sim\text{'});$

$$p(F21\,\text{'l'}\,\overline{K2}\,\overline{K1}\,\overline{K3}\,K4) := 0.5 \tag{169}$$

> $p(F22\,\text{'l'}\,\overline{K2}\,\overline{K1}\,\overline{K3}\,K4) := p(F22\,\text{'l'}\,K4\text{'}\sim\text{'});$

$$p(F22\,\text{'l'}\,\overline{K2}\,\overline{K1}\,\overline{K3}\,K4) := 0.3 \tag{170}$$

> $p(F23\,\text{'l'}\,\overline{K2}\,\overline{K1}\,\overline{K3}\,K4) := p(F23\,\text{'l'}\,K4\text{'}\sim\text{'});$

$$p(F23\,\text{'l'}\,\overline{K2}\,\overline{K1}\,\overline{K3}\,K4) := 0.3 \tag{171}$$

> $p(F24\,\text{'l'}\,\overline{K2}\,\overline{K1}\,\overline{K3}\,K4) := p(F24\,\text{'l'}\,K4\text{'}\sim\text{'});$

$$p(F24\,\text{'l'}\,\overline{K2}\,\overline{K1}\,\overline{K3}\,K4) := 0 \tag{172}$$

> $p(F25\,\text{'l'}\,\overline{K2}\,\overline{K1}\,\overline{K3}\,K4) := p(F25\,\text{'l'}\,K4\text{'}\sim\text{'});$

$$p(F25\,\text{'l'}\,\overline{K2}\,\overline{K1}\,\overline{K3}\,K4) := 0 \tag{173}$$

> $p(F26\,\text{'l'}\,\overline{K2}\,\overline{K1}\,\overline{K3}\,K4) := p(F26\,\text{'l'}\,K4\text{'}\sim\text{'});$

$$p(F26\,\text{'l'}\,\overline{K2}\,\overline{K1}\,\overline{K3}\,K4) := 0.2 \tag{174}$$

> $a26 := (f21 \cdot p(F21\,\text{'l'}\,\overline{K2}\,\overline{K1}\,\overline{K3}\,K4) + (1 - f21) \cdot (1 - p(F21\,\text{'l'}\,\overline{K2}\,\overline{K1}\,\overline{K3}\,K4))) \cdot (f22 \cdot p(F22\,\text{'l'}\,\overline{K2}\,\overline{K1}\,\overline{K3}\,K4)$
$+ (1 - f22) \cdot (1 - p(F22\,\text{'l'}\,\overline{K2}\,\overline{K1}\,\overline{K3}\,K4))) \cdot (f23 \cdot p(F23\,\text{'l'}\,\overline{K2}\,\overline{K1}\,\overline{K3}\,K4) + (1 - f23) \cdot (1 - p(F23\,\text{'l'}\,\overline{K2}\,\overline{K1}$
$\overline{K3}\,K4))) \cdot (f24 \cdot p(F24\,\text{'l'}\,\overline{K2}\,\overline{K1}\,\overline{K3}\,K4) + (1 - f24) \cdot (1 - p(F24\,\text{'l'}\,\overline{K2}\,\overline{K1}\,\overline{K3}\,K4))) \cdot (f25 \cdot p(F25\,\text{'l'}\,\overline{K2}\,\overline{K1}$
$\overline{K3}\,K4) + (1 - f25) \cdot (1 - p(F25\,\text{'l'}\,\overline{K2}\,\overline{K1}\,\overline{K3}\,K4))) \cdot (f26 \cdot p(F26\,\text{'l'}\,\overline{K2}\,\overline{K1}\,\overline{K3}\,K4) + (1 - f26) \cdot (1 - p(F26\,\text{'l'}$
$\overline{K2}\,\overline{K1}\,\overline{K3}\,K4)));$

$$a26 := 0.036 \tag{175}$$

> $a27 := 0;$

$$a27 := 0 \tag{176}$$

> $p(F21\,\text{'l'}\,K2\,K1\,K3\,K4) := 1 - (1 - p(F21\,\text{'l'}\,K1\text{'}\sim\text{'})) \cdot (1 - p(F21\,\text{'l'}\,K2\text{'}\sim\text{'})) \cdot (1 - p(F21\,\text{'l'}\,K3\text{'}\sim\text{'})) \cdot (1$
$- p(F21\,\text{'l'}\,K4\text{'}\sim\text{'}));$

$$p(F21\,\text{'l'}\,K2\,K1\,K3\,K4) := 0.92 \tag{177}$$

> $p(F22\,\text{'l'}\,K2\,K1\,K3\,K4) := 1 - (1 - p(F22\,\text{'l'}\,K1\text{'}\sim\text{'})) \cdot (1 - p(F22\,\text{'l'}\,K2\text{'}\sim\text{'})) \cdot (1 - p(F22\,\text{'l'}\,K3\text{'}\sim\text{'})) \cdot (1$
$- p(F22\,\text{'l'}\,K4\text{'}\sim\text{'}));$

$$p(F22\,\text{'l'}\,K2\,K1\,K3\,K4) := 0.87 \tag{178}$$

> $p(F23\,\text{'l'}\,K2\,K1\,K3\,K4) := 1 - (1 - p(F23\,\text{'l'}\,K1\text{'}\sim\text{'})) \cdot (1 - p(F23\,\text{'l'}\,K2\text{'}\sim\text{'})) \cdot (1 - p(F23\,\text{'l'}\,K3\text{'}\sim\text{'})) \cdot (1$
$- p(F23\,\text{'l'}\,K4\text{'}\sim\text{'}));$

$$p(F23\,\text{'l'}\,K2\,K1\,K3\,K4) := 0.82 \tag{179}$$

> $p(F24\,\text{'l'}\,K2\,K1\,K3\,K4) := 1 - (1 - p(F24\,\text{'l'}\,K1\text{'}\sim\text{'})) \cdot (1 - p(F24\,\text{'l'}\,K2\text{'}\sim\text{'})) \cdot (1 - p(F24\,\text{'l'}\,K3\text{'}\sim\text{'})) \cdot (1$
$- p(F24\,\text{'l'}\,K4\text{'}\sim\text{'}));$

$$p(F24\,\text{'l'}\,K2\,K1\,K3\,K4) := 0.89 \tag{180}$$

> $p(F25\,\text{'l'}\,K2\,K1\,K3\,K4) := 1 - (1 - p(F25\,\text{'l'}\,K1\text{'}\sim\text{'})) \cdot (1 - p(F25\,\text{'l'}\,K2\text{'}\sim\text{'})) \cdot (1 - p(F25\,\text{'l'}\,K3\text{'}\sim\text{'})) \cdot (1$
$- p(F25\,\text{'l'}\,K4\text{'}\sim\text{'}));$

$$p(F25\,\text{'l'}\,K2\,K1\,K3\,K4) := 0.84 \tag{181}$$

> $p(F26\,\text{'l'}\,K2\,K1\,K3\,K4) := 1 - (1 - p(F26\,\text{'l'}\,K1\text{'}\sim\text{'})) \cdot (1 - p(F26\,\text{'l'}\,K2\text{'}\sim\text{'})) \cdot (1 - p(F26\,\text{'l'}\,K3\text{'}\sim\text{'})) \cdot (1$
$- p(F26\,\text{'l'}\,K4\text{'}\sim\text{'}));$

$$p(F26\,\text{'l'}\,K2\,K1\,K3\,K4) := 0.76 \tag{182}$$

> $b20 := (f21 \cdot p(F21\,\text{'l'}\,K2\,K1\,K3\,K4) + (1 - f21) \cdot (1 - p(F21\,\text{'l'}\,K2\,K1\,K3\,K4))) \cdot (f22 \cdot p(F22\,\text{'l'}\,K2\,K1\,K3\,K4) + (1$
$- f22) \cdot (1 - p(F22\,\text{'l'}\,K2\,K1\,K3\,K4))) \cdot (f23 \cdot p(F23\,\text{'l'}\,K2\,K1\,K3\,K4) + (1 - f23) \cdot (1$

$-p(F23\text{'I'}K2\,K1\,K3\,K4)))\cdot(f24\cdot p(F24\text{'I'}K2\,K1\,K3\,K4) + (1-f24)\cdot(1-p(F24\text{'I'}K2\,K1\,K3\,K4)))\cdot(f25$
$\cdot p(F25\text{'I'}K2\,K1\,K3\,K4) + (1-f25)\cdot(1-p(F25\text{'I'}K2\,K1\,K3\,K4)))\cdot(f26\cdot p(F26\text{'I'}K2\,K1\,K3\,K4) + (1-f26)$
$\cdot(1-p(F26\text{'I'}K2\,K1\,K3\,K4))));$

$$b20 := 0.0029 \tag{183}$$

$>\ p(F21\text{'I'}K2\,K1\,K3\,\overline{K4}) := 1 - (1 - p(F21\text{'I'}K1\text{'}\sim\text{'})) \cdot (1 - p(F21\text{'I'}K2\text{'}\sim\text{'})) \cdot (1 - p(F21\text{'I'}K3\text{'}\sim\text{'}));$
$$p(F21\text{ 'I' }K2\,K1\,K3\,\overline{K4}) := 0.84 \tag{184}$$

$>\ p(\overline{F22}\text{'I'}K2\,\overline{K1}\,K3\,\overline{K4}) := 1 - (1 - p(\overline{F22}\text{'I'}K1\text{'}\sim\text{'})) \cdot (1 - p(\overline{F22}\text{'I'}K2\text{'}\sim\text{'})) \cdot (1 - p(\overline{F22}\text{'I'}K3\text{'}\sim\text{'}));$
$$p(F22\text{ 'I' }K2\,K1\,K3\,\overline{K4}) := 0.82 \tag{185}$$

$>\ p(F23\text{'I'}K2\,K1\,K3\,\overline{K4}) := 1 - (1 - p(F23\text{'I'}K1\text{'}\sim\text{'})) \cdot (1 - p(F23\text{'I'}K2\text{'}\sim\text{'})) \cdot (1 - p(F23\text{'I'}K3\text{'}\sim\text{'}));$
$$p(F23\text{ 'I' }K2\,K1\,K3\,\overline{K4}) := 0.75 \tag{186}$$

$>\ p(F24\text{'I'}K2\,K1\,K3\,\overline{K4}) := 1 - (1 - p(F24\text{'I'}K1\text{'}\sim\text{'})) \cdot (1 - p(F24\text{'I'}K2\text{'}\sim\text{'})) \cdot (1 - p(F24\text{'I'}K3\text{'}\sim\text{'}));$
$$p(F24\text{ 'I' }K2\,K1\,K3\,\overline{K4}) := 0.89 \tag{187}$$

$>\ p(F25\text{'I'}K2\,K1\,K3\,\overline{K4}) := 1 - (1 - p(F25\text{'I'}K1\text{'}\sim\text{'})) \cdot (1 - p(F25\text{'I'}K2\text{'}\sim\text{'})) \cdot (1 - p(F25\text{'I'}K3\text{'}\sim\text{'}));$
$$p(F25\text{ 'I' }K2\,K1\,K3\,\overline{K4}) := 0.84 \tag{188}$$

$>\ p(F26\text{'I'}K2\,K1\,K3\,\overline{K4}) := 1 - (1 - p(F26\text{'I'}K1\text{'}\sim\text{'})) \cdot (1 - p(F26\text{'I'}K2\text{'}\sim\text{'})) \cdot (1 - p(F26\text{'I'}K3\text{'}\sim\text{'}));$
$$p(F26\text{ 'I' }K2\,K1\,K3\,\overline{K4}) := 0.7 \tag{189}$$

$>\ b21 := (f21\cdot p(F21\text{'I'}K2\,K1\,K3\,\overline{K4}) + (1-f21)\cdot(1-p(F21\text{'I'}K2\,K1\,K3\,\overline{K4})))\cdot(f22\cdot p(F22\text{'I'}K2\,K1\,K3\,\overline{K4}) + (1$
$-f22)\cdot(1-p(F22\text{'I'}K2\,K1\,K3\,\overline{K4})))\cdot(f23\cdot p(F23\text{'I'}K2\,K1\,K3\,\overline{K4}) + (1-f23)\cdot(1-p(F23\text{'I'}K2\,K1\,K3\,\overline{K4})))\cdot(f24\cdot p(F24\text{'I'}K2\,K1\,K3\,\overline{K4}) + (1-f24)\cdot(1-p(F24\text{'I'}K2\,K1\,K3\,\overline{K4})))\cdot(f25\cdot p(F25\text{'I'}K2\,K1\,K3\,\overline{K4})$
$+ (1-f25)\cdot(1-p(F25\text{'I'}K2\,K1\,K3\,\overline{K4})))\cdot(f26\cdot p(F26\text{'I'}K2\,K1\,K3\,\overline{K4}) + (1-f26)\cdot(1-p(F26\text{'I'}K2\,K1\,K3\,\overline{K4}))));$

$$b21 := 0.0027 \tag{190}$$

$>\ p(F21\text{'I'}K2\,K1\,\overline{K3}\,K4) := 1 - (1 - p(F21\text{'I'}K1\text{'}\sim\text{'})) \cdot (1 - p(F21\text{'I'}K2\text{'}\sim\text{'})) \cdot (1 - p(F21\text{'I'}K4\text{'}\sim\text{'}));$
$$p(F21\text{ 'I' }K2\,K1\,\overline{K3}\,K4) := 0.92 \tag{191}$$

$>\ p(F22\text{'I'}K2\,K1\,\overline{K3}\,K4) := 1 - (1 - p(F22\text{'I'}K1\text{'}\sim\text{'})) \cdot (1 - p(F22\text{'I'}K2\text{'}\sim\text{'})) \cdot (1 - p(F22\text{'I'}K4\text{'}\sim\text{'}));$
$$p(F22\text{ 'I' }K2\,K1\,\overline{K3}\,K4) := 0.82 \tag{192}$$

$>\ p(F23\text{'I'}K2\,K1\,\overline{K3}\,K4) := 1 - (1 - p(F23\text{'I'}K1\text{'}\sim\text{'})) \cdot (1 - p(F23\text{'I'}K2\text{'}\sim\text{'})) \cdot (1 - p(F23\text{'I'}K4\text{'}\sim\text{'}));$
$$p(F23\text{ 'I' }K2\,K1\,\overline{K3}\,K4) := 0.82 \tag{193}$$

$>\ p(F24\text{'I'}K2\,K1\,\overline{K3}\,K4) := 1 - (1 - p(F24\text{'I'}K1\text{'}\sim\text{'})) \cdot (1 - p(F24\text{'I'}K2\text{'}\sim\text{'})) \cdot (1 - p(F24\text{'I'}K4\text{'}\sim\text{'}));$
$$p(F24\text{ 'I' }K2\,K1\,\overline{K3}\,K4) := 0.82 \tag{194}$$

$>\ p(F25\text{'I'}K2\,K1\,\overline{K3}\,K4) := 1 - (1 - p(F25\text{'I'}K1\text{'}\sim\text{'})) \cdot (1 - p(F25\text{'I'}K2\text{'}\sim\text{'})) \cdot (1 - p(F25\text{'I'}K4\text{'}\sim\text{'}));$
$$p(F25\text{ 'I' }K2\,K1\,\overline{K3}\,K4) := 0.6 \tag{195}$$

$>\ p(F26\text{'I'}K2\,K1\,\overline{K3}\,K4) := 1 - (1 - p(F26\text{'I'}K1\text{'}\sim\text{'})) \cdot (1 - p(F26\text{'I'}K2\text{'}\sim\text{'})) \cdot (1 - p(F26\text{'I'}K4\text{'}\sim\text{'}));$
$$p(F26\text{ 'I' }K2\,K1\,\overline{K3}\,K4) := 0.76 \tag{196}$$

$>\ b22 := (f21\cdot p(F21\text{'I'}K2\,K1\,\overline{K3}\,K4) + (1-f21)\cdot(1-p(F21\text{'I'}K2\,K1\,\overline{K3}\,K4)))\cdot(f22\cdot p(F22\text{'I'}K2\,K1\,\overline{K3}\,K4) + (1$
$-f22)\cdot(1-p(F22\text{'I'}K2\,K1\,\overline{K3}\,K4)))\cdot(f23\cdot p(F23\text{'I'}K2\,K1\,\overline{K3}\,K4) + (1-f23)\cdot(1-p(F23\text{'I'}K2\,K1\,\overline{K3}\,K4)))\cdot(f24\cdot p(F24\text{'I'}K2\,K1\,\overline{K3}\,K4) + (1-f24)\cdot(1-p(F24\text{'I'}K2\,K1\,\overline{K3}\,K4)))\cdot(f25\cdot p(F25\text{'I'}K2\,K1\,\overline{K3}\,K4) + (1-f25)\cdot(1-p(F25\text{'I'}K2\,K1\,\overline{K3}\,K4)))\cdot(f26\cdot p(F26\text{'I'}K2\,K1\,\overline{K3}\,K4) + (1-f26)\cdot(1-p(F26\text{'I'}K2\,K1\,\overline{K3}\,K4)));$

$$b22 := 0.011 \tag{197}$$

$>\ p(F21\text{'I'}K2\,K1\,\overline{K3}\,\overline{K4}) := 1 - (1 - p(F21\text{'I'}K1\text{'}\sim\text{'})) \cdot (1 - p(F21\text{'I'}K2\text{'}\sim\text{'}));$
$$p(F21\text{ 'I' }K2\,K1\,\overline{K3}\,\overline{K4}) := 0.84 \tag{198}$$

$>\ p(F22\text{'I'}K2\,K1\,\overline{K3}\,\overline{K4}) := 1 - (1 - p(F22\text{'I'}K1\text{'}\sim\text{'})) \cdot (1 - p(F22\text{'I'}K2\text{'}\sim\text{'}));$
$$p(F22\text{ 'I' }K2\,K1\,\overline{K3}\,\overline{K4}) := 0.75 \tag{199}$$

$>\ p(F23\text{'I'}K2\,K1\,\overline{K3}\,\overline{K4}) := 1 - (1 - p(F23\text{'I'}K1\text{'}\sim\text{'})) \cdot (1 - p(F23\text{'I'}K2\text{'}\sim\text{'}));$
$$p(F23\text{ 'I' }K2\,K1\,K3\,\overline{K4}) := 0.75 \tag{200}$$

$>\ p(F24\text{'I'}K2\,K1\,\overline{K3}\,\overline{K4}) := 1 - (1 - p(F24\text{'I'}K1\text{'}\sim\text{'})) \cdot (1 - p(F24\text{'I'}K2\text{'}\sim\text{'}));$

$$p(F24\ \`|\`\ K2\ K1\ \overline{K3}\ \overline{K4}) := 0.82 \tag{201}$$

> $p(F25\`|\`K2K1\overline{K3}\overline{K4}) := 1 - (1 - p(F25\`|\`K1\`\sim\`)) \cdot (1 - p(F25\`|\`K2\`\sim\`));$
$$p(F25\ \`|\`\ K2\ K1\ K3\ \overline{K4}) := 0.6 \tag{202}$$

> $p(F26\`|\`K2K1\overline{K3}\overline{K4}) := 1 - (1 - p(F26\`|\`K1\`\sim\`)) \cdot (1 - p(F26\`|\`K2\`\sim\`));$
$$p(F26\ \`|\`\ K2\ K1\ K3\ \overline{K4}) := 0.7 \tag{203}$$

>

> $b23 := (f21 \cdot p(F21\`|\`K2 K1 \overline{K3}\overline{K4}) + (1 - f21) \cdot (1 - p(F21\`|\`K2K1\overline{K3}\overline{K4}))) \cdot (f22 \cdot p(F22\`|\`K2 K1 \overline{K3}\overline{K4}) + (1 - f22) \cdot (1 - p(F22\`|\`K2 K1 \overline{K3}\overline{K4}))) \cdot (f23 \cdot p(F23\`|\`K2 K1 K3 K4) + (1 - f23) \cdot (1 - p(F23\`|\`K2 K1 K3 K4))) \cdot (f24 \cdot p(F24\`|\`K2 K1 K3 K4) + (1 - f24) \cdot (1 - p(F24\`|\`K2K1\overline{K3}K4))) \cdot (f25 \cdot p(F25\`|\`K2 K1 \overline{K3}\overline{K4}) + (1 - f25) \cdot (1 - p(F25\`|\`K2K1\overline{K3}K4))) \cdot (f26 \cdot p(F26\`|\`K2 K1 \overline{K3}K4) + (1 - f26) \cdot (1 - p(F26\`|\`K2 K1 K3 \overline{K4})));$
$$b23 := 0.010 \tag{204}$$

> $p(F21\`|\`K2\overline{K1}K3K4) := 1 - (1 - p(F21\`|\`K2\`\sim\`)) \cdot (1 - p(F21\`|\`K3\`\sim\`)) \cdot (1 - p(F21\`|\`K4\`\sim\`));$
$$p(F21\ \`|\`\ K2\ K1\ K3\ K4) := 0.80 \tag{205}$$

> $p(F22\`|\`K2\overline{K1}K3K4) := 1 - (1 - p(F22\`|\`K2\`\sim\`)) \cdot (1 - p(F22\`|\`K3\`\sim\`)) \cdot (1 - p(F22\`|\`K4\`\sim\`));$
$$p(F22\ \`|\`\ K2\ K1\ K3\ K4) := 0.76 \tag{206}$$

> $p(F23\`|\`K2\overline{K1}K3K4) := 1 - (1 - p(F23\`|\`K2\`\sim\`)) \cdot (1 - p(F23\`|\`K3\`\sim\`)) \cdot (1 - p(F23\`|\`K4\`\sim\`));$
$$p(F23\ \`|\`\ K2\ K1\ K3\ K4) := 0.65 \tag{207}$$

> $p(F24\`|\`K2\overline{K1}K3K4) := 1 - (1 - p(F24\`|\`K2\`\sim\`)) \cdot (1 - p(F24\`|\`K3\`\sim\`)) \cdot (1 - p(F24\`|\`K4\`\sim\`));$
$$p(F24\ \`|\`\ K2\ K1\ K3\ K4) := 0.64 \tag{208}$$

> $p(F25\`|\`K2\overline{K1}K3K4) := 1 - (1 - p(F25\`|\`K2\`\sim\`)) \cdot (1 - p(F25\`|\`K3\`\sim\`)) \cdot (1 - p(F25\`|\`K4\`\sim\`));$
$$p(F25\ \`|\`\ K2\ K1\ K3\ K4) := 0.84 \tag{209}$$

> $p(F26\`|\`K2\overline{K1}K3K4) := 1 - (1 - p(F26\`|\`K2\`\sim\`)) \cdot (1 - p(F26\`|\`K3\`\sim\`)) \cdot (1 - p(F26\`|\`K4\`\sim\`));$
$$p(F26\ \`|\`\ K2\ K1\ K3\ K4) := 0.76 \tag{210}$$

> $b24 := (f21 \cdot p(F21\`|\`K2\overline{K1}K3K4) + (1 - f21) \cdot (1 - p(F21\`|\`K2\overline{K1}K3K4))) \cdot (f22 \cdot p(F22\`|\`K2\overline{K1}K3K4) + (1 - f22) \cdot (1 - p(F22\`|\`K2\overline{K1}K3K4))) \cdot (f23 \cdot p(F23\`|\`K2\overline{K1}K3K4) + (1 - f23) \cdot (1 - p(F23\`|\`K2\overline{K1}K3K4))) \cdot (f24 \cdot p(F24\`|\`K2\overline{K1}K3K4) + (1 - f24) \cdot (1 - p(F24\`|\`K2\overline{K1}K3K4))) \cdot (f25 \cdot p(F25\`|\`K2\overline{K1}K3K4) + (1 - f25) \cdot (1 - p(F25\`|\`K2\overline{K1}K3K4))) \cdot (f26 \cdot p(F26\`|\`K2\overline{K1}K3K4) + (1 - f26) \cdot (1 - p(F26\`|\`K2\overline{K1}K3K4)));$
$$b24 := 0.0053 \tag{211}$$

> $p(F21\`|\`K2\overline{K1}K3\overline{K4}) := 1 - (1 - p(F21\`|\`K2\`\sim\`)) \cdot (1 - p(F21\`|\`K3\`\sim\`));$
$$p(F21\ \`|\`\ K2\ K1\ K3\ \overline{K4}) := 0.6 \tag{212}$$

> $p(F22\`|\`K2\overline{K1}K3\overline{K4}) := 1 - (1 - p(F22\`|\`K2\`\sim\`)) \cdot (1 - p(F22\`|\`K3\`\sim\`));$
$$p(F22\ \`|\`\ K2\ K1\ K3\ K4) := 0.65 \tag{213}$$

> $p(F23\`|\`K2\overline{K1}K3\overline{K4}) := 1 - (1 - p(F23\`|\`K2\`\sim\`)) \cdot (1 - p(F23\`|\`K3\`\sim\`));$
$$p(F23\ \`|\`\ K2\ K1\ K3\ \overline{K4}) := 0.5 \tag{214}$$

> $p(F24\`|\`K2\overline{K1}K3\overline{K4}) := 1 - (1 - p(F24\`|\`K2\`\sim\`)) \cdot (1 - p(F24\`|\`K3\`\sim\`));$
$$p(F24\ \`|\`\ K2\ K1\ K3\ \overline{K4}) := 0.64 \tag{215}$$

> $p(F25\`|\`K2\overline{K1}K3\overline{K4}) := 1 - (1 - p(F25\`|\`K2\`\sim\`)) \cdot (1 - p(F25\`|\`K3\`\sim\`));$
$$p(F25\ \`|\`\ K2\ K1\ K3\ \overline{K4}) := 0.84 \tag{216}$$

> $p(F26\`|\`K2\overline{K1}K3\overline{K4}) := 1 - (1 - p(F26\`|\`K2\`\sim\`)) \cdot (1 - p(F26\`|\`K3\`\sim\`));$
$$p(F26\ \`|\`\ K2\ K1\ K3\ \overline{K4}) := 0.7 \tag{217}$$

> $b25 := (f21 \cdot p(F21\`|\`K2\overline{K1}K3\overline{K4}) + (1 - f21) \cdot (1 - p(F21\`|\`K2\overline{K1}K3\overline{K4}))) \cdot (f22 \cdot p(F22\`|\`K2\overline{K1}K3\overline{K4}) + (1 - f22) \cdot (1 - p(F22\`|\`K2\overline{K1}K3\overline{K4}))) \cdot (f23 \cdot p(F23\`|\`K2\overline{K1}K3\overline{K4}) + (1 - f23) \cdot (1 - p(F23\`|\`K2\overline{K1}K3 \overline{K4}))) \cdot (f24 \cdot p(F24\`|\`K2\overline{K1}K3\overline{K4}) + (1 - f24) \cdot (1 - p(F24\`|\`K2\overline{K1}K3\overline{K4}))) \cdot (f25 \cdot p(F25\`|\`K2\overline{K1}K3\overline{K4}) + (1 - f25) \cdot (1 - p(F25\`|\`K2\overline{K1}K3\overline{K4}))) \cdot (f26 \cdot p(F26\`|\`K2\overline{K1}K3\overline{K4}) + (1 - f26) \cdot (1 - p(F26\`|\`K2\overline{K1}K3$

$\overline{K4})\,)\,)\,;$

$$b25 := 0.0036 \tag{218}$$

> $p\left(F21`1`K2\overline{K1}\ \overline{K3}\,K4\right) := 1 - \left(1 - p\left(F21`1`K2`\text{~}`\right)\right)\cdot\left(1 - p\left(F21`1`K4`\text{~}`\right)\right);$

$$p\left(F21\ `1`\ K2\ K1\ K3\ K4\right) := 0.80 \tag{219}$$

> $p\left(F22`1`K2\overline{K1}\ \overline{K3}\,K4\right) := 1 - \left(1 - p\left(F22`1`K2`\text{~}`\right)\right)\cdot\left(1 - p\left(F22`1`K4`\text{~}`\right)\right);$

$$p\left(F22\ `1`\ K2\ K1\ K3\ K4\right) := 0.65 \tag{220}$$

> $p\left(F23`1`K2\overline{K1}\ \overline{K3}\,K4\right) := 1 - \left(1 - p\left(F23`1`K2`\text{~}`\right)\right)\cdot\left(1 - p\left(F23`1`K4`\text{~}`\right)\right);$

$$p\left(F23\ `1`\ K2\ K1\ K3\ K4\right) := 0.65 \tag{221}$$

> $p\left(F24`1`K2\overline{K1}\ \overline{K3}\,K4\right) := 1 - \left(1 - p\left(F24`1`K2`\text{~}`\right)\right)\cdot\left(1 - p\left(F24`1`K4`\text{~}`\right)\right);$

$$p\left(F24\ `1`\ K2\ K1\ K3\ K4\right) := 0.4 \tag{222}$$

> $p\left(F25`1`K2\overline{K1}\ \overline{K3}\,K4\right) := 1 - \left(1 - p\left(F25`1`K2`\text{~}`\right)\right)\cdot\left(1 - p\left(F25`1`K4`\text{~}`\right)\right);$

$$p\left(F25\ `1`\ K2\ K1\ K3\ K4\right) := 0.6 \tag{223}$$

> $p\left(F26`1`K2\overline{K1}\ \overline{K3}\,K4\right) := 1 - \left(1 - p\left(F26`1`K2`\text{~}`\right)\right)\cdot\left(1 - p\left(F26`1`K4`\text{~}`\right)\right);$

$$p\left(F26\ `1`\ K2\ K1\ K3\ K4\right) := 0.76 \tag{224}$$

> $b26 := \left(f21\cdot p\left(F21`1`K2\overline{K1}\overline{K3}K4\right) + \left(1 - f21\right)\cdot\left(1 - p\left(F21`1`K2\overline{K1}\overline{K3}K4\right)\right)\right)\cdot\left(f22\cdot p\left(F22`1`K2\overline{K1}\overline{K3}K4\right) + \left(1 - f22\right)\cdot\left(1 - p\left(F22`1`K2\overline{K1}\overline{K3}K4\right)\right)\right)\cdot\left(f23\cdot p\left(F23`1`K2\overline{K1}\overline{K3}K4\right) + \left(1 - f23\right)\cdot\left(1 - p\left(F23`1`K2\overline{K1}\ \overline{K3}K4\right)\right)\right)\cdot\left(f24\cdot p\left(F24`1`K2\overline{K1}\overline{K3}K4\right) + \left(1 - f24\right)\cdot\left(1 - p\left(F24`1`K2\overline{K1}\overline{K3}K4\right)\right)\right)\cdot\left(f25\cdot p\left(F25`1`K2\overline{K1}\ \overline{K3}K4\right) + \left(1 - f25\right)\cdot\left(1 - p\left(F25`1`K2\overline{K1}\overline{K3}K4\right)\right)\right)\cdot\left(f26\cdot p\left(F26`1`K2\overline{K1}\overline{K3}K4\right) + \left(1 - f26\right)\cdot\left(1 - p\left(F26`1`K2\overline{K1}\overline{K3}K4\right)\right)\right);$

$$b26 := 0.019 \tag{225}$$

> $p\left(F21`1`K2\overline{K1}\ \overline{K3}\,\overline{K4}\right) := p\left(F21`1`K2`\text{~}`\right);$

$$p\left(F21\ `1`\ K2\ \overline{K1}\ \overline{K3}\ \overline{K4}\right) := 0.6 \tag{226}$$

> $p\left(F22`1`K2\overline{K1}\ \overline{K3}\,\overline{K4}\right) := p\left(F22`1`K2`\text{~}`\right);$

$$p\left(F22\ `1`\ K2\ \overline{K1}\ \overline{K3}\ \overline{K4}\right) := 0.5 \tag{227}$$

> $p\left(F23`1`K2\overline{K1}\ \overline{K3}\,\overline{K4}\right) := p\left(F23`1`K2`\text{~}`\right);$

$$p\left(F23\ `1`\ K2\ \overline{K1}\ \overline{K3}\ \overline{K4}\right) := 0.5 \tag{228}$$

> $p\left(F24`1`K2\overline{K1}\ \overline{K3}\,\overline{K4}\right) := p\left(F24`1`K2`\text{~}`\right);$

$$p\left(F24\ `1`\ K2\ \overline{K1}\ \overline{K3}\ \overline{K4}\right) := 0.4 \tag{229}$$

> $p\left(F25`1`K2\overline{K1}\ \overline{K3}\,\overline{K4}\right) := p\left(F25`1`K2`\text{~}`\right);$

$$p\left(F25\ `1`\ K2\ \overline{K1}\ \overline{K3}\ \overline{K4}\right) := 0.6 \tag{230}$$

> $p\left(F26`1`K2\overline{K1}\ \overline{K3}\,\overline{K4}\right) := p\left(F26`1`K2`\text{~}`\right);$

$$p\left(F26\ `1`\ K2\ \overline{K1}\ \overline{K3}\ \overline{K4}\right) := 0.7 \tag{231}$$

> $b27 := \left(f21\cdot p\left(F21`1`K2\overline{K1}\overline{K3}\overline{K4}\right) + \left(1 - f21\right)\cdot\left(1 - p\left(F21`1`K2\overline{K1}\overline{K3}\overline{K4}\right)\right)\right)\cdot\left(f22\cdot p\left(F22`1`K2\overline{K1}\overline{K3}\overline{K4}\right) + \left(1 - f22\right)\cdot\left(1 - p\left(F22`1`K2\overline{K1}\overline{K3}\overline{K4}\right)\right)\right)\cdot\left(f23\cdot p\left(F23`1`K2\overline{K1}\overline{K3}\overline{K4}\right) + \left(1 - f23\right)\cdot\left(1 - p\left(F23`1`K2\overline{K1}\overline{K3}\,\overline{K4}\right)\right)\right)\cdot\left(f24\cdot p\left(F24`1`K2\overline{K1}\overline{K3}\overline{K4}\right) + \left(1 - f24\right)\cdot\left(1 - p\left(F24`1`K2\overline{K1}\overline{K3}\overline{K4}\right)\right)\right)\cdot\left(f25\cdot p\left(F25`1`K2\overline{K1}\overline{K3}\overline{K4}\right) + \left(1 - f25\right)\cdot\left(1 - p\left(F25`1`K2\overline{K1}\overline{K3}\overline{K4}\right)\right)\right)\cdot\left(f26\cdot p\left(F26`1`K2\overline{K1}\overline{K3}\overline{K4}\right) + \left(1 - f26\right)\cdot\left(1 - p\left(F26`1`K2\overline{K1}\overline{K3}\,\overline{K4}\right)\right)\right);$

$$b27 := 0.011 \tag{232}$$

> $c2 := 0.25;$

$$c2 := 0.25 \tag{233}$$

> $eq2 := x2 = 1 \Big/ \Big( 1 + \left(a20\cdot x1\cdot x3\cdot x4 + a21\cdot x1\cdot x3\cdot\left(1 - x4\right) + a22\cdot x1\cdot\left(1 - x3\right)\cdot x4 + a23\cdot x1\cdot\left(1 - x3\right)\cdot\left(1 - x4\right)\right.$

$\left. + 0 + 0 + 0 + 0 + 0 + 0 + 0 + 0 + 0 + a24\cdot\left(1 - x1\right)\cdot x3\cdot x4 + a25\cdot\left(1 - x1\right)\cdot x3\cdot\left(1 - x4\right) + a26\cdot\left(1\right.\right.$

$\left.\left. - x1\right)\cdot\left(1 - x3\right)\cdot x4 + a27\cdot\left(1 - x1\right)\cdot\left(1 - x3\right)\cdot\left(1 - x4\right)\right) \Big/ \left(b20\cdot x1\cdot x3\cdot x4 + b21\cdot x1\cdot x3\cdot\left(1 - x4\right) + b22\cdot x1\right.$

$\cdot (1-x3) \cdot x4 + b23 \cdot x1 \cdot (1-x3) \cdot (1-x4) + b24 \cdot (1-x1) \cdot x3 \cdot x4 + b25 \cdot (1-x1) \cdot x3 \cdot (1-x4) + b26 \cdot (1$

$-x1) \cdot (1-x3) \cdot x4 + b27 \cdot (1-x1) \cdot (1-x3) \cdot (1-x4)) \cdot \left( \dfrac{(1-c2)}{c2} \right) );$

$$eq2 := x2 = 1 \Big/ (1 + (3.0\ (0.023\ x1\ x3\ x4 + 0.014\ x1\ x3\ (1-x4) + 0.080\ x1\ (1-x3)\ x4 \tag{234}$$

$+ 0.045\ x1\ (1-x3)\ (1-x4) + 0.015\ (1-x1)\ x3\ x4 + 0.036\ (1-x1)\ (1-x3)\ x4))\Big/$

$(0.0029\ x1\ x3\ x4 + 0.0027\ x1\ x3\ (1-x4) + 0.011\ x1\ (1-x3)\ x4 + 0.010\ x1\ (1-x3)\ (1$

$-x4) + 0.0053\ (1-x1)\ x3\ x4 + 0.0036\ (1-x1)\ x3\ (1-x4) + 0.019\ (1-x1)\ (1$

$-x3)\ x4 + 0.011\ (1-x1)\ (1-x3)\ (1-x4)))$

> $f31 := S2; f32 := S4; f33 := S5; f34 := S6; f35 := S7; f36 := S9;$

$$f31 := 1$$
$$f32 := 1$$
$$f33 := 0$$
$$f34 := 0$$
$$f35 := 0$$
$$f36 := 0 \tag{235}$$

>
> $p(F31`|`K1`~`) := 0.5; p(F31`|`K2`~`) := 0.5; p(F31`|`K3`~`) := 0.3; p(F31`|`K4`~`) := 0.3;$

$$p(F31 `|` K1 `~`) := 0.5$$
$$p(F31 `|` K2 `~`) := 0.5$$
$$p(F31 `|` K3 `~`) := 0.3$$
$$p(F31 `|` K4 `~`) := 0.3 \tag{236}$$

> $p(F32`|`K1`~`) := 0.6; p(F32`|`K2`~`) := 0; p(F32`|`K3`~`) := 0.2; p(F32`|`K4`~`) := 0.5;$

$$p(F32 `|` K1 `~`) := 0.6$$
$$p(F32 `|` K2 `~`) := 0$$
$$p(F32 `|` K3 `~`) := 0.2$$
$$p(F32 `|` K4 `~`) := 0.5 \tag{237}$$

> $p(F33`|`K1`~`) := 0.9; p(F33`|`K2`~`) := 0; p(F33`|`K3`~`) := 0.5; p(F33`|`K4`~`) := 0;$

$$p(F33 `|` K1 `~`) := 0.9$$
$$p(F33 `|` K2 `~`) := 0$$
$$p(F33 `|` K3 `~`) := 0.5$$
$$p(F33 `|` K4 `~`) := 0 \tag{238}$$

> $p(F34`|`K1`~`) := 0.7; p(F34`|`K2`~`) := 0.4; p(F34`|`K3`~`) := 0.4; p(F34`|`K4`~`) := 0;$

$$p(F34 `|` K1 `~`) := 0.7$$
$$p(F34 `|` K2 `~`) := 0.4$$
$$p(F34 `|` K3 `~`) := 0.4$$
$$p(F34 `|` K4 `~`) := 0 \tag{239}$$

> $p(F35`|`K1`~`) := 0; p(F35`|`K2`~`) := 0.6; p(F35`|`K3`~`) := 0.6; p(F35`|`K4`~`) := 0;$

$$p(F35 `|` K1 `~`) := 0$$
$$p(F35 `|` K2 `~`) := 0.6$$
$$p(F35 `|` K3 `~`) := 0.6$$
$$p(F35 `|` K4 `~`) := 0 \tag{240}$$

> $p(F36`|`K1`~`) := 0; p(F36`|`K2`~`) := 0; p(F36`|`K3`~`) := 0.7; p(F36`|`K4`~`) := 0.4;$

$$p(F36 `|` K1 `~`) := 0$$

$$p(F36 \text{'} \text{'} K2 \text{'} \sim \text{'}) := 0$$
$$p(F36 \text{'} \text{'} K3 \text{'} \sim \text{'}) := 0.7$$
$$p(F36 \text{'} \text{'} K4 \text{'} \sim \text{'}) := 0.4 \tag{241}$$

$$> p(F31 \text{'} \text{'} \overline{K3} \, K2 \, K1 \, K4) := 1 - (1 - p(F31 \text{'} \text{'} \underline{K2} \text{'} \sim \text{'})) \cdot (1 - p(F31 \text{'} \text{'} K1 \text{'} \sim \text{'})) \cdot (1 - p(F31 \text{'} \text{'} K4 \text{'} \sim \text{'}));$$
$$p(F31 \text{'} \text{'} \overline{K3} \, K2 \, K1 \, K4) := 0.82 \tag{242}$$

$$> p(F32 \text{'} \text{'} \overline{K3} \, K2 \, K1 \, K4) := 1 - (1 - p(F32 \text{'} \text{'} \underline{K2} \text{'} \sim \text{'})) \cdot (1 - p(F32 \text{'} \text{'} K1 \text{'} \sim \text{'})) \cdot (1 - p(F32 \text{'} \text{'} K4 \text{'} \sim \text{'}));$$
$$p(F32 \text{'} \text{'} \overline{K3} \, K2 \, K1 \, K4) := 0.80 \tag{243}$$

$$> p(F33 \text{'} \text{'} \overline{K3} \, K2 \, K1 \, K4) := 1 - (1 - p(F33 \text{'} \text{'} \underline{K2} \text{'} \sim \text{'})) \cdot (1 - p(F33 \text{'} \text{'} K1 \text{'} \sim \text{'})) \cdot (1 - p(F33 \text{'} \text{'} K4 \text{'} \sim \text{'}));$$
$$p(F33 \text{'} \text{'} \overline{K3} \, K2 \, K1 \, K4) := 0.9 \tag{244}$$

$$> p(F34 \text{'} \text{'} \overline{K3} \, K2 \, K1 \, K4) := 1 - (1 - p(F34 \text{'} \text{'} \underline{K2} \text{'} \sim \text{'})) \cdot (1 - p(F34 \text{'} \text{'} K1 \text{'} \sim \text{'})) \cdot (1 - p(F34 \text{'} \text{'} K4 \text{'} \sim \text{'}));$$
$$p(F34 \text{'} \text{'} \overline{K3} \, K2 \, K1 \, K4) := 0.82 \tag{245}$$

$$> p(F35 \text{'} \text{'} \overline{K3} \, K2 \, K1 \, K4) := 1 - (1 - p(F35 \text{'} \text{'} \underline{K2} \text{'} \sim \text{'})) \cdot (1 - p(F35 \text{'} \text{'} K1 \text{'} \sim \text{'})) \cdot (1 - p(F35 \text{'} \text{'} K4 \text{'} \sim \text{'}));$$
$$p(F35 \text{'} \text{'} \overline{K3} \, K2 \, K1 \, K4) := 0.6 \tag{246}$$

$$> p(F36 \text{'} \text{'} \overline{K3} \, K2 \, K1 \, K4) := 1 - (1 - p(F36 \text{'} \text{'} \underline{K2} \text{'} \sim \text{'})) \cdot (1 - p(F36 \text{'} \text{'} K1 \text{'} \sim \text{'})) \cdot (1 - p(F36 \text{'} \text{'} K4 \text{'} \sim \text{'}));$$
$$p(F36 \text{'} \text{'} \overline{K3} \, K2 \, K1 \, K4) := 0.4 \tag{247}$$

$$> a30 := (f31 \cdot p(F31 \text{'} \text{'} \overline{K3} \, K2 \, K1 \, \underline{K4}) + (1 - f31) \cdot (1 - p(F31 \text{'} \text{'} \overline{K3} \, K2 \, K1 \, K4))) \cdot (f32 \cdot p(F32 \text{'} \text{'} \overline{K3} \, K2 \, K1 \, K4)$$
$$+ (1 - f32) \cdot (1 - p(F32 \text{'} \text{'} K3 \, \underline{K2} \, K1 \, K4))) \cdot (f33 \cdot p(F33 \text{'} \text{'} \overline{K3} \, K2 \, K1 \, K4) + (1 - f33) \cdot (1 - p(F33 \text{'} \text{'}$$
$$\overline{K3} \, K2 \, K1 \, K4))) \cdot (f34 \cdot p(F34 \text{'} \text{'} \overline{K3} \, K2 \, K1 \, \underline{K4}) + (1 - f34) \cdot (1 - p(F34 \text{'} \text{'} \overline{K3} \, K2 \, K1 \, K4))) \cdot (f35 \cdot p(F35 \text{'} \text{'}$$
$$\overline{K3} \, K2 \, K1 \, \underline{K4}) + (1 - f35) \cdot (1 - p(F35 \text{'} \text{'} \overline{K3} \, K2 \, K1 \, K4))) \cdot (f36 \cdot p(F36 \text{'} \text{'} \overline{K3} \, K2 \, K1 \, K4) + (1 - f36) \cdot (1$$
$$- p(F36 \text{'} \text{'} \overline{K3} \, K2 \, K1 \, K4)));$$
$$a30 := 0.0029 \tag{248}$$

$$> p(F31 \text{'} \text{'} \overline{K3} \, K2 \, K1 \, \overline{K4}) := 1 - (1 - p(F31 \text{'} \text{'} \underline{K2} \text{'} \sim \text{'})) \cdot (1 - \underline{p}(F31 \text{'} \text{'} K1 \text{'} \sim \text{'}));$$
$$p(F31 \text{'} \text{'} \overline{K3} \, K2 \, K1 \, \overline{K4}) := 0.75 \tag{249}$$

$$> p(F32 \text{'} \text{'} \overline{K3} \, K2 \, K1 \, \overline{K4}) := 1 - (1 - p(F32 \text{'} \text{'} \underline{K2} \text{'} \sim \text{'})) \cdot (1 - \underline{p}(F32 \text{'} \text{'} K1 \text{'} \sim \text{'}));$$
$$p(F32 \text{'} \text{'} \overline{K3} \, K2 \, K1 \, \overline{K4}) := 0.6 \tag{250}$$

$$> p(F33 \text{'} \text{'} \overline{K3} \, K2 \, K1 \, \overline{K4}) := 1 - (1 - p(F33 \text{'} \text{'} \underline{K2} \text{'} \sim \text{'})) \cdot (1 - \underline{p}(F33 \text{'} \text{'} K1 \text{'} \sim \text{'}));$$
$$p(F33 \text{'} \text{'} \overline{K3} \, K2 \, K1 \, \overline{K4}) := 0.9 \tag{251}$$

$$> p(F34 \text{'} \text{'} \overline{K3} \, K2 \, K1 \, \overline{K4}) := 1 - (1 - p(F34 \text{'} \text{'} \underline{K2} \text{'} \sim \text{'})) \cdot (1 - \underline{p}(F34 \text{'} \text{'} K1 \text{'} \sim \text{'}));$$
$$p(F34 \text{'} \text{'} \overline{K3} \, K2 \, K1 \, \overline{K4}) := 0.82 \tag{252}$$

$$> p(F35 \text{'} \text{'} \overline{K3} \, K2 \, K1 \, \overline{K4}) := 1 - (1 - p(F35 \text{'} \text{'} \underline{K2} \text{'} \sim \text{'})) \cdot (1 - \underline{p}(F35 \text{'} \text{'} K1 \text{'} \sim \text{'}));$$
$$p(F35 \text{'} \text{'} \overline{K3} \, K2 \, K1 \, \overline{K4}) := 0.6 \tag{253}$$

$$> p(F36 \text{'} \text{'} \overline{K3} \, K2 \, K1 \, \overline{K4}) := 1 - (1 - p(F36 \text{'} \text{'} \underline{K2} \text{'} \sim \text{'})) \cdot (1 - \underline{p}(F36 \text{'} \text{'} K1 \text{'} \sim \text{'}));$$
$$p(F36 \text{'} \text{'} \overline{K3} \, K2 \, K1 \, \overline{K4}) := 0 \tag{254}$$

$$> a31 := (f31 \cdot p(F31 \text{'} \text{'} \overline{K3} \, K2 \, K1 \, \overline{K4}) + (1 - f31) \cdot (1 - p(F31 \text{'} \text{'} \overline{K3} \, K2 \, K1 \, \overline{K4}))) \cdot (f32 \cdot p(F32 \text{'} \text{'} \overline{K3} \, K2 \, K1 \, \overline{K4})$$
$$+ (1 - f32) \cdot (1 - p(F32 \text{'} \text{'} \overline{K3} \, K2 \, K1 \, \overline{K4}))) \cdot (f33 \cdot p(F33 \text{'} \text{'} \overline{K3} \, K2 \, K1 \, \overline{K4}) + (1 - f33) \cdot (1 - p(F33 \text{'} \text{'}$$
$$\overline{K3} \, K2 \, K1 \, \overline{K4}))) \cdot (f34 \cdot p(F34 \text{'} \text{'} \overline{K3} \, K2 \, K1 \, \overline{K4}) + (1 - f34) \cdot (1 - p(F34 \text{'} \text{'} \overline{K3} \, K2 \, K1 \, \overline{K4}))) \cdot (f35 \cdot p(F35 \text{'} \text{'}$$
$$\overline{K3} \, K2 \, K1 \, \overline{K4}) + (1 - f35) \cdot (1 - p(F35 \text{'} \text{'} \overline{K3} \, K2 \, K1 \, \overline{K4}))) \cdot (f36 \cdot p(F36 \text{'} \text{'} \overline{K3} \, K2 \, K1 \, \overline{K4}) + (1 - f36) \cdot (1$$
$$- p(F36 \text{'} \text{'} \overline{K3} \, K2 \, K1 \, \overline{K4})));$$
$$a31 := 0.0032 \tag{255}$$

$$> p(F31 \text{'} \text{'} \overline{K3} \, K2 \, \overline{K1} \, K4) := 1 - (1 - p(F31 \text{'} \text{'} \underline{K2} \text{'} \sim \text{'})) \cdot \underline{(1} - p(F31 \text{'} \text{'} K4 \text{'} \sim \text{'}));$$
$$p(F31 \text{'} \text{'} \overline{K3} \, K2 \, \overline{K1} \, K4) := 0.65 \tag{256}$$

$$> p(F32 \text{'} \text{'} \overline{K3} \, K2 \, \overline{K1} \, K4) := 1 - (1 - p(F32 \text{'} \text{'} \underline{K2} \text{'} \sim \text{'})) \cdot \underline{(1} - p(F32 \text{'} \text{'} K4 \text{'} \sim \text{'}));$$
$$p(F32 \text{'} \text{'} \overline{K3} \, K2 \, \overline{K1} \, K4) := 0.5 \tag{257}$$

$$> p(F33 \text{'} \text{'} \overline{K3} \, K2 \, \overline{K1} \, K4) := 1 - (1 - p(F33 \text{'} \text{'} \underline{K2} \text{'} \sim \text{'})) \cdot \underline{(1} - p(F33 \text{'} \text{'} K4 \text{'} \sim \text{'}));$$
$$p(F33 \text{'} \text{'} \overline{K3} \, K2 \, \overline{K1} \, K4) := 0 \tag{258}$$

> $p\left(F34`|`\overline{K3}\ K2\ \overline{K1}\ K4\right) := 1 - \left(1 - p\left(F34`|`K2`\sim`\right)\right)\cdot\left(1 - p\left(F34`|`K4`\sim`\right)\right);$
$$p\left(F34\ `|`\ \overline{K3}\ K2\ \overline{K1}\ K4\right) := 0.4 \qquad\qquad (259)$$

> $p\left(F35`|`\overline{K3}\ K2\ \overline{K1}\ K4\right) := 1 - \left(1 - p\left(F35`|`K2`\sim`\right)\right)\cdot\left(1 - p\left(F35`|`K4`\sim`\right)\right);$
$$p\left(F35\ `|`\ \overline{K3}\ K2\ \overline{K1}\ K4\right) := 0.6 \qquad\qquad (260)$$

> $p\left(F36`|`\overline{K3}\ K2\ \overline{K1}\ K4\right) := 1 - \left(1 - p\left(F36`|`K2`\sim`\right)\right)\cdot\left(1 - p\left(F36`|`K4`\sim`\right)\right);$
$$p\left(F36\ `|`\ \overline{K3}\ K2\ \overline{K1}\ K4\right) := 0.4 \qquad\qquad (261)$$

> $a32 := \left(f31\cdot p\left(F31`|`\overline{K3}\ K2\ \overline{K1}\ K4\right) + \left(1 - f31\right)\cdot\left(1 - p\left(F31`|`\overline{K3}\ K2\ \overline{K1}\ K4\right)\right)\right)\cdot\left(f32\cdot p\left(F32`|`\overline{K3}\ K2\ \overline{K1}\ K4\right)\right.$
$+ \left(1 - f32\right)\cdot\left(1 - p\left(F32`|`\overline{K3}\ K2\ \overline{K1}\ K4\right)\right)\right)\cdot\left(f33\cdot p\left(F33`|`\overline{K3}\ K2\ \overline{K1}\ K4\right) + \left(1 - f33\right)\cdot\left(1 - p\left(F33`|`\overline{K3}\ K2\ \right.\right.$
$\left.\left.\overline{K1}\ K4\right)\right)\right)\cdot\left(f34\cdot p\left(F34`|`\overline{K3}\ K2\ \overline{K1}\ K4\right) + \left(1 - f34\right)\cdot\left(1 - p\left(F34`|`\overline{K3}\ K2\ \overline{K1}\ K4\right)\right)\right)\cdot\left(f35\cdot p\left(F35`|`\overline{K3}\ K2\ \right.\right.$
$\left.\left.\overline{K1}\ K4\right) + \left(1 - f35\right)\cdot\left(1 - p\left(F35`|`\overline{K3}\ K2\ \overline{K1}\ K4\right)\right)\right)\cdot\left(f36\cdot p\left(F36`|`\overline{K3}\ K2\ \overline{K1}\ K4\right) + \left(1 - f36\right)\cdot\left(1 - p\left(F36`|`\right.\right.\right.$
$\left.\left.\left.\overline{K3}\ K2\ \overline{K1}\ K4\right)\right)\right);$
$$a32 := 0.046 \qquad\qquad (262)$$

> $p\left(F31`|`\overline{K3}\ K2\ \overline{K1}\ \overline{K4}\right) := p\left(F31`|`K2`\sim`\right);$
$$p\left(F31\ `|`\ \overline{K3}\ K2\ \overline{K1}\ \overline{K4}\right) := 0.5 \qquad\qquad (263)$$

> $p\left(F32`|`\overline{K3}\ K2\ \overline{K1}\ \overline{K4}\right) := p\left(F32`|`K2`\sim`\right);$
$$p\left(F32\ `|`\ \overline{K3}\ K2\ \overline{K1}\ \overline{K4}\right) := 0 \qquad\qquad (264)$$

> $p\left(F33`|`\overline{K3}\ K2\ \overline{K1}\ \overline{K4}\right) := p\left(F33`|`K2`\sim`\right);$
$$p\left(F33\ `|`\ \overline{K3}\ K2\ \overline{K1}\ \overline{K4}\right) := 0 \qquad\qquad (265)$$

> $p\left(F34`|`\overline{K3}\ K2\ \overline{K1}.K4\right) := p\left(F34`|`K2`\sim`\right);$
$$p\left(F34\ `|`\ \overline{K3}\ K2\ \overline{K1}\ \overline{K4}\right) := 0.4 \qquad\qquad (266)$$

> $p\left(F35`|`\overline{K3}\ K2\ \overline{K1}\ \overline{K4}\right) := p\left(F35`|`K2`\sim`\right);$
$$p\left(F35\ `|`\ \overline{K3}\ K2\ \overline{K1}\ \overline{K4}\right) := 0.6 \qquad\qquad (267)$$

> $p\left(F36`|`\overline{K3}\ K2\ \overline{K1}\ \overline{K4}\right) := p\left(F36`|`K2`\sim`\right);$
$$p\left(F36\ `|`\ \overline{K3}\ K2\ \overline{K1}\ \overline{K4}\right) := 0 \qquad\qquad (268)$$

> $a33 := \left(f31\cdot p\left(F31`|`\overline{K3}\ K2\ \overline{K1}\ \overline{K4}\right) + \left(1 - f31\right)\cdot\left(1 - p\left(F31`|`\overline{K3}\ K2\ \overline{K1}\ \overline{K4}\right)\right)\right)\cdot\left(f32\cdot p\left(F32`|`\overline{K3}\ K2\ \overline{K1}\ \overline{K4}\right)\right.$
$+ \left(1 - f32\right)\cdot\left(1 - p\left(F32`|`\overline{K3}\ K2\ \overline{K1}\ \overline{K4}\right)\right)\right)\cdot\left(f33\cdot p\left(F33`|`\overline{K3}\ K2\ \overline{K1}\ \overline{K4}\right) + \left(1 - f33\right)\cdot\left(1 - p\left(F33`|`\overline{K3}\ K2\ \right.\right.$
$\left.\left.\overline{K1}\ \overline{K4}\right)\right)\right)\cdot\left(f34\cdot p\left(F34`|`\overline{K3}\ K2\ \overline{K1}\ \overline{K4}\right) + \left(1 - f34\right)\cdot\left(1 - p\left(F34`|`\overline{K3}\ K2\ \overline{K1}\ \overline{K4}\right)\right)\right)\cdot\left(f35\cdot p\left(F35`|`\overline{K3}\ K2\ \overline{K1}\ \right.\right.$
$\left.\left.\overline{K4}\right) + \left(1 - f35\right)\cdot\left(1 - p\left(F35`|`\overline{K3}\ K2\ \overline{K1}\ \overline{K4}\right)\right)\right)\cdot\left(f36\cdot p\left(F36`|`\overline{K3}\ K2\ \overline{K1}\ \overline{K4}\right) + \left(1 - f36\right)\cdot\left(1 - p\left(F36`|`\right.\right.\right.$
$\left.\left.\left.\overline{K3}\ K2\ \overline{K1}\ \overline{K4}\right)\right)\right);$
$$a33 := 0. \qquad\qquad (269)$$

> $p\left(F31`|`\overline{K3}\ \overline{K2}\ K1\ K4\right) := 1 - \left(1 - p\left(F31`|`K1`\sim`\right)\right)\cdot\left(1 - p\left(F31`|`K4`\sim`\right)\right);$
$$p\left(F31\ `|`\ \overline{K3}\ \overline{K2}\ K1\ K4\right) := 0.65 \qquad\qquad (270)$$

> $p\left(F32`|`\overline{K3}\ \overline{K2}\ K1\ K4\right) := 1 - \left(1 - p\left(F32`|`K1`\sim`\right)\right)\cdot\left(1 - p\left(F32`|`K4`\sim`\right)\right);$
$$p\left(F32\ `|`\ \overline{K3}\ \overline{K2}\ K1\ K4\right) := 0.80 \qquad\qquad (271)$$

> $p\left(F33`|`\overline{K3}\ \overline{K2}\ K1\ K4\right) := 1 - \left(1 - p\left(F33`|`K1`\sim`\right)\right)\cdot\left(1 - p\left(F33`|`K4`\sim`\right)\right);$
$$p\left(F33\ `|`\ \overline{K3}\ \overline{K2}\ K1\ K4\right) := 0.9 \qquad\qquad (272)$$

> $p\left(F34`|`\overline{K3}\ \overline{K2}\ K1\ K4\right) := 1 - \left(1 - p\left(F34`|`K1`\sim`\right)\right)\cdot\left(1 - p\left(F34`|`K4`\sim`\right)\right);$
$$p\left(F34\ `|`\ \overline{K3}\ \overline{K2}\ K1\ K4\right) := 0.7 \qquad\qquad (273)$$

> $p\left(F35`|`\overline{K3}\ \overline{K2}\ K1\ K4\right) := 1 - \left(1 - p\left(F35`|`K1`\sim`\right)\right)\cdot\left(1 - p\left(F35`|`K4`\sim`\right)\right);$
$$p\left(F35\ `|`\ \overline{K3}\ \overline{K2}\ K1\ K4\right) := 0 \qquad\qquad (274)$$

> $p\left(F36`|`\overline{K3}\ \overline{K2}\ K1\ K4\right) := 1 - \left(1 - p\left(F36`|`K1`\sim`\right)\right)\cdot\left(1 - p\left(F36`|`K4`\sim`\right)\right);$
$$p\left(F36\ `|`\ \overline{K3}\ \overline{K2}\ K1\ K4\right) := 0.4 \qquad\qquad (275)$$

> $a34 := \left(f31\cdot p\left(F31`|`\overline{K3}\ \overline{K2}\ K1\ K4\right) + \left(1 - f31\right)\cdot\left(1 - p\left(F31`|`\overline{K3}\ \overline{K2}\ K1\ K4\right)\right)\right)\cdot\left(f32\cdot p\left(F32`|`\overline{K3}\ \overline{K2}\ K1\ K4\right)\right.$
$+ \left(1 - f32\right)\cdot\left(1 - p\left(F32`|`\overline{K3}\ \overline{K2}\ K1\ K4\right)\right)\right)\cdot\left(f33\cdot p\left(F33`|`\overline{K3}\ \overline{K2}\ K1\ K4\right) + \left(1 - f33\right)\cdot\left(1 - p\left(F33`|`\overline{K3}\ \right.\right.$
$\left.\left.\overline{K2}\ K1\ K4\right)\right)\right)\cdot\left(f34\cdot p\left(F34`|`\overline{K3}\ \overline{K2}\ K1\ K4\right) + \left(1 - f34\right)\cdot\left(1 - p\left(F34`|`\overline{K3}\ \overline{K2}\ K1\ K4\right)\right)\right)\cdot\left(f35\cdot p\left(F35`|`\overline{K3}\ \right.\right.$
$\left.\left.\overline{K2}\ K1\ K4\right) + \left(1 - f35\right)\cdot\left(1 - p\left(F35`|`\overline{K3}\ \overline{K2}\ K1\ K4\right)\right)\right)\cdot\left(f36\cdot p\left(F36`|`\overline{K3}\ \overline{K2}\ K1\ K4\right) + \left(1 - f36\right)\cdot\left(1\right.\right.$

$$-p\left(F36\,|\,\overline{K3}\ \overline{K2}\,K1\,K4\right)\right)\right);$$

$$a34 := 0.0096 \tag{276}$$

$$> p\left(F31\,|\,\overline{K3}\ \overline{K2}\,K1\ \overline{K4}\right) := p\left(F31\,|\,K1\,\text{\textasciitilde}\right);$$
$$p\left(F31\,|\,\overline{K3}\ \overline{K2}\,K1\ \overline{K4}\right) := 0.5 \tag{277}$$

$$> p\left(F32\,|\,\overline{K3}\ \overline{K2}\,K1\ \overline{K4}\right) := p\left(F32\,|\,K1\,\text{\textasciitilde}\right);$$
$$p\left(F32\,|\,\overline{K3}\ \overline{K2}\,K1\ \overline{K4}\right) := 0.6 \tag{278}$$

$$> p\left(F33\,|\,\overline{K3}\ \overline{K2}\,K1\ \overline{K4}\right) := p\left(F33\,|\,K1\,\text{\textasciitilde}\right);$$
$$p\left(F33\,|\,\overline{K3}\ \overline{K2}\,K1\ \overline{K4}\right) := 0.9 \tag{279}$$

$$> p\left(F34\,|\,\overline{K3}\ \overline{K2}\,K1\ \overline{K4}\right) := p\left(F34\,|\,K1\,\text{\textasciitilde}\right);$$
$$p\left(F34\,|\,\overline{K3}\ \overline{K2}\,K1\ \overline{K4}\right) := 0.7 \tag{280}$$

$$> p\left(F35\,|\,\overline{K3}\ \overline{K2}\,K1\ \overline{K4}\right) := p\left(F35\,|\,K1\,\text{\textasciitilde}\right);$$
$$p\left(F35\,|\,\overline{K3}\ \overline{K2}\,K1\ \overline{K4}\right) := 0 \tag{281}$$

$$> p\left(F36\,|\,\overline{K3}\ \overline{K2}\,K1\ \overline{K4}\right) := p\left(F36\,|\,K1\,\text{\textasciitilde}\right);$$
$$p\left(F36\,|\,\overline{K3}\ \overline{K2}\,K1\ \overline{K4}\right) := 0 \tag{282}$$

$$> a35 := \left(f31 \cdot p\left(F31\,|\,\overline{K3}\ \overline{K2}\,K1\,\overline{K4}\right) + (1 - f31)\cdot\left(1 - p\left(F31\,|\,\overline{K3}\ \overline{K2}\,K1\,\overline{K4}\right)\right)\right)\cdot\left(f32 \cdot p\left(F32\,|\,\overline{K3}\ \overline{K2}\,K1\,\overline{K4}\right)\right.$$
$$+ (1 - f32)\cdot\left(1 - p\left(F32\,|\,\overline{K3}\ \overline{K2}\,K1\,\overline{K4}\right)\right)\right)\cdot\left(f33 \cdot p\left(F33\,|\,\overline{K3}\ \overline{K2}\,K1\,\overline{K4}\right) + (1 - f33)\cdot\left(1 - p\left(F33\,|\,\overline{K3}\right.\right.\right.$$
$$\left.\left.\left.\overline{K2}\,K1\,\overline{K4}\right)\right)\right)\cdot\left(f34 \cdot p\left(F34\,|\,\overline{K3}\ \overline{K2}\,K1\,\overline{K4}\right) + (1 - f34)\cdot\left(1 - p\left(F34\,|\,\overline{K3}\ \overline{K2}\,K1\,\overline{K4}\right)\right)\right)\cdot\left(f35 \cdot p\left(F35\,|\,\overline{K3}\right.\right.$$
$$\left.\overline{K2}\,K1\,\overline{K4}\right) + (1 - f35)\cdot\left(1 - p\left(F35\,|\,\overline{K3}\ \overline{K2}\,K1\,\overline{K4}\right)\right)\right)\cdot\left(f36 \cdot p\left(F36\,|\,\overline{K3}\ \overline{K2}\,K1\,\overline{K4}\right) + (1 - f36)\cdot\left(1\right.\right.$$
$$\left.\left.- p\left(F36\,|\,\overline{K3}\ \overline{K2}\,K1\,\overline{K4}\right)\right)\right);$$

$$a35 := 0.0090 \tag{283}$$

$$> p\left(F31\,|\,\overline{K3}\ \overline{K2}\,\overline{K1}\,K4\right) := p\left(F31\,|\,K4\,\text{\textasciitilde}\right);$$
$$p\left(F31\,|\,\overline{K3}\ \overline{K2}\,\overline{K1}\,K4\right) := 0.3 \tag{284}$$

$$> p\left(F32\,|\,\overline{K3}\ \overline{K2}\,\overline{K1}\,K4\right) := p\left(F32\,|\,K4\,\text{\textasciitilde}\right);$$
$$p\left(F32\,|\,\overline{K3}\ \overline{K2}\,\overline{K1}\,K4\right) := 0.5 \tag{285}$$

$$> p\left(F33\,|\,\overline{K3}\ \overline{K2}\,\overline{K1}\,K4\right) := p\left(F33\,|\,K4\,\text{\textasciitilde}\right);$$
$$p\left(F33\,|\,\overline{K3}\ \overline{K2}\,\overline{K1}\,K4\right) := 0 \tag{286}$$

$$> p\left(F34\,|\,\overline{K3}\ \overline{K2}\,\overline{K1}\,K4\right) := p\left(F34\,|\,K4\,\text{\textasciitilde}\right);$$
$$p\left(F34\,|\,\overline{K3}\ \overline{K2}\,\overline{K1}\,K4\right) := 0 \tag{287}$$

$$> p\left(F35\,|\,\overline{K3}\ \overline{K2}\,\overline{K1}\,K4\right) := p\left(F35\,|\,K4\,\text{\textasciitilde}\right);$$
$$p\left(F35\,|\,\overline{K3}\ \overline{K2}\,\overline{K1}\,K4\right) := 0 \tag{288}$$

$$> p\left(F36\,|\,\overline{K3}\ \overline{K2}\,\overline{K1}\,K4\right) := p\left(F36\,|\,K4\,\text{\textasciitilde}\right);$$
$$p\left(F36\,|\,\overline{K3}\ \overline{K2}\,\overline{K1}\,K4\right) := 0.4 \tag{289}$$

$$> a36 := \left(f31 \cdot p\left(F31\,|\,\overline{K3}\ \overline{K2}\,\overline{K1}\,K4\right) + (1 - f31)\cdot\left(1 - p\left(F31\,|\,\overline{K3}\ \overline{K2}\,\overline{K1}\,K4\right)\right)\right)\cdot\left(f32 \cdot p\left(F32\,|\,\overline{K3}\ \overline{K2}\,\overline{K1}\,K4\right)\right.$$
$$+ (1 - f32)\cdot\left(1 - p\left(F32\,|\,\overline{K3}\ \overline{K2}\,\overline{K1}\,K4\right)\right)\right)\cdot\left(f33 \cdot p\left(F33\,|\,\overline{K3}\ \overline{K2}\,\overline{K1}\,K4\right) + (1 - f33)\cdot\left(1 - p\left(F33\,|\,\overline{K3}\ \overline{K2}\right.\right.\right.$$
$$\left.\left.\left.\overline{K1}\,K4\right)\right)\right)\cdot\left(f34 \cdot p\left(F34\,|\,\overline{K3}\ \overline{K2}\,\overline{K1}\,K4\right) + (1 - f34)\cdot\left(1 - p\left(F34\,|\,\overline{K3}\ \overline{K2}\,\overline{K1}\,K4\right)\right)\right)\cdot\left(f35 \cdot p\left(F35\,|\,\overline{K3}\ \overline{K2}\right.\right.$$
$$\left.\overline{K1}\,K4\right) + (1 - f35)\cdot\left(1 - p\left(F35\,|\,\overline{K3}\ \overline{K2}\,\overline{K1}\,K4\right)\right)\right)\cdot\left(f36 \cdot p\left(F36\,|\,\overline{K3}\ \overline{K2}\,\overline{K1}\,K4\right) + (1 - f36)\cdot\left(1 - p\left(F36\,|\,\right.\right.\right.$$
$$\left.\left.\left.\overline{K3}\ \overline{K2}\,\overline{K1}\,K4\right)\right)\right);$$

$$a36 := 0.090 \tag{290}$$

$$> a37 := 0;$$
$$a37 := 0 \tag{291}$$

$$> p\left(F31\,|\,K3\,K2\,K1\,K4\right) := 1 - \left(1 - p\left(F31\,|\,K1\,\text{\textasciitilde}\right)\right)\cdot\left(1 - p\left(F31\,|\,K2\,\text{\textasciitilde}\right)\right)\cdot\left(1 - p\left(F31\,|\,K3\,\text{\textasciitilde}\right)\right)\cdot\left(1\right.$$
$$\left.- p\left(F31\,|\,K4\,\text{\textasciitilde}\right)\right);$$
$$p\left(F31\,|\,K3\,K2\,K1\,K4\right) := 0.87 \tag{292}$$

$$> p\left(F32\,|\,K3\,K2\,K1\,K4\right) := 1 - \left(1 - p\left(F32\,|\,K1\,\text{\textasciitilde}\right)\right)\cdot\left(1 - p\left(F32\,|\,K2\,\text{\textasciitilde}\right)\right)\cdot\left(1 - p\left(F32\,|\,K3\,\text{\textasciitilde}\right)\right)\cdot\left(1\right.$$
$$\left.- p\left(F32\,|\,K4\,\text{\textasciitilde}\right)\right);$$
$$p\left(F32\,|\,K3\,K2\,K1\,K4\right) := 0.84 \tag{293}$$

> $p(F33 \cap K3\,K2\,K1\,K4) := 1 - (1 - p(F33 \cap K1 \sim)) \cdot (1 - p(F33 \cap K2 \sim)) \cdot (1 - p(F33 \cap K3 \sim)) \cdot (1 - p(F33 \cap K4 \sim));$

$$p(F33 \cap K3\,K2\,K1\,K4) := 0.95 \tag{294}$$

> $p(F34 \cap K3\,K2\,K1\,K4) := 1 - (1 - p(F34 \cap K1 \sim)) \cdot (1 - p(F34 \cap K2 \sim)) \cdot (1 - p(F34 \cap K3 \sim)) \cdot (1 - p(F34 \cap K4 \sim));$

$$p(F34 \cap K3\,K2\,K1\,K4) := 0.89 \tag{295}$$

> $p(F35 \cap K3\,K2\,K1\,K4) := 1 - (1 - p(F35 \cap K1 \sim)) \cdot (1 - p(F35 \cap K2 \sim)) \cdot (1 - p(F35 \cap K3 \sim)) \cdot (1 - p(F35 \cap K4 \sim));$

$$p(F35 \cap K3\,K2\,K1\,K4) := 0.84 \tag{296}$$

> $p(F36 \cap K3\,K2\,K1\,K4) := 1 - (1 - p(F36 \cap K1 \sim)) \cdot (1 - p(F36 \cap K2 \sim)) \cdot (1 - p(F36 \cap K3 \sim)) \cdot (1 - p(F36 \cap K4 \sim));$

$$p(F36 \cap K3\,K2\,K1\,K4) := 0.82 \tag{297}$$

> $b30 := (f31 \cdot p(F31 \cap K3\,K2\,K1\,K4) + (1 - f31) \cdot (1 - p(F31 \cap K3\,K2\,K1\,K4))) \cdot (f32 \cdot p(F32 \cap K3\,K2\,K1\,K4) + (1 - f32) \cdot (1 - p(F32 \cap K3\,K2\,K1\,K4))) \cdot (f33 \cdot p(F33 \cap K3\,K2\,K1\,K4) + (1 - f33) \cdot (1 - p(F33 \cap K3\,K2\,K1\,K4))) \cdot (f34 \cdot p(F34 \cap K3\,K2\,K1\,K4) + (1 - f34) \cdot (1 - p(F34 \cap K3\,K2\,K1\,K4))) \cdot (f35 \cdot p(F35 \cap K3\,K2\,K1\,K4) + (1 - f35) \cdot (1 - p(F35 \cap K3\,K2\,K1\,K4))) \cdot (f36 \cdot p(F36 \cap K3\,K2\,K1\,K4) + (1 - f36) \cdot (1 - p(F36 \cap K3\,K2\,K1\,K4)));$

$$b30 := 0.00012 \tag{298}$$

> $p(F31 \cap K3\,K2\,K1\,\overline{K4}) := 1 - (1 - p(F31 \cap K1 \sim)) \cdot (1 - p(F31 \cap K2 \sim)) \cdot (1 - p(F31 \cap K3 \sim));$
$$p(F31 \cap K3\,K2\,K1\,\overline{K4}) := 0.82 \tag{299}$$

> $p(F32 \cap K3\,K2\,K1\,\overline{K4}) := 1 - (1 - p(F32 \cap K1 \sim)) \cdot (1 - p(F32 \cap K2 \sim)) \cdot (1 - p(F32 \cap K3 \sim));$
$$p(F32 \cap K3\,K2\,K1\,\overline{K4}) := 0.68 \tag{300}$$

> $p(F33 \cap K3\,K2\,K1\,\overline{K4}) := 1 - (1 - p(F33 \cap K1 \sim)) \cdot (1 - p(F33 \cap K2 \sim)) \cdot (1 - p(F33 \cap K3 \sim));$
$$p(F33 \cap K3\,K2\,K1\,\overline{K4}) := 0.95 \tag{301}$$

> $p(F34 \cap K3\,K2\,K1\,\overline{K4}) := 1 - (1 - p(F34 \cap K1 \sim)) \cdot (1 - p(F34 \cap K2 \sim)) \cdot (1 - p(F34 \cap K3 \sim));$
$$p(F34 \cap K3\,K2\,K1\,\overline{K4}) := 0.89 \tag{302}$$

> $p(F35 \cap K3\,K2\,K1\,\overline{K4}) := 1 - (1 - p(F35 \cap K1 \sim)) \cdot (1 - p(F35 \cap K2 \sim)) \cdot (1 - p(F35 \cap K3 \sim));$
$$p(F35 \cap K3\,K2\,K1\,\overline{K4}) := 0.84 \tag{303}$$

> $p(F36 \cap K3\,K2\,K1\,\overline{K4}) := 1 - (1 - p(F36 \cap K1 \sim)) \cdot (1 - p(F36 \cap K2 \sim)) \cdot (1 - p(F36 \cap K3 \sim));$
$$p(F36 \cap K3\,K2\,K1\,\overline{K4}) := 0.7 \tag{304}$$

> $b31 := (f31 \cdot p(F31 \cap K3\,K2\,K1\,\overline{K4}) + (1 - f31) \cdot (1 - p(F31 \cap K3\,K2\,K1\,\overline{K4}))) \cdot (f32 \cdot p(F32 \cap K3\,K2\,K1\,\overline{K4}) + (1 - f32) \cdot (1 - p(F32 \cap K3\,K2\,K1\,\overline{K4}))) \cdot (f33 \cdot p(F33 \cap K3\,K2\,K1\,\overline{K4}) + (1 - f33) \cdot (1 - p(F33 \cap K3\,K2\,K1\,\overline{K4}))) \cdot (f34 \cdot p(F34 \cap K3\,K2\,K1\,\overline{K4}) + (1 - f34) \cdot (1 - p(F34 \cap K3\,K2\,K1\,\overline{K4}))) \cdot (f35 \cdot p(F35 \cap K3\,K2\,K1\,\overline{K4}) + (1 - f35) \cdot (1 - p(F35 \cap K3\,K2\,K1\,\overline{K4}))) \cdot (f36 \cdot p(F36 \cap K3\,K2\,K1\,\overline{K4}) + (1 - f36) \cdot (1 - p(F36 \cap K3\,K2\,K1\,\overline{K4})));$

$$b31 := 0.00015 \tag{305}$$

> $p(F31 \cap K3\,K2\,\overline{K1}\,K4) := 1 - (1 - p(F31 \cap K3 \sim)) \cdot (1 - p(F31 \cap K2 \sim)) \cdot (1 - p(F31 \cap K4 \sim));$
$$p(F31 \cap K3\,K2\,\overline{K1}\,K4) := 0.76 \tag{306}$$

> $p(F32 \cap K3\,K2\,\overline{K1}\,K4) := 1 - (1 - p(F32 \cap K3 \sim)) \cdot (1 - p(F32 \cap K2 \sim)) \cdot (1 - p(F32 \cap K4 \sim));$
$$p(F32 \cap K3\,K2\,\overline{K1}\,K4) := 0.60 \tag{307}$$

> $p(F33 \cap K3\,K2\,\overline{K1}\,K4) := 1 - (1 - p(F33 \cap K3 \sim)) \cdot (1 - p(F33 \cap K2 \sim)) \cdot (1 - p(F33 \cap K4 \sim));$
$$p(F33 \cap K3\,K2\,\overline{K1}\,K4) := 0.5 \tag{308}$$

> $p(F34 \cap K3\,K2\,\overline{K1}\,K4) := 1 - (1 - p(F34 \cap K3 \sim)) \cdot (1 - p(F34 \cap K2 \sim)) \cdot (1 - p(F34 \cap K4 \sim));$
$$p(F34 \cap K3\,K2\,\overline{K1}\,K4) := 0.64 \tag{309}$$

> $p(F35 \cap K3\,K2\,\overline{K1}\,K4) := 1 - (1 - p(F35 \cap K3 \sim)) \cdot (1 - p(F35 \cap K2 \sim)) \cdot (1 - p(F35 \cap K4 \sim));$
$$p(F35 \cap K3\,K2\,\overline{K1}\,K4) := 0.84 \tag{310}$$

> $p(F36 \cap K3\,K2\,\overline{K1}\,K4) := 1 - (1 - p(F36 \cap K3 \sim)) \cdot (1 - p(F36 \cap K2 \sim)) \cdot (1 - p(F36 \cap K4 \sim));$
$$p(F36 \cap K3\,K2\,\overline{K1}\,K4) := 0.82 \tag{311}$$

> $b32 := (f31 \cdot p(F31`1`K3\,K2\,\overline{K1}\,K4) + (1-f31)\cdot(1-p(F31`1`K3\,K2\,\overline{K1}\,K4)))\cdot(f32\cdot p(F32`1`K3\,K2\,\overline{K1}\,K4) + (1$
$-f32)\cdot(1-p(F32`1`K3\,K2\,\overline{K1}\,K4)))\cdot(f33\cdot p(F33`1`K3\,K2\,\overline{K1}\,K4) + (1-f33)\cdot(1-p(F33`1`K3\,K2$
$\overline{K1}\,K4)))\cdot(f34\cdot p(F34`1`K3\,K2\,\overline{K1}\,K4) + (1-f34)\cdot(1-p(F34`1`K3\,K2\,\overline{K1}\,K4)))\cdot(f35\cdot p(F35`1`K3\,K2$
$\overline{K1}\,K4) + (1-f35)\cdot(1-p(F35`1`K3\,K2\,\overline{K1}\,K4)))\cdot(f36\cdot p(F36`1`K3\,K2\,\overline{K1}\,K4) + (1-f36)\cdot(1$
$-p(F36`1`K3\,K2\,\overline{K1}\,K4)));$

$$b32 := 0.0023 \qquad (312)$$

> $p(F31`1`K3\,K2\,\overline{K1}\,\overline{K4}) := 1-(1-p(F31`1`K3`\sim`))\cdot(1-p(F31`1`K2`\sim`));$
$$p(F31\ `1`\ K3\ K2\ K1\ \overline{K4}) := 0.65 \qquad (313)$$

> $p(F32`1`K3\,K2\,\overline{K1}\,\overline{K4}) := 1-(1-p(F32`1`K3`\sim`))\cdot(1-p(F32`1`K2`\sim`));$
$$p(F32\ `1`\ K3\ K2\ K1\ \overline{K4}) := 0.2 \qquad (314)$$

> $p(F33`1`K3\,K2\,\overline{K1}\,\overline{K4}) := 1-(1-p(F33`1`K3`\sim`))\cdot(1-p(F33`1`K2`\sim`));$
$$p(F33\ `1`\ K3\ K2\ K1\ \overline{K4}) := 0.5 \qquad (315)$$

> $p(F34`1`K3\,K2\,\overline{K1}\,\overline{K4}) := 1-(1-p(F34`1`K3`\sim`))\cdot(1-p(F34`1`K2`\sim`));$
$$p(F34\ `1`\ K3\ K2\ K1\ \overline{K4}) := 0.64 \qquad (316)$$

> $p(F35`1`K3\,K2\,\overline{K1}\,\overline{K4}) := 1-(1-p(F35`1`K3`\sim`))\cdot(1-p(F35`1`K2`\sim`));$
$$p(F35\ `1`\ K3\ K2\ K1\ \overline{K4}) := 0.84 \qquad (317)$$

> $p(F36`1`K3\,K2\,\overline{K1}\,\overline{K4}) := 1-(1-p(F36`1`K3`\sim`))\cdot(1-p(F36`1`K2`\sim`));$
$$p(F36\ `1`\ K3\ K2\ K1\ \overline{K4}) := 0.7 \qquad (318)$$

>
> $b33 := (f31 \cdot p(F31`1`K3\,K2\,\overline{K1}\,\overline{K4}) + (1-f31)\cdot(1-p(F31`1`K3\,K2\,\overline{K1}\,\overline{K4})))\cdot(f32\cdot p(F32`1`K3\,K2\,\overline{K1}\,\overline{K4}) + (1$
$-f32)\cdot(1-p(F32`1`K3\,K2\,\overline{K1}\,K4)))\cdot(f33\cdot p(F33`1`K3\,K2\,\overline{K1}\,K4) + (1-f33)\cdot(1-p(F33`1`K3\,K2\,\overline{K1}$
$\overline{K4})))\cdot(f34\cdot p(F34`1`K3\,K2\,\overline{K1}\,K4) + (1-f34)\cdot(1-p(F34`1`K3\,K2\,\overline{K1}\,\overline{K4})))\cdot(f35\cdot p(F35`1`K3\,K2\,\overline{K1}\,\overline{K4})$
$+ (1-f35)\cdot(1-p(F35`1`K3\,K2\,\overline{K1}\,K4)))\cdot(f36\cdot p(F36`1`K3\,K2\,\overline{K1}\,K4) + (1-f36)\cdot(1-p(F36`1`K3\,K2\,\overline{K1}$
$\overline{K4})));$

$$b33 := 0.0011 \qquad (319)$$

> $p(F31`1`K3\,\overline{K2}\,K1\,K4) := 1-(1-p(F31`1`K1`\sim`))\cdot(1-p(F31`1`K3`\sim`))\cdot(1-p(F31`1`K4`\sim`));$
$$p(F31\ `1`\ K3\ K2\ K1\ K4) := 0.76 \qquad (320)$$

> $p(F32`1`K3\,\overline{K2}\,K1\,K4) := 1-(1-p(F32`1`K1`\sim`))\cdot(1-p(F32`1`K3`\sim`))\cdot(1-p(F32`1`K4`\sim`));$
$$p(F32\ `1`\ K3\ K2\ K1\ K4) := 0.84 \qquad (321)$$

> $p(F33`1`K3\,\overline{K2}\,K1\,K4) := 1-(1-p(F33`1`K1`\sim`))\cdot(1-p(F33`1`K3`\sim`))\cdot(1-p(F33`1`K4`\sim`));$
$$p(F33\ `1`\ K3\ K2\ K1\ K4) := 0.95 \qquad (322)$$

> $p(F34`1`K3\,\overline{K2}\,K1\,K4) := 1-(1-p(F34`1`K1`\sim`))\cdot(1-p(F34`1`K3`\sim`))\cdot(1-p(F34`1`K4`\sim`));$
$$p(F34\ `1`\ K3\ K2\ K1\ K4) := 0.82 \qquad (323)$$

> $p(F35`1`K3\,\overline{K2}\,K1\,K4) := 1-(1-p(F35`1`K1`\sim`))\cdot(1-p(F35`1`K3`\sim`))\cdot(1-p(F35`1`K4`\sim`));$
$$p(F35\ `1`\ K3\ K2\ K1\ K4) := 0.6 \qquad (324)$$

> $p(F36`1`K3\,\overline{K2}\,K1\,K4) := 1-(1-p(F36`1`K1`\sim`))\cdot(1-p(F36`1`K3`\sim`))\cdot(1-p(F36`1`K4`\sim`));$
$$p(F36\ `1`\ K3\ K2\ K1\ K4) := 0.82 \qquad (325)$$

> $b34 := (f31 \cdot p(F31`1`K3\,\overline{K2}\,K1\,K4) + (1-f31)\cdot(1-p(F31`1`K3\,\overline{K2}\,K1\,K4)))\cdot(f32\cdot p(F32`1`K3\,\overline{K2}\,K1\,K4) + (1$
$-f32)\cdot(1-p(F32`1`K3\,\overline{K2}\,K1\,K4)))\cdot(f33\cdot p(F33`1`K3\,\overline{K2}\,K1\,K4) + (1-f33)\cdot(1-p(F33`1`K3$
$\overline{K2}\,K1\,K4)))\cdot(f34\cdot p(F34`1`K3\,\overline{K2}\,K1\,K4) + (1-f34)\cdot(1-p(F34`1`K3\,\overline{K2}\,K1\,K4)))\cdot(f35\cdot p(F35`1`K3$
$\overline{K2}\,K1\,K4) + (1-f35)\cdot(1-p(F35`1`K3\,\overline{K2}\,K1\,K4)))\cdot(f36\cdot p(F36`1`K3\,\overline{K2}\,K1\,K4) + (1-f36)\cdot(1$
$-p(F36`1`K3\,\overline{K2}\,K1\,K4)));$

$$b34 := 0.00041 \qquad (326)$$

> $p(F31`1`K3\,\overline{K2}\,K1\,\overline{K4}) := 1-(1-p(F31`1`K1`\sim`))\cdot(1-p(F31`1`K3`\sim`));$
$$p(F31\ `1`\ K3\ K2\ K1\ \overline{K4}) := 0.65 \qquad (327)$$

> $p(F32`1`K3\,\overline{K2}\,K1\,\overline{K4}) := 1-(1-p(F32`1`K1`\sim`))\cdot(1-p(F32`1`K3`\sim`));$
$$p(F32\ `1`\ K3\ K2\ K1\ \overline{K4}) := 0.68 \qquad (328)$$

> $p(F33\urcorner K3\overline{K2}\,K1\,\overline{K4}) := 1 - (1 - p(F33\urcorner K1\urcorner\sim\urcorner)) \cdot (1 - p(F33\urcorner K3\urcorner\sim\urcorner))$;
$$p(F33\;\urcorner\;K3\;\overline{K2}\;K1\;\overline{K4}) := 0.95 \qquad (329)$$

> $p(F34\urcorner K3\overline{K2}\,K1\,\overline{K4}) := 1 - (1 - p(F34\urcorner K1\urcorner\sim\urcorner)) \cdot (1 - p(F34\urcorner K3\urcorner\sim\urcorner))$;
$$p(F34\;\urcorner\;K3\;\overline{K2}\;K1\;\overline{K4}) := 0.82 \qquad (330)$$

> $p(F35\urcorner K3\overline{K2}\,K1\,\overline{K4}) := 1 - (1 - p(F35\urcorner K1\urcorner\sim\urcorner)) \cdot (1 - p(F35\urcorner K3\urcorner\sim\urcorner))$;
$$p(F35\;\urcorner\;K3\;\overline{K2}\;K1\;\overline{K4}) := 0.6 \qquad (331)$$

> $p(F36\urcorner K3\overline{K2}\,K1\,\overline{K4}) := 1 - (1 - p(F36\urcorner K1\urcorner\sim\urcorner)) \cdot (1 - p(F36\urcorner K3\urcorner\sim\urcorner))$;
$$p(F36\;\urcorner\;K3\;\overline{K2}\;K1\;\overline{K4}) := 0.7 \qquad (332)$$

> $b35 := (f31 \cdot p(F31\urcorner K3\overline{K2}\,K1\,\overline{K4}) + (1 - f31) \cdot (1 - p(F31\urcorner K3\overline{K2}\,K1\,\overline{K4}))) \cdot (f32 \cdot p(F32\urcorner K3\overline{K2}\,K1\,\overline{K4}) + (1 - f32) \cdot (1 - p(F32\urcorner K3\overline{K2}\,K1\,\overline{K4}))) \cdot (f33 \cdot p(F33\urcorner K3\overline{K2}\,K1\,\overline{K4}) + (1 - f33) \cdot (1 - p(F33\urcorner K3\overline{K2}\,K1\,\overline{K4}))) \cdot (f34 \cdot p(F34\urcorner K3\overline{K2}\,K1\,\overline{K4}) + (1 - f34) \cdot (1 - p(F34\urcorner K3\overline{K2}\,K1\,\overline{K4}))) \cdot (f35 \cdot p(F35\urcorner K3\overline{K2}\,K1\,\overline{K4}) + (1 - f35) \cdot (1 - p(F35\urcorner K3\overline{K2}\,K1\,\overline{K4}))) \cdot (f36 \cdot p(F36\urcorner K3\overline{K2}\,K1\,\overline{K4}) + (1 - f36) \cdot (1 - p(F36\urcorner K3\overline{K2}\,K1\,\overline{K4})))$;
$$b35 := 0.00048 \qquad (333)$$

> $p(F31\urcorner K3\overline{K2}\,\overline{K1}\,K4) := 1 - (1 - p(F31\urcorner K3\urcorner\sim\urcorner)) \cdot (1 - p(F31\urcorner K4\urcorner\sim\urcorner))$;
$$p(F31\;\urcorner\;K3\;\overline{K2}\;\overline{K1}\;K4) := 0.51 \qquad (334)$$

> $p(F32\urcorner K3\overline{K2}\,\overline{K1}\,K4) := 1 - (1 - p(F32\urcorner K3\urcorner\sim\urcorner)) \cdot (1 - p(F32\urcorner K4\urcorner\sim\urcorner))$;
$$p(F32\;\urcorner\;K3\;\overline{K2}\;\overline{K1}\;K4) := 0.60 \qquad (335)$$

> $p(F33\urcorner K3\overline{K2}\,\overline{K1}\,K4) := 1 - (1 - p(F33\urcorner K3\urcorner\sim\urcorner)) \cdot (1 - p(F33\urcorner K4\urcorner\sim\urcorner))$;
$$p(F33\;\urcorner\;K3\;\overline{K2}\;\overline{K1}\;K4) := 0.5 \qquad (336)$$

> $p(F34\urcorner K3\overline{K2}\,\overline{K1}\,K4) := 1 - (1 - p(F34\urcorner K3\urcorner\sim\urcorner)) \cdot (1 - p(F34\urcorner K4\urcorner\sim\urcorner))$;
$$p(F34\;\urcorner\;K3\;\overline{K2}\;\overline{K1}\;K4) := 0.4 \qquad (337)$$

> $p(F35\urcorner K3\overline{K2}\,\overline{K1}\,K4) := 1 - (1 - p(F35\urcorner K3\urcorner\sim\urcorner)) \cdot (1 - p(F35\urcorner K4\urcorner\sim\urcorner))$;
$$p(F35\;\urcorner\;K3\;\overline{K2}\;\overline{K1}\;K4) := 0.6 \qquad (338)$$

> $p(F36\urcorner K3\overline{K2}\,\overline{K1}\,K4) := 1 - (1 - p(F36\urcorner K3\urcorner\sim\urcorner)) \cdot (1 - p(F36\urcorner K4\urcorner\sim\urcorner))$;
$$p(F36\;\urcorner\;K3\;\overline{K2}\;\overline{K1}\;K4) := 0.82 \qquad (339)$$

> $b36 := (f31 \cdot p(F31\urcorner K3\overline{K2}\,\overline{K1}\,K4) + (1 - f31) \cdot (1 - p(F31\urcorner K3\overline{K2}\,\overline{K1}\,K4))) \cdot (f32 \cdot p(F32\urcorner K3\overline{K2}\,\overline{K1}\,K4) + (1 - f32) \cdot (1 - p(F32\urcorner K3\overline{K2}\,\overline{K1}\,K4))) \cdot (f33 \cdot p(F33\urcorner K3\overline{K2}\,\overline{K1}\,K4) + (1 - f33) \cdot (1 - p(F33\urcorner K3\overline{K2}\,\overline{K1}\,K4))) \cdot (f34 \cdot p(F34\urcorner K3\overline{K2}\,\overline{K1}\,K4) + (1 - f34) \cdot (1 - p(F34\urcorner K3\overline{K2}\,\overline{K1}\,K4))) \cdot (f35 \cdot p(F35\urcorner K3\overline{K2}\,\overline{K1}\,K4) + (1 - f35) \cdot (1 - p(F35\urcorner K3\overline{K2}\,\overline{K1}\,K4))) \cdot (f36 \cdot p(F36\urcorner K3\overline{K2}\,\overline{K1}\,K4) + (1 - f36) \cdot (1 - p(F36\urcorner K3\overline{K2}\,\overline{K1}\,K4)))$;
$$b36 := 0.0068 \qquad (340)$$

> $p(F31\urcorner K3\overline{K2}\,\overline{K1}\,\overline{K4}) := p(F31\urcorner K3\urcorner\sim\urcorner)$;
$$p(F31\;\urcorner\;K3\;\overline{K2}\;\overline{K1}\;\overline{K4}) := 0.3 \qquad (341)$$

> $p(F32\urcorner K3\overline{K2}\,\overline{K1}\,\overline{K4}) := p(F32\urcorner K3\urcorner\sim\urcorner)$;
$$p(F32\;\urcorner\;K3\;\overline{K2}\;\overline{K1}\;\overline{K4}) := 0.2 \qquad (342)$$

> $p(F33\urcorner K3\overline{K2}\,\overline{K1}\,\overline{K4}) := p(F33\urcorner K3\urcorner\sim\urcorner)$;
$$p(F33\;\urcorner\;K3\;\overline{K2}\;\overline{K1}\;\overline{K4}) := 0.5 \qquad (343)$$

> $p(F34\urcorner K3\overline{K2}\,\overline{K1}\,\overline{K4}) := p(F34\urcorner K3\urcorner\sim\urcorner)$;
$$p(F34\;\urcorner\;K3\;\overline{K2}\;\overline{K1}\;\overline{K4}) := 0.4 \qquad (344)$$

> $p(F35\urcorner K3\overline{K2}\,\overline{K1}\,\overline{K4}) := p(F35\urcorner K3\urcorner\sim\urcorner)$;
$$p(F35\;\urcorner\;K3\;\overline{K2}\;\overline{K1}\;\overline{K4}) := 0.6 \qquad (345)$$

> $p(F36\urcorner K3\overline{K2}\,\overline{K1}\,\overline{K4}) := p(F36\urcorner K3\urcorner\sim\urcorner)$;
$$p(F36\;\urcorner\;K3\;\overline{K2}\;\overline{K1}\;\overline{K4}) := 0.7 \qquad (346)$$

> $b37 := (f31 \cdot p(F31\urcorner K3\overline{K2}\,\overline{K1}\,\overline{K4}) + (1 - f31) \cdot (1 - p(F31\urcorner K3\overline{K2}\,\overline{K1}\,\overline{K4}))) \cdot (f32 \cdot p(F32\urcorner K3\overline{K2}\,\overline{K1}\,\overline{K4}) + (1 - f32) \cdot (1 - p(F32\urcorner K3\overline{K2}\,\overline{K1}\,\overline{K4}))) \cdot (f33 \cdot p(F33\urcorner K3\overline{K2}\,\overline{K1}\,\overline{K4}) + (1 - f33) \cdot (1 - p(F33\urcorner K3\overline{K2}\,\overline{K1}$

$\overline{K4})))\cdot(f34\cdot p(F34\`1\`K3\,\overline{K2}\,\overline{K1}\,\overline{K4}) + (1-f34)\cdot(1-p(F34\`1\`K3\,\overline{K2}\,\overline{K1}\,\overline{K4})))\cdot(f35\cdot p(F35\`1\`K3\,\overline{K2}\,\overline{K1}\,\overline{K4})$
$+ (1-f35)\cdot(1-p(F35\`1\`K3\,\overline{K2}\,\overline{K1}\,\overline{K4})))\cdot(f36\cdot p(F36\`1\`K3\,\overline{K2}\,\overline{K1}\,\overline{K4}) + (1-f36)\cdot(1-p(F36\`1\`K3\,\overline{K2}\,\overline{K1}$
$\overline{K4})));$

$$b37 := 0.0022 \tag{347}$$

> $c3 := 0.25;$

$$c3 := 0.25 \tag{348}$$

> $eq3 := x3 = 1 \Big/ \Big( 1 + (a30\cdot x2\cdot x1\cdot x4 + a31\cdot x2\cdot x1\cdot(1-x4) + a32\cdot x2\cdot(1-x1)\cdot x4 + a33\cdot x2\cdot(1-x1)\cdot(1-x4)$

$+ 0 + 0 + 0 + 0 + 0 + 0 + 0 + 0 + 0 + 0 + a34\cdot(1-x2)\cdot x1\cdot x4 + a35\cdot(1-x2)\cdot x1\cdot(1-x4) + a36\cdot(1$

$-x2)\cdot(1-x1)\cdot x4 + a37\cdot(1-x2)\cdot(1-x1)\cdot(1-x4)) \Big/ (b30\cdot x2\cdot x1\cdot x4 + b31\cdot x2\cdot x1\cdot(1-x4) + b32\cdot x2$

$\cdot(1-x1)\cdot x4 + b33\cdot x2\cdot(1-x1)\cdot(1-x4) + b34\cdot(1-x2)\cdot x1\cdot x4 + b35\cdot(1-x2)\cdot x1\cdot(1-x4) + b36\cdot(1$

$-x2)\cdot(1-x1)\cdot x4 + b37\cdot(1-x2)\cdot(1-x1)\cdot(1-x4)\Big)\cdot\Big(\dfrac{(1-c3)}{c3}\Big)\Big);$

$$eq3 := x3 = 1 \Big/ (1 + (3.0\,(0.0029\,x2\,x1\,x4 + 0.0032\,x2\,x1\,(1-x4) + 0.046\,x2\,(1-x1)\,x4 \tag{349}$$

$+ 0.0096\,(1-x2)\,x1\,x4 + 0.0090\,(1-x2)\,x1\,(1-x4) + 0.090\,(1-x2)\,(1-x1)\,x4))$

$\Big/ (0.00012\,x2\,x1\,x4 + 0.00015\,x2\,x1\,(1-x4) + 0.0023\,x2\,(1-x1)\,x4 + 0.0011\,x2\,(1$

$-x1)\,(1-x4) + 0.00041\,(1-x2)\,x1\,x4 + 0.00048\,(1-x2)\,x1\,(1-x4) + 0.0068\,(1$

$-x2)\,(1-x1)\,x4 + 0.0022\,(1-x2)\,(1-x1)\,(1-x4)))$

> $f41 := S1; f42 := S2; f43 := S3; f44 := S4; f45 := S8; f46 := S9;$

$$f41 := 1$$
$$f42 := 1$$
$$f43 := 1$$
$$f44 := 1$$
$$f45 := 0$$
$$f46 := 0 \tag{350}$$

>
> $p(F41\`1\`K1\`\sim\`) := 0.6; p(F41\`1\`K2\`\sim\`) := 0.6; p(F41\`1\`K3\`\sim\`) := 0; p(F41\`1\`K4\`\sim\`) := 0.5;$

$$p(F41\`1\`K1\`\sim\`) := 0.6$$
$$p(F41\`1\`K2\`\sim\`) := 0.6$$
$$p(F41\`1\`K3\`\sim\`) := 0$$
$$p(F41\`1\`K4\`\sim\`) := 0.5 \tag{351}$$

> $p(F42\`1\`K1\`\sim\`) := 0.5; p(F42\`1\`K2\`\sim\`) := 0.5; p(F42\`1\`K3\`\sim\`) := 0.3; p(F42\`1\`K4\`\sim\`) := 0.3;$

$$p(F42\`1\`K1\`\sim\`) := 0.5$$
$$p(F42\`1\`K2\`\sim\`) := 0.5$$
$$p(F42\`1\`K3\`\sim\`) := 0.3$$
$$p(F42\`1\`K4\`\sim\`) := 0.3 \tag{352}$$

> $p(F43\`1\`K1\`\sim\`) := 0.5; p(F43\`1\`K2\`\sim\`) := 0.5; p(F43\`1\`K3\`\sim\`) := 0; p(F43\`1\`K4\`\sim\`) := 0.3;$

$$p(F43\`1\`K1\`\sim\`) := 0.5$$
$$p(F43\`1\`K2\`\sim\`) := 0.5$$
$$p(F43\`1\`K3\`\sim\`) := 0$$
$$p(F43\`1\`K4\`\sim\`) := 0.3 \tag{353}$$

> $p(F44\`1\`K1\`\sim\`) := 0.6; p(F44\`1\`K2\`\sim\`) := 0; p(F44\`1\`K3\`\sim\`) := 0.2; p(F44\`1\`K4\`\sim\`) := 0.5;$

$$p(F44\ \text{'}|\text{'}\ K1\ \text{'~'}) := 0.6$$

$$p(F44\ \text{'}|\text{'}\ K2\ \text{'~'}) := 0$$

$$p(F44\ \text{'}|\text{'}\ K3\ \text{'~'}) := 0.2$$

$$p(F44\ \text{'}|\text{'}\ K4\ \text{'~'}) := 0.5 \tag{354}$$

$$> p(F45\text{'}|\text{'}K1\text{'~'}) := 0;\ p(F45\text{'}|\text{'}K2\text{'~'}) := 0.7;\ p(F45\text{'}|\text{'}K3\text{'~'}) := 0;\ p(F45\text{'}|\text{'}K4\text{'~'}) := 0.2;$$

$$p(F45\ \text{'}|\text{'}\ K1\ \text{'~'}) := 0$$

$$p(F45\ \text{'}|\text{'}\ K2\ \text{'~'}) := 0.7$$

$$p(F45\ \text{'}|\text{'}\ K3\ \text{'~'}) := 0$$

$$p(F45\ \text{'}|\text{'}\ K4\ \text{'~'}) := 0.2 \tag{355}$$

$$> p(F46\text{'}|\text{'}K1\text{'~'}) := 0;\ p(F46\text{'}|\text{'}K2\text{'~'}) := 0;\ p(F46\text{'}|\text{'}K3\text{'~'}) := 0.7;\ p(F46\text{'}|\text{'}K4\text{'~'}) := 0.4;$$

$$p(F46\ \text{'}|\text{'}\ K1\ \text{'~'}) := 0$$

$$p(F46\ \text{'}|\text{'}\ K2\ \text{'~'}) := 0$$

$$p(F46\ \text{'}|\text{'}\ K3\ \text{'~'}) := 0.7$$

$$p(F46\ \text{'}|\text{'}\ K4\ \text{'~'}) := 0.4 \tag{356}$$

$$> p(F41\text{'}|\text{'}\overline{K4}\ K2\,K3\,K1) := 1 - (1 - p(F41\text{'}|\text{'}\underline{K2\text{'}}\text{~'}))\cdot(1 - p(F41\text{'}|\text{'}K3\text{'~'}))\cdot(1 - p(F41\text{'}|\text{'}K1\text{'~'}));$$

$$p(F41\ \text{'}|\text{'}\ \overline{K4}\ K2\ K3\ K1) := 0.84 \tag{357}$$

$$> p(F42\text{'}|\text{'}\overline{K4}\ K2\,K3\,K1) := 1 - (1 - p(F42\text{'}|\text{'}\underline{K2\text{'}}\text{~'}))\cdot(1 - p(F42\text{'}|\text{'}K3\text{'~'}))\cdot(1 - \bar{p}(F42\text{'}|\text{'}K1\text{'~'}));$$

$$p(F42\ \text{'}|\text{'}\ \overline{K4}\ K2\ K3\ K1) := 0.82 \tag{358}$$

$$> p(F43\text{'}|\text{'}\overline{K4}\ K2\,K3\,K1) := 1 - (1 - p(F43\text{'}|\text{'}\underline{K2\text{'}}\text{~'}))\cdot(1 - p(F43\text{'}|\text{'}K3\text{'~'}))\cdot(1 - p(F43\text{'}|\text{'}K1\text{'~'}));$$

$$p(F43\ \text{'}|\text{'}\ \overline{K4}\ K2\ K3\ K1) := 0.75 \tag{359}$$

$$> p(F44\text{'}|\text{'}\overline{K4}\ K2\,K3\,K1) := 1 - (1 - p(F44\text{'}|\text{'}\underline{K2\text{'}}\text{~'}))\cdot(1 - p(F44\text{'}|\text{'}K3\text{'~'}))\cdot(1 - p(F44\text{'}|\text{'}K1\text{'~'}));$$

$$p(F44\ \text{'}|\text{'}\ \overline{K4}\ K2\ K3\ K1) := 0.68 \tag{360}$$

$$> p(F45\text{'}|\text{'}\overline{K4}\ K2\,K3\,K1) := 1 - (1 - p(F45\text{'}|\text{'}\underline{K2\text{'}}\text{~'}))\cdot(1 - p(F45\text{'}|\text{'}K3\text{'~'}))\cdot(1 - p(F45\text{'}|\text{'}K1\text{'~'}));$$

$$p(F45\ \text{'}|\text{'}\ \overline{K4}\ K2\ K3\ K1) := 0.7 \tag{361}$$

$$> p(F46\text{'}|\text{'}\overline{K4}\ K2\,K3\,K1) := 1 - (1 - p(F46\text{'}|\text{'}\underline{K2\text{'}}\text{~'}))\cdot(1 - p(F46\text{'}|\text{'}K3\text{'~'}))\cdot(1 - p(F46\text{'}|\text{'}K1\text{'~'}));$$

$$p(F46\ \text{'}|\text{'}\ \overline{K4}\ K2\ K3\ K1) := 0.7 \tag{362}$$

$$> a40 := \big(f41\cdot p(F41\text{'}|\text{'}\overline{K4}\ K2\,K3\,\underline{K1}) + (1 - f41)\cdot(1 - p(F41\text{'}|\text{'}\overline{K4}\ K2\,K3\,K1))\big)\cdot\big(f42\cdot p(F42\text{'}|\text{'}\overline{K4}\ K2\,K3\,K1)$$
$$+ (1 - f42)\cdot(1 - p(F42\text{'}|\text{'}\overline{K4}\ K2\,K3\,K1))\big)\cdot\big(f43\cdot p(F43\text{'}|\text{'}\overline{K4}\ K2\,K3\,K1) + (1 - f43)\cdot(1 - p(F43\text{'}|\text{'}$$
$$\overline{K4}\ K2\,K3\,K1))\big)\cdot\big(f44\cdot p(F44\text{'}|\text{'}\overline{K4}\ K2\,K3\,\underline{K1}) + (1 - f44)\cdot(1 - p(F44\text{'}|\text{'}\overline{K4}\ K2\,K3\,K1))\big)\cdot\big(f45\cdot p(F45\text{'}|\text{'}$$
$$\overline{K4}\ K2\,K3\,\underline{K1}) + (1 - f45)\cdot(1 - p(F45\text{'}|\text{'}\overline{K4}\ K2\,K3\,K1))\big)\cdot\big(f46\cdot p(F46\text{'}|\text{'}\overline{K4}\ K2\,K3\,K1) + (1 - f46)\cdot(1$$
$$- p(F46\text{'}|\text{'}\overline{K4}\ K2\,K3\,K1))\big);$$

$$a40 := 0.030 \tag{363}$$

$$> p(F41\text{'}|\text{'}\overline{K4}\ K2\,K3\,\overline{K1}) := 1 - (1 - p(F41\text{'}|\text{'}\underline{K2\text{'}}\text{~'}))\cdot(1 - \underline{p}(F41\text{'}|\text{'}K3\text{'~'}));$$

$$p(F41\ \text{'}|\text{'}\ \overline{K4}\ K2\ K3\ \overline{K1}) := 0.6 \tag{364}$$

$$> p(F42\text{'}|\text{'}\overline{K4}\ K2\,K3\,\overline{K1}) := 1 - (1 - p(F42\text{'}|\text{'}\underline{K2\text{'}}\text{~'}))\cdot(1 - \underline{p}(F42\text{'}|\text{'}K3\text{'~'}));$$

$$p(F42\ \text{'}|\text{'}\ \overline{K4}\ K2\ K3\ \overline{K1}) := 0.65 \tag{365}$$

$$> p(F43\text{'}|\text{'}\overline{K4}\ K2\,K3\,\overline{K1}) := 1 - (1 - p(F43\text{'}|\text{'}\underline{K2\text{'}}\text{~'}))\cdot(1 - \underline{p}(F43\text{'}|\text{'}K3\text{'~'}));$$

$$p(F43\ \text{'}|\text{'}\ \overline{K4}\ K2\ K3\ \overline{K1}) := 0.5 \tag{366}$$

$$> p(F44\text{'}|\text{'}\overline{K4}\ K2\,K3\,\overline{K1}) := 1 - (1 - p(F44\text{'}|\text{'}\underline{K2\text{'}}\text{~'}))\cdot(1 - \underline{p}(F44\text{'}|\text{'}K3\text{'~'}));$$

$$p(F44\ \text{'}|\text{'}\ \overline{K4}\ K2\ K3\ \overline{K1}) := 0.2 \tag{367}$$

$$> p(F45\text{'}|\text{'}\overline{K4}\ K2\,K3\,\overline{K1}) := 1 - (1 - p(F45\text{'}|\text{'}\underline{K2\text{'}}\text{~'}))\cdot(1 - \underline{p}(F45\text{'}|\text{'}K3\text{'~'}));$$

$$p(F45\ \text{'}|\text{'}\ \overline{K4}\ K2\ K3\ \overline{K1}) := 0.7 \tag{368}$$

$$> p(F46\text{'}|\text{'}\overline{K4}\ K2\,K3\,\overline{K1}) := 1 - (1 - p(F46\text{'}|\text{'}\underline{K2\text{'}}\text{~'}))\cdot(1 - \underline{p}(F46\text{'}|\text{'}K3\text{'~'}));$$

$$p(F46\ \text{'}|\text{'}\ \overline{K4}\ K2\ K3\ \overline{K1}) := 0.7 \tag{369}$$

$$> a41 := \big(f41\cdot p(F41\text{'}|\text{'}\overline{K4}\ K2\,K3\,\overline{K1}) + (1 - f41)\cdot(1 - p(F41\text{'}|\text{'}\overline{K4}\ K2\,K3\,\overline{K1}))\big)\cdot\big(f42\cdot p(F42\text{'}|\text{'}\overline{K4}\ K2\,K3\,\overline{K1})$$

$+ (1 - f42) \cdot (1 - p(F42 \cap \overline{K4}\, K2\, K3\, \overline{K1})))) \cdot (f43 \cdot p(F43 \cap \overline{K4}\, K2\, K3\, \overline{K1}) + (1 - f43) \cdot (1 - p(F43 \cap \overline{K4}\, K2\, K3\, \overline{K1})))) \cdot (f44 \cdot p(F44 \cap \overline{K4}\, K2\, K3\, \overline{K1}) + (1 - f44) \cdot (1 - p(F44 \cap \overline{K4}\, K2\, K3\, \overline{K1})))) \cdot (f45 \cdot p(F45 \cap \overline{K4}\, K2\, K3\, \overline{K1}) + (1 - f45) \cdot (1 - p(F45 \cap \overline{K4}\, K2\, K3\, \overline{K1})))) \cdot (f46 \cdot p(F46 \cap \overline{K4}\, K2\, K3\, \overline{K1}) + (1 - f46) \cdot (1 - p(F46 \cap \overline{K4}\, K2\, K3\, \overline{K1}))));$

$$a41 := 0.0036 \tag{370}$$

$> \quad p(F41 \cap \overline{K4}\, K2\, \overline{K3}\, K1) := 1 - (1 - p(F41 \cap K2 \sim)) \cdot (1 - p(F41 \cap K1 \sim));$

$$p(F41 \cap \overline{K4}\, K2\, K3\, K1) := 0.84 \tag{371}$$

$> \quad p(F42 \cap \overline{K4}\, K2\, \overline{K3}\, K1) := 1 - (1 - p(F42 \cap K2 \sim)) \cdot (1 - p(F42 \cap K1 \sim));$

$$p(F42 \cap \overline{K4}\, K2\, K3\, K1) := 0.75 \tag{372}$$

$> \quad p(F43 \cap \overline{K4}\, K2\, \overline{K3}\, K1) := 1 - (1 - p(F43 \cap K2 \sim)) \cdot (1 - p(F43 \cap K1 \sim));$

$$p(F43 \cap \overline{K4}\, K2\, K3\, K1) := 0.75 \tag{373}$$

$> \quad p(F44 \cap \overline{K4}\, K2\, \overline{K3}\, K1) := 1 - (1 - p(F44 \cap K2 \sim)) \cdot (1 - p(F44 \cap K1 \sim));$

$$p(F44 \cap \overline{K4}\, K2\, K3\, K1) := 0.6 \tag{374}$$

$> \quad p(F45 \cap \overline{K4}\, K2\, \overline{K3}\, K1) := 1 - (1 - p(F45 \cap K2 \sim)) \cdot (1 - p(F45 \cap K1 \sim));$

$$p(F45 \cap \overline{K4}\, K2\, K3\, K1) := 0.7 \tag{375}$$

$> \quad p(F46 \cap \overline{K4}\, K2\, \overline{K3}\, K1) := 1 - (1 - p(F46 \cap K2 \sim)) \cdot (1 - p(F46 \cap K1 \sim));$

$$p(F46 \cap \overline{K4}\, K2\, K3\, K1) := 0 \tag{376}$$

$> \quad a42 := (f41 \cdot p(F41 \cap \overline{K4}\, K2\, \overline{K3}\, K1) + (1 - f41) \cdot (1 - p(F41 \cap \overline{K4}\, K2\, \overline{K3}\, K1)))) \cdot (f42 \cdot p(F42 \cap \overline{K4}\, K2\, \overline{K3}\, K1) + (1 - f42) \cdot (1 - p(F42 \cap \overline{K4}\, K2\, \overline{K3}\, K1)))) \cdot (f43 \cdot p(F43 \cap \overline{K4}\, K2\, \overline{K3}\, K1) + (1 - f43) \cdot (1 - p(F43 \cap \overline{K4}\, K2\, \overline{K3}\, K1)))) \cdot (f44 \cdot p(F44 \cap \overline{K4}\, K2\, \overline{K3}\, K1) + (1 - f44) \cdot (1 - p(F44 \cap \overline{K4}\, K2\, \overline{K3}\, K1)))) \cdot (f45 \cdot p(F45 \cap \overline{K4}\, K2\, \overline{K3}\, K1) + (1 - f45) \cdot (1 - p(F45 \cap \overline{K4}\, K2\, \overline{K3}\, K1)))) \cdot (f46 \cdot p(F46 \cap \overline{K4}\, K2\, \overline{K3}\, K1) + (1 - f46) \cdot (1 - p(F46 \cap \overline{K4}\, K2\, \overline{K3}\, K1))));$

$$a42 := 0.084 \tag{377}$$

$> \quad p(F41 \cap \overline{K4}\, K2\, \overline{K3}\, \overline{K1}) := p(F41 \cap K2 \sim);$

$$p(F41 \cap \overline{K4}\, K2\, \overline{K3}\, \overline{K1}) := 0.6 \tag{378}$$

$> \quad p(F42 \cap \overline{K4}\, K2\, \overline{K3}\, \overline{K1}) := p(F42 \cap K2 \sim);$

$$p(F42 \cap \overline{K4}\, K2\, \overline{K3}\, \overline{K1}) := 0.5 \tag{379}$$

$> \quad p(F43 \cap \overline{K4}\, K2\, \overline{K3}\, \overline{K1}) := p(F43 \cap K2 \sim);$

$$p(F43 \cap \overline{K4}\, K2\, \overline{K3}\, \overline{K1}) := 0.5 \tag{380}$$

$> \quad p(F44 \cap \overline{K4}\, K2\, \overline{K3}\, \overline{K1}) := p(F44 \cap K2 \sim);$

$$p(F44 \cap \overline{K4}\, K2\, \overline{K3}\, \overline{K1}) := 0 \tag{381}$$

$> \quad p(F45 \cap \overline{K4}\, K2\, \overline{K3}\, \overline{K1}) := p(F45 \cap K2 \sim);$

$$p(F45 \cap \overline{K4}\, K2\, \overline{K3}\, \overline{K1}) := 0.7 \tag{382}$$

$> \quad p(F46 \cap \overline{K4}\, K2\, \overline{K3}\, \overline{K1}) := p(F46 \cap K2 \sim);$

$$p(F46 \cap \overline{K4}\, K2\, \overline{K3}\, \overline{K1}) := 0 \tag{383}$$

$> \quad a43 := (f41 \cdot p(F41 \cap \overline{K4}\, K2\, \overline{K3}\, \overline{K1}) + (1 - f41) \cdot (1 - p(F41 \cap \overline{K4}\, K2\, \overline{K3}\, \overline{K1}))) \cdot (f42 \cdot p(F42 \cap \overline{K4}\, K2\, \overline{K3}\, \overline{K1}) + (1 - f42) \cdot (1 - p(F42 \cap \overline{K4}\, K2\, \overline{K3}\, \overline{K1})))) \cdot (f43 \cdot p(F43 \cap \overline{K4}\, K2\, \overline{K3}\, \overline{K1}) + (1 - f43) \cdot (1 - p(F43 \cap \overline{K4}\, K2\, \overline{K3}\, \overline{K1})))) \cdot (f44 \cdot p(F44 \cap \overline{K4}\, K2\, \overline{K3}\, \overline{K1}) + (1 - f44) \cdot (1 - p(F44 \cap \overline{K4}\, K2\, \overline{K3}\, \overline{K1})))) \cdot (f45 \cdot p(F45 \cap \overline{K4}\, K2\, \overline{K3}\, \overline{K1}) + (1 - f45) \cdot (1 - p(F45 \cap \overline{K4}\, K2\, \overline{K3}\, \overline{K1})))) \cdot (f46 \cdot p(F46 \cap \overline{K4}\, K2\, \overline{K3}\, \overline{K1}) + (1 - f46) \cdot (1 - p(F46 \cap \overline{K4}\, K2\, \overline{K3}\, \overline{K1}))));$

$$a43 := 0. \tag{384}$$

$> \quad p(F41 \cap \overline{K4}\, \overline{K2}\, K3\, K1) := 1 - (1 - p(F41 \cap K3 \sim)) \cdot (1 - p(F41 \cap K1 \sim));$

$$p(F41 \cap \overline{K4}\, K2\, K3\, K1) := 0.6 \tag{385}$$

$> \quad p(F42 \cap \overline{K4}\, \overline{K2}\, K3\, K1) := 1 - (1 - p(F42 \cap K3 \sim)) \cdot (1 - p(F42 \cap K1 \sim));$

$$p(F42 \cap \overline{K4}\, K2\, K3\, K1) := 0.65 \tag{386}$$

$> \quad p(F43 \cap \overline{K4}\, \overline{K2}\, K3\, K1) := 1 - (1 - p(F43 \cap K3 \sim)) \cdot (1 - p(F43 \cap K1 \sim));$

$$p(F43 \text{'|'} \overline{K4}\, \overline{K2}\, K3\, K1) := 0.5 \tag{387}$$

$$> p(F44\text{'|'}\overline{K4}\, \overline{K2}\, K3\, K1) := 1 - (1 - p(F44\text{'|'}K3\text{'}\sim\text{'})) \cdot (1 - p(F44\text{'|'}K1\text{'}\sim\text{'}));$$
$$p(F44 \text{'|'} \overline{K4}\, K2\, K3\, K1) := 0.68 \tag{388}$$

$$> p(F45\text{'|'}\overline{K4}\, \overline{K2}\, K3\, K1) := 1 - (1 - p(F45\text{'|'}K3\text{'}\sim\text{'})) \cdot (1 - p(F45\text{'|'}K1\text{'}\sim\text{'}));$$
$$p(F45 \text{'|'} \overline{K4}\, K2\, K3\, K1) := 0 \tag{389}$$

$$> p(F46\text{'|'}\overline{K4}\, \overline{K2}\, K3\, K1) := 1 - (1 - p(F46\text{'|'}K3\text{'}\sim\text{'})) \cdot (1 - p(F46\text{'|'}K1\text{'}\sim\text{'}));$$
$$p(F46 \text{'|'} \overline{K4}\, K2\, K3\, K1) := 0.7 \tag{390}$$

$$> a44 := \big(f41 \cdot p(F41\text{'|'}\overline{K4}\, \overline{K2}\, K3\, K1) + (1-f41) \cdot (1 - p(F41\text{'|'}\overline{K4}\, \overline{K2}\, K3\, K1))\big) \cdot \big(f42 \cdot p(F42\text{'|'}\overline{K4}\, \overline{K2}\, K3\, K1)$$
$$+ (1-f42) \cdot (1 - p(F42\text{'|'}\overline{K4}\, \overline{K2}\, K3\, K1))\big) \cdot \big(f43 \cdot p(F43\text{'|'}\overline{K4}\, \overline{K2}\, K3\, K1) + (1-f43) \cdot (1 - p(F43\text{'|'}\overline{K4}$$
$$\overline{K2}\, K3\, K1))\big) \cdot \big(f44 \cdot p(F44\text{'|'}\overline{K4}\, \overline{K2}\, K3\, K1) + (1-f44) \cdot (1 - p(F44\text{'|'}\overline{K4}\, \overline{K2}\, K3\, K1))\big) \cdot \big(f45 \cdot p(F45\text{'|'}\overline{K4}$$
$$\overline{K2}\, K3\, K1) + (1-f45) \cdot (1 - p(F45\text{'|'}\overline{K4}\, \overline{K2}\, K3\, K1))\big) \cdot \big(f46 \cdot p(F46\text{'|'}\overline{K4}\, \overline{K2}\, K3\, K1) + (1-f46) \cdot (1$$
$$- p(F46\text{'|'}\overline{K4}\, \overline{K2}\, K3\, K1))\big);$$
$$a44 := 0.042 \tag{391}$$

$$> p(F41\text{'|'}\overline{K4}\, \overline{K2}\, K3\, \overline{K1}) := p(F41\text{'|'}K3\text{'}\sim\text{'});$$
$$p(F41 \text{'|'} \overline{K4}\, \overline{K2}\, K3\, \overline{K1}) := 0 \tag{392}$$

$$> p(F42\text{'|'}\overline{K4}\, \overline{K2}\, K3\, \overline{K1}) := p(F42\text{'|'}K3\text{'}\sim\text{'});$$
$$p(F42 \text{'|'} \overline{K4}\, \overline{K2}\, K3\, \overline{K1}) := 0.3 \tag{393}$$

$$> p(F43\text{'|'}\overline{K4}\, \overline{K2}\, K3\, \overline{K1}) := p(F43\text{'|'}K3\text{'}\sim\text{'});$$
$$p(F43 \text{'|'} \overline{K4}\, \overline{K2}\, K3\, \overline{K1}) := 0 \tag{394}$$

$$> p(F44\text{'|'}\overline{K4}\, \overline{K2}\, K3\, \overline{K1}) := p(F44\text{'|'}K3\text{'}\sim\text{'});$$
$$p(F44 \text{'|'} \overline{K4}\, \overline{K2}\, K3\, \overline{K1}) := 0.2 \tag{395}$$

$$> p(F45\text{'|'}\overline{K4}\, \overline{K2}\, K3\, \overline{K1}) := p(F45\text{'|'}K3\text{'}\sim\text{'});$$
$$p(F45 \text{'|'} \overline{K4}\, \overline{K2}\, K3\, \overline{K1}) := 0 \tag{396}$$

$$> p(F46\text{'|'}\overline{K4}\, \overline{K2}\, K3\, \overline{K1}) := p(F46\text{'|'}K3\text{'}\sim\text{'});$$
$$p(F46 \text{'|'} \overline{K4}\, \overline{K2}\, K3\, \overline{K1}) := 0.7 \tag{397}$$

$$> a45 := \big(f41 \cdot p(F41\text{'|'}\overline{K4}\, \overline{K2}\, K3\, \overline{K1}) + (1-f41) \cdot (1 - p(F41\text{'|'}\overline{K4}\, \overline{K2}\, K3\, \overline{K1}))\big) \cdot \big(f42 \cdot p(F42\text{'|'}\overline{K4}\, \overline{K2}\, K3\, \overline{K1})$$
$$+ (1-f42) \cdot (1 - p(F42\text{'|'}\overline{K4}\, \overline{K2}\, K3\, \overline{K1}))\big) \cdot \big(f43 \cdot p(F43\text{'|'}\overline{K4}\, \overline{K2}\, K3\, \overline{K1}) + (1-f43) \cdot (1 - p(F43\text{'|'}\overline{K4}$$
$$\overline{K2}\, K3\, \overline{K1}))\big) \cdot \big(f44 \cdot p(F44\text{'|'}\overline{K4}\, \overline{K2}\, K3\, \overline{K1}) + (1-f44) \cdot (1 - p(F44\text{'|'}\overline{K4}\, \overline{K2}\, K3\, \overline{K1}))\big) \cdot \big(f45 \cdot p(F45\text{'|'}\overline{K4}$$
$$\overline{K2}\, K3\, \overline{K1}) + (1-f45) \cdot (1 - p(F45\text{'|'}\overline{K4}\, \overline{K2}\, K3\, \overline{K1}))\big) \cdot \big(f46 \cdot p(F46\text{'|'}\overline{K4}\, \overline{K2}\, K3\, \overline{K1}) + (1=f46) \cdot (1$$
$$- p(F46\text{'|'}\overline{K4}\, \overline{K2}\, K3\, \overline{K1}))\big);$$
$$a45 := 0. \tag{398}$$

$$> p(F41\text{'|'}\overline{K4}\, \overline{K2}\, \overline{K3}\, K1) := p(F41\text{'|'}K1\text{'}\sim\text{'});$$
$$p(F41 \text{'|'} \overline{K4}\, \overline{K2}\, \overline{K3}\, K1) := 0.6 \tag{399}$$

$$> p(F42\text{'|'}\overline{K4}\, \overline{K2}\, \overline{K3}\, K1) := p(F42\text{'|'}K1\text{'}\sim\text{'});$$
$$p(F42 \text{'|'} \overline{K4}\, \overline{K2}\, \overline{K3}\, K1) := 0.5 \tag{400}$$

$$> p(F43\text{'|'}\overline{K4}\, \overline{K2}\, \overline{K3}\, K1) := p(F43\text{'|'}K1\text{'}\sim\text{'});$$
$$p(F43 \text{'|'} \overline{K4}\, \overline{K2}\, \overline{K3}\, K1) := 0.5 \tag{401}$$

$$> p(F44\text{'|'}\overline{K4}\, \overline{K2}\, \overline{K3}\, K1) := p(F44\text{'|'}K1\text{'}\sim\text{'});$$
$$p(F44 \text{'|'} \overline{K4}\, \overline{K2}\, \overline{K3}\, K1) := 0.6 \tag{402}$$

$$> p(F45\text{'|'}\overline{K4}\, \overline{K2}\, \overline{K3}\, K1) := p(F45\text{'|'}K1\text{'}\sim\text{'});$$
$$p(F45 \text{'|'} \overline{K4}\, \overline{K2}\, \overline{K3}\, K1) := 0 \tag{403}$$

$$> p(F46\text{'|'}\overline{K4}\, \overline{K2}\, \overline{K3}\, K1) := p(F46\text{'|'}K1\text{'}\sim\text{'});$$
$$p(F46 \text{'|'} \overline{K4}\, \overline{K2}\, \overline{K3}\, K1) := 0 \tag{404}$$

$$> a46 := \big(f41 \cdot p(F41\text{'|'}\overline{K4}\, \overline{K2}\, \overline{K3}\, K1) + (1-f41) \cdot (1 - p(F41\text{'|'}\overline{K4}\, \overline{K2}\, \overline{K3}\, K1))\big) \cdot \big(f42 \cdot p(F42\text{'|'}\overline{K4}\, \overline{K2}\, \overline{K3}\, K1)$$
$$+ (1-f42) \cdot (1 - p(F42\text{'|'}\overline{K4}\, \overline{K2}\, \overline{K3}\, K1))\big) \cdot \big(f43 \cdot p(F43\text{'|'}\overline{K4}\, \overline{K2}\, \overline{K3}\, K1) + (1-f43) \cdot (1 - p(F43\text{'|'}\overline{K4}$$

$\overline{K2}\,\overline{K3}\,K1)\,)\,)\cdot(f44\cdot p(F44\,\grave{}\,1\,\grave{}\,\overline{K4}\,\overline{K2}\,\overline{K3}\,K1)\,+\,(1-f44)\cdot(1-p(F44\,\grave{}\,1\,\grave{}\,\overline{K4}\,\overline{K2}\,\overline{K3}\,K1))\,)\cdot(f45\cdot p(F45\,\grave{}\,1\,\grave{}\,\overline{K4}\,\overline{K2}\,\overline{K3}\,K1)\,+\,(1-f45)\cdot(1-p(F45\,\grave{}\,1\,\grave{}\,\overline{K4}\,\overline{K2}\,\overline{K3}\,K1))\,)\cdot(f46\cdot p(F46\,\grave{}\,1\,\grave{}\,\overline{K4}\,\overline{K2}\,\overline{K3}\,K1)\,+\,(1-f46)\cdot(1-p(F46\,\grave{}\,1\,\grave{}\,\overline{K4}\,\overline{K2}\,\overline{K3}\,K1))\,)$;

$$a46 := 0.090 \tag{405}$$

> $a47 := 0$;

$$a47 := 0 \tag{406}$$

> $p(F41\,\grave{}\,1\,\grave{}\,K4\,K2\,K3\,K1) := 1-(1-p(F41\,\grave{}\,1\,\grave{}\,K1\grave{}\sim))\cdot(1-p(F41\,\grave{}\,1\,\grave{}\,K2\grave{}\sim))\cdot(1-p(F41\,\grave{}\,1\,\grave{}\,K3\grave{}\sim))\cdot(1-p(F41\,\grave{}\,1\,\grave{}\,K4\grave{}\sim))$;

$$p(F41 \,\grave{}\,1\,\grave{}\, K4\,K2\,K3\,K1) := 0.92 \tag{407}$$

> $p(F42\,\grave{}\,1\,\grave{}\,K4\,K2\,K3\,K1) := 1-(1-p(F42\,\grave{}\,1\,\grave{}\,K1\grave{}\sim))\cdot(1-p(F42\,\grave{}\,1\,\grave{}\,K2\grave{}\sim))\cdot(1-p(F42\,\grave{}\,1\,\grave{}\,K3\grave{}\sim))\cdot(1-p(F42\,\grave{}\,1\,\grave{}\,K4\grave{}\sim))$;

$$p(F42 \,\grave{}\,1\,\grave{}\, K4\,K2\,K3\,K1) := 0.87 \tag{408}$$

> $p(F43\,\grave{}\,1\,\grave{}\,K4\,K2\,K3\,K1) := 1-(1-p(F43\,\grave{}\,1\,\grave{}\,K1\grave{}\sim))\cdot(1-p(F43\,\grave{}\,1\,\grave{}\,K2\grave{}\sim))\cdot(1-p(F43\,\grave{}\,1\,\grave{}\,K3\grave{}\sim))\cdot(1-p(F43\,\grave{}\,1\,\grave{}\,K4\grave{}\sim))$;

$$p(F43 \,\grave{}\,1\,\grave{}\, K4\,K2\,K3\,K1) := 0.82 \tag{409}$$

> $p(F44\,\grave{}\,1\,\grave{}\,K4\,K2\,K3\,K1) := 1-(1-p(F44\,\grave{}\,1\,\grave{}\,K1\grave{}\sim))\cdot(1-p(F44\,\grave{}\,1\,\grave{}\,K2\grave{}\sim))\cdot(1-p(F44\,\grave{}\,1\,\grave{}\,K3\grave{}\sim))\cdot(1-p(F44\,\grave{}\,1\,\grave{}\,K4\grave{}\sim))$;

$$p(F44 \,\grave{}\,1\,\grave{}\, K4\,K2\,K3\,K1) := 0.84 \tag{410}$$

> $p(F45\,\grave{}\,1\,\grave{}\,K4\,K2\,K3\,K1) := 1-(1-p(F45\,\grave{}\,1\,\grave{}\,K1\grave{}\sim))\cdot(1-p(F45\,\grave{}\,1\,\grave{}\,K2\grave{}\sim))\cdot(1-p(F45\,\grave{}\,1\,\grave{}\,K3\grave{}\sim))\cdot(1-p(F45\,\grave{}\,1\,\grave{}\,K4\grave{}\sim))$;

$$p(F45 \,\grave{}\,1\,\grave{}\, K4\,K2\,K3\,K1) := 0.76 \tag{411}$$

> $p(F46\,\grave{}\,1\,\grave{}\,K4\,K2\,K3\,K1) := 1-(1-p(F46\,\grave{}\,1\,\grave{}\,K1\grave{}\sim))\cdot(1-p(F46\,\grave{}\,1\,\grave{}\,K2\grave{}\sim))\cdot(1-p(F46\,\grave{}\,1\,\grave{}\,K3\grave{}\sim))\cdot(1-p(F46\,\grave{}\,1\,\grave{}\,K4\grave{}\sim))$;

$$p(F46 \,\grave{}\,1\,\grave{}\, K4\,K2\,K3\,K1) := 0.82 \tag{412}$$

> $b40 := (f41\cdot p(F41\,\grave{}\,1\,\grave{}\,K4\,K2\,K3\,K1)\,+\,(1-f41)\cdot(1-p(F41\,\grave{}\,1\,\grave{}\,K4\,K2\,K3\,K1))\,)\cdot(f42\cdot p(F42\,\grave{}\,1\,\grave{}\,K4\,K2\,K3\,K1)\,+\,(1-f42)\cdot(1-p(F42\,\grave{}\,1\,\grave{}\,K4\,K2\,K3\,K1))\,)\cdot(f43\cdot p(F43\,\grave{}\,1\,\grave{}\,K4\,K2\,K3\,K1)\,+\,(1-f43)\cdot(1-p(F43\,\grave{}\,1\,\grave{}\,K4\,K2\,K3\,K1))\,)\cdot(f44\cdot p(F44\,\grave{}\,1\,\grave{}\,K4\,K2\,K3\,K1)\,+\,(1-f44)\cdot(1-p(F44\,\grave{}\,1\,\grave{}\,K4\,K2\,K3\,K1))\,)\cdot(f45\cdot p(F45\,\grave{}\,1\,\grave{}\,K4\,K2\,K3\,K1)\,+\,(1-f45)\cdot(1-p(F45\,\grave{}\,1\,\grave{}\,K4\,K2\,K3\,K1))\,)\cdot(f46\cdot p(F46\,\grave{}\,1\,\grave{}\,K4\,K2\,K3\,K1)\,+\,(1-f46)\cdot(1-p(F46\,\grave{}\,1\,\grave{}\,K4\,K2\,K3\,K1))\,)$;

$$b40 := 0.023 \tag{413}$$

> $p(F41\,\grave{}\,1\,\grave{}\,K4\,K2\,K3\,\overline{K1}) := 1-(1-p(F41\,\grave{}\,1\,\grave{}\,K4\grave{}\sim))\cdot(1-p(F41\,\grave{}\,1\,\grave{}\,K2\grave{}\sim))\cdot(1-p(F41\,\grave{}\,1\,\grave{}\,K3\grave{}\sim))$;
$$p(F41 \,\grave{}\,1\,\grave{}\, K4\,K2\,K3\,K1) := 0.80 \tag{414}$$

> $p(F42\,\grave{}\,1\,\grave{}\,K4\,K2\,K3\,\overline{K1}) := 1-(1-p(F42\,\grave{}\,1\,\grave{}\,K4\grave{}\sim))\cdot(1-p(F42\,\grave{}\,1\,\grave{}\,K2\grave{}\sim))\cdot(1-p(F42\,\grave{}\,1\,\grave{}\,K3\grave{}\sim))$;
$$p(F42 \,\grave{}\,1\,\grave{}\, K4\,K2\,K3\,K1) := 0.76 \tag{415}$$

> $p(F43\,\grave{}\,1\,\grave{}\,K4\,K2\,K3\,\overline{K1}) := 1-(1-p(F43\,\grave{}\,1\,\grave{}\,K4\grave{}\sim))\cdot(1-p(F43\,\grave{}\,1\,\grave{}\,K2\grave{}\sim))\cdot(1-p(F43\,\grave{}\,1\,\grave{}\,K3\grave{}\sim))$;
$$p(F43 \,\grave{}\,1\,\grave{}\, K4\,K2\,K3\,K1) := 0.65 \tag{416}$$

> $p(F44\,\grave{}\,1\,\grave{}\,K4\,K2\,K3\,\overline{K1}) := 1-(1-p(F44\,\grave{}\,1\,\grave{}\,K4\grave{}\sim))\cdot(1-p(F44\,\grave{}\,1\,\grave{}\,K2\grave{}\sim))\cdot(1-p(F44\,\grave{}\,1\,\grave{}\,K3\grave{}\sim))$;
$$p(F44 \,\grave{}\,1\,\grave{}\, K4\,K2\,K3\,K1) := 0.60 \tag{417}$$

> $p(F45\,\grave{}\,1\,\grave{}\,K4\,K2\,K3\,\overline{K1}) := 1-(1-p(F45\,\grave{}\,1\,\grave{}\,K4\grave{}\sim))\cdot(1-p(F45\,\grave{}\,1\,\grave{}\,K2\grave{}\sim))\cdot(1-p(F45\,\grave{}\,1\,\grave{}\,K3\grave{}\sim))$;
$$p(F45 \,\grave{}\,1\,\grave{}\, K4\,K2\,K3\,K1) := 0.76 \tag{418}$$

> $p(F46\,\grave{}\,1\,\grave{}\,K4\,K2\,K3\,\overline{K1}) := 1-(1-p(F46\,\grave{}\,1\,\grave{}\,K4\grave{}\sim))\cdot(1-p(F46\,\grave{}\,1\,\grave{}\,K2\grave{}\sim))\cdot(1-p(F46\,\grave{}\,1\,\grave{}\,K3\grave{}\sim))$;
$$p(F46 \,\grave{}\,1\,\grave{}\, K4\,K2\,K3\,K1) := 0.82 \tag{419}$$

> $b41 := (f41\cdot p(F41\,\grave{}\,1\,\grave{}\,K4\,K2\,K3\,\overline{K1})\,+\,(1-f41)\cdot(1-p(F41\,\grave{}\,1\,\grave{}\,K4\,K2\,K3\,\overline{K1}))\,)\cdot(f42\cdot p(F42\,\grave{}\,1\,\grave{}\,K4\,K2\,K3\,\overline{K1})\,+\,(1-f42)\cdot(1-p(F42\,\grave{}\,1\,\grave{}\,K4\,K2\,K3\,\overline{K1}))\,)\cdot(f43\cdot p(F43\,\grave{}\,1\,\grave{}\,K4\,K2\,K3\,\overline{K1})\,+\,(1-f43)\cdot(1-p(F43\,\grave{}\,1\,\grave{}\,K4\,K2\,K3\,\overline{K1}))\,)\cdot(f44\cdot p(F44\,\grave{}\,1\,\grave{}\,K4\,K2\,K3\,\overline{K1})\,+\,(1-f44)\cdot(1-p(F44\,\grave{}\,1\,\grave{}\,K4\,K2\,K3\,\overline{K1}))\,)\cdot(f45\cdot p(F45\,\grave{}\,1\,\grave{}\,K4\,K2\,K3\,\overline{K1})\,+\,(1-f45)\cdot(1-p(F45\,\grave{}\,1\,\grave{}\,K4\,K2\,K3\,\overline{K1}))\,)\cdot(f46\cdot p(F46\,\grave{}\,1\,\grave{}\,K4\,K2\,K3\,\overline{K1})\,+\,(1-f46)\cdot(1-p(F46\,\grave{}\,1\,\grave{}\,K4\,K2\,K3\,\overline{K1}))\,)$;

$$b41 := 0.010 \tag{420}$$

> $p(F41\,\grave{}\,1\,\grave{}\,K4\,K2\,\overline{K3}\,K1) := 1-(1-p(F41\,\grave{}\,1\,\grave{}\,K1\grave{}\sim))\cdot(1-p(F41\,\grave{}\,1\,\grave{}\,K2\grave{}\sim))\cdot(1-p(F41\,\grave{}\,1\,\grave{}\,K4\grave{}\sim))$;

$$\tag{421}$$

$$p\left(F41 \mid K4\, K2\, \overline{K3}\, K1\right) := 0.92 \tag{421}$$

$$p\left(F42 \mid K4\, K2\, \overline{K3}\, K1\right) := 1 - \left(1 - p\left(F42 \mid K1 \sim\right)\right) \cdot \left(1 - p\left(F42 \mid K2 \sim\right)\right) \cdot \left(1 - p\left(F42 \mid K4 \sim\right)\right);$$
$$p\left(F42 \mid K4\, K2\, K3\, K1\right) := 0.82 \tag{422}$$

$$p\left(F43 \mid K4\, K2\, \overline{K3}\, K1\right) := 1 - \left(1 - p\left(F43 \mid K1 \sim\right)\right) \cdot \left(1 - p\left(F43 \mid K2 \sim\right)\right) \cdot \left(1 - p\left(F43 \mid K4 \sim\right)\right);$$
$$p\left(F43 \mid K4\, K2\, K3\, K1\right) := 0.82 \tag{423}$$

$$p\left(F44 \mid K4\, K2\, \overline{K3}\, K1\right) := 1 - \left(1 - p\left(F44 \mid K1 \sim\right)\right) \cdot \left(1 - p\left(F44 \mid K2 \sim\right)\right) \cdot \left(1 - p\left(F44 \mid K4 \sim\right)\right);$$
$$p\left(F44 \mid K4\, K2\, K3\, K1\right) := 0.80 \tag{424}$$

$$p\left(F45 \mid K4\, K2\, \overline{K3}\, K1\right) := 1 - \left(1 - p\left(F45 \mid K1 \sim\right)\right) \cdot \left(1 - p\left(F45 \mid K2 \sim\right)\right) \cdot \left(1 - p\left(F45 \mid K4 \sim\right)\right);$$
$$p\left(F45 \mid K4\, K2\, K3\, K1\right) := 0.76 \tag{425}$$

$$p\left(F46 \mid K4\, K2\, \overline{K3}\, K1\right) := 1 - \left(1 - p\left(F46 \mid K1 \sim\right)\right) \cdot \left(1 - p\left(F46 \mid K2 \sim\right)\right) \cdot \left(1 - p\left(F46 \mid K4 \sim\right)\right);$$
$$p\left(F46 \mid K4\, K2\, K3\, K1\right) := 0.4 \tag{426}$$

$$b42 := \left(f41 \cdot p\left(F41 \mid K4\, K2\, \overline{K3}\, K1\right) + \left(1 - f41\right) \cdot \left(1 - p\left(F41 \mid K4\, K2\, \overline{K3}\, K1\right)\right)\right) \cdot \left(f42 \cdot p\left(F42 \mid K4\, K2\, \overline{K3}\, K1\right)\right.$$
$$+ \left(1 - f42\right) \cdot \left(1 - p\left(F42 \mid K4\, K2\, \overline{K3}\, K1\right)\right)\right) \cdot \left(f43 \cdot p\left(F43 \mid K4\, K2\, \overline{K3}\, K1\right) + \left(1 - f43\right) \cdot \left(1 - p\left(F43 \mid K4\, K2\,\right.\right.$$
$$\left.\left.\overline{K3}\, K1\right)\right)\right) \cdot \left(f44 \cdot p\left(F44 \mid K4\, K2\, \overline{K3}\, K1\right) + \left(1 - f44\right) \cdot \left(1 - p\left(F44 \mid K4\, K2\, \overline{K3}\, K1\right)\right)\right) \cdot \left(f45 \cdot p\left(F45 \mid K4\, K2\,\right.\right.$$
$$\left.\overline{K3}\, K1\right) + \left(1 - f45\right) \cdot \left(1 - p\left(F45 \mid K4\, K2\, \overline{K3}\, K1\right)\right)\right) \cdot \left(f46 \cdot p\left(F46 \mid K4\, K2\, \overline{K3}\, K1\right) + \left(1 - f46\right) \cdot \left(1\right.\right.$$
$$\left.\left. - p\left(F46 \mid K4\, K2\, \overline{K3}\, K1\right)\right)\right);$$
$$b42 := 0.072 \tag{427}$$

$$p\left(F41 \mid K4\, K2\, \overline{K3}\, \overline{K1}\right) := 1 - \left(1 - p\left(F41 \mid K4 \sim\right)\right) \cdot \left(1 - p\left(F41 \mid K2 \sim\right)\right);$$
$$p\left(F41 \mid K4\, K2\, K3\, K1\right) := 0.80 \tag{428}$$

$$p\left(F42 \mid K4\, K2\, \overline{K3}\, \overline{K1}\right) := 1 - \left(1 - p\left(F42 \mid K4 \sim\right)\right) \cdot \left(1 - p\left(F42 \mid K2 \sim\right)\right);$$
$$p\left(F42 \mid K4\, K2\, K3\, K1\right) := 0.65 \tag{429}$$

$$p\left(F43 \mid K4\, K2\, \overline{K3}\, \overline{K1}\right) := 1 - \left(1 - p\left(F43 \mid K4 \sim\right)\right) \cdot \left(1 - p\left(F43 \mid K2 \sim\right)\right);$$
$$p\left(F43 \mid K4\, K2\, K3\, K1\right) := 0.65 \tag{430}$$

$$p\left(F44 \mid K4\, K2\, \overline{K3}\, \overline{K1}\right) := 1 - \left(1 - p\left(F44 \mid K4 \sim\right)\right) \cdot \left(1 - p\left(F44 \mid K2 \sim\right)\right);$$
$$p\left(F44 \mid K4\, K2\, K3\, K1\right) := 0.5 \tag{431}$$

$$p\left(F45 \mid K4\, K2\, \overline{K3}\, \overline{K1}\right) := 1 - \left(1 - p\left(F45 \mid K4 \sim\right)\right) \cdot \left(1 - p\left(F45 \mid K2 \sim\right)\right);$$
$$p\left(F45 \mid K4\, K2\, K3\, K1\right) := 0.76 \tag{432}$$

$$p\left(F46 \mid K4\, K2\, \overline{K3}\, \overline{K1}\right) := 1 - \left(1 - p\left(F46 \mid K4 \sim\right)\right) \cdot \left(1 - p\left(F46 \mid K2 \sim\right)\right);$$
$$p\left(F46 \mid K4\, K2\, K3\, K1\right) := 0.4 \tag{433}$$

$$b43 := \left(f41 \cdot p\left(F41 \mid K4\, K2\, \overline{K3}\, \overline{K1}\right) + \left(1 - f41\right) \cdot \left(1 - p\left(F41 \mid K4\, K2\, \overline{K3}\, \overline{K1}\right)\right)\right) \cdot \left(f42 \cdot p\left(F42 \mid K4\, K2\, \overline{K3}\, \overline{K1}\right)\right.$$
$$+ \left(1 - f42\right) \cdot \left(1 - p\left(F42 \mid K4\, K2\, \overline{K3}\, \overline{K1}\right)\right)\right) \cdot \left(f43 \cdot p\left(F43 \mid K4\, K2\, \overline{K3}\, \overline{K1}\right) + \left(1 - f43\right) \cdot \left(1 - p\left(F43 \mid K4\, K2\,\right.\right.$$
$$\left.\left.\overline{K3}\, \overline{K1}\right)\right)\right) \cdot \left(f44 \cdot p\left(F44 \mid K4\, K2\, \overline{K3}\, \overline{K1}\right) + \left(1 - f44\right) \cdot \left(1 - p\left(F44 \mid K4\, K2\, \overline{K3}\, \overline{K1}\right)\right)\right) \cdot \left(f45 \cdot p\left(F45 \mid K4\, K2\, \overline{K3}\,\right.\right.$$
$$\left.\overline{K1}\right) + \left(1 - f45\right) \cdot \left(1 - p\left(F45 \mid K4\, K2\, \overline{K3}\, \overline{K1}\right)\right)\right) \cdot \left(f46 \cdot p\left(F46 \mid K4\, K2\, \overline{K3}\, \overline{K1}\right) + \left(1 - f46\right) \cdot \left(1\right.\right.$$
$$\left.\left. - p\left(F46 \mid K4\, K2\, \overline{K3}\, \overline{K1}\right)\right)\right);$$
$$b43 := 0.025 \tag{434}$$

$$p\left(F41 \mid K4\, \overline{K2}\, K3\, K1\right) := 1 - \left(1 - p\left(F41 \mid K1 \sim\right)\right) \cdot \left(1 - p\left(F41 \mid K3 \sim\right)\right) \cdot \left(1 - p\left(F41 \mid K4 \sim\right)\right);$$
$$p\left(F41 \mid K4\, K2\, K3\, K1\right) := 0.80 \tag{435}$$

$$p\left(F42 \mid K4\, \overline{K2}\, K3\, K1\right) := 1 - \left(1 - p\left(F42 \mid K1 \sim\right)\right) \cdot \left(1 - p\left(F42 \mid K3 \sim\right)\right) \cdot \left(1 - p\left(F42 \mid K4 \sim\right)\right);$$
$$p\left(F42 \mid K4\, K2\, K3\, K1\right) := 0.76 \tag{436}$$

$$p\left(F43 \mid K4\, \overline{K2}\, K3\, K1\right) := 1 - \left(1 - p\left(F43 \mid K1 \sim\right)\right) \cdot \left(1 - p\left(F43 \mid K3 \sim\right)\right) \cdot \left(1 - p\left(F43 \mid K4 \sim\right)\right);$$
$$p\left(F43 \mid K4\, K2\, K3\, K1\right) := 0.65 \tag{437}$$

$$p\left(F44 \mid K4\, \overline{K2}\, K3\, K1\right) := 1 - \left(1 - p\left(F44 \mid K1 \sim\right)\right) \cdot \left(1 - p\left(F44 \mid K3 \sim\right)\right) \cdot \left(1 - p\left(F44 \mid K4 \sim\right)\right);$$
$$p\left(F44 \mid K4\, K2\, K3\, K1\right) := 0.84 \tag{438}$$

$$p\left(F45 \mid K4\, \overline{K2}\, K3\, K1\right) := 1 - \left(1 - p\left(F45 \mid K1 \sim\right)\right) \cdot \left(1 - p\left(F45 \mid K3 \sim\right)\right) \cdot \left(1 - p\left(F45 \mid K4 \sim\right)\right);$$
$$\tag{439}$$

$$p(F45 \ ` 1` K4 \overline{K2} K3 K1) := 0.2 \tag{439}$$

$$> p(F46` 1` K4\overline{K2} K3 K1) := 1 - (1 - p(F46` 1` K1` \sim `)) \cdot (1 - p(F46` 1` K3` \sim `)) \cdot (1 - p(F46` 1` K4` \sim `));$$
$$p(F46 \ ` 1` K4 \ K2 \ K3 \ K1) := 0.82 \tag{440}$$

$$> b44 := \left( f41 \cdot p(F41` 1` K4 \overline{K2} K3 K1) + (1 - f41) \cdot (1 - p(F41` 1` K4 \overline{K2} K3 K1)) \right) \cdot \left( f42 \cdot p(F42` 1` K4 \overline{K2} K3 K1) \right.$$
$$+ (1 - f42) \cdot (1 - p(F42` 1` K4 \overline{K2} K3 K1)) \right) \cdot \left( f43 \cdot p(F43` 1` K4 \overline{K2} K3 K1) + (1 - f43) \cdot (1 - p(F43` 1` K4 \right.$$
$$\left. \overline{K2} K3 K1)) \right) \cdot \left( f44 \cdot p(F44` 1` K4 \overline{K2} K3 K1) + (1 - f44) \cdot (1 - p(F44` 1` K4 \overline{K2} K3 K1)) \right) \cdot \left( f45 \cdot p(F45` 1` K4 \right.$$
$$\left. \overline{K2} K3 K1) + (1 - f45) \cdot (1 - p(F45` 1` K4 \overline{K2} K3 K1)) \right) \cdot \left( f46 \cdot p(F46` 1` K4 \overline{K2} K3 K1) + (1 - f46) \cdot (1 \right.$$
$$\left. - p(F46` 1` K4 \overline{K2} K3 K1)) \right);$$
$$b44 := 0.049 \tag{441}$$

$$> p(F41` 1` K4 \overline{K2} K3 \overline{K1}) := 1 - (1 - p(F41` 1` K4` \sim `)) \cdot (1 - p(F41` 1` K3` \sim `));$$
$$p(F41 \ ` 1` K4 \ K2 \ K3 \ K1) := 0.5 \tag{442}$$

$$> p(F42` 1` K4 \overline{K2} K3 \overline{K1}) := 1 - (1 - p(F42` 1` K4` \sim `)) \cdot (1 - p(F42` 1` K3` \sim `));$$
$$p(F42 \ ` 1` K4 \ K2 \ K3 \ K1) := 0.51 \tag{443}$$

$$> p(F43` 1` K4 \overline{K2} K3 \overline{K1}) := 1 - (1 - p(F43` 1` K4` \sim `)) \cdot (1 - p(F43` 1` K3` \sim `));$$
$$p(F43 \ ` 1` K4 \ K2 \ K3 \ K1) := 0.3 \tag{444}$$

$$> p(F44` 1` K4 \overline{K2} K3 \overline{K1}) := 1 - (1 - p(F44` 1` K4` \sim `)) \cdot (1 - p(F44` 1` K3` \sim `));$$
$$p(F44 \ ` 1` K4 \ K2 \ K3 \ K1) := 0.60 \tag{445}$$

$$> p(F45` 1` K4 \overline{K2} K3 \overline{K1}) := 1 - (1 - p(F45` 1` K4` \sim `)) \cdot (1 - p(F45` 1` K3` \sim `));$$
$$p(F45 \ ` 1` K4 \ K2 \ K3 \ K1) := 0.2 \tag{446}$$

$$> p(F46` 1` K4 \overline{K2} K3 \overline{K1}) := 1 - (1 - p(F46` 1` K4` \sim `)) \cdot (1 - p(F46` 1` K3` \sim `));$$
$$p(F46 \ ` 1` K4 \ K2 \ K3 \ K1) := 0.82 \tag{447}$$

$$> b45 := \left( f41 \cdot p(F41` 1` K4 \overline{K2} K3 \overline{K1}) + (1 - f41) \cdot (1 - p(F41` 1` K4 \overline{K2} K3 \overline{K1})) \right) \cdot \left( f42 \cdot p(F42` 1` K4 \overline{K2} K3 \overline{K1}) \right.$$
$$+ (1 - f42) \cdot (1 - p(F42` 1` K4 \overline{K2} K3 \overline{K1})) \right) \cdot \left( f43 \cdot p(F43` 1` K4 \overline{K2} K3 \overline{K1}) + (1 - f43) \cdot (1 - p(F43` 1` K4 \right.$$
$$\left. \overline{K2} K3 \overline{K1})) \right) \cdot \left( f44 \cdot p(F44` 1` K4 \overline{K2} K3 \overline{K1}) + (1 - f44) \cdot (1 - p(F44` 1` K4 \overline{K2} K3 \overline{K1})) \right) \cdot \left( f45 \cdot p(F45` 1` K4 \right.$$
$$\left. \overline{K2} K3 \overline{K1}) + (1 - f45) \cdot (1 - p(F45` 1` K4 \overline{K2} K3 \overline{K1})) \right) \cdot \left( f46 \cdot p(F46` 1` K4 \overline{K2} K3 \overline{K1}) + (1 - f46) \cdot (1 \right.$$
$$\left. - p(F46` 1` K4 \overline{K2} K3 \overline{K1})) \right);$$
$$b45 := 0.0068 \tag{448}$$

$$> p(F41` 1` K4 \overline{K2} \ \overline{K3} K1) := 1 - (1 - p(F41` 1` K1` \sim `)) \cdot (1 - p(F41` 1` K4` \sim `));$$
$$p(F41 \ ` 1` K4 \ K2 \ K3 \ K1) := 0.80 \tag{449}$$

$$> p(F42` 1` K4 \overline{K2} \ \overline{K3} K1) := 1 - (1 - p(F42` 1` K1` \sim `)) \cdot (1 - p(F42` 1` K4` \sim `));$$
$$p(F42 \ ` 1` K4 \ K2 \ K3 \ K1) := 0.65 \tag{450}$$

$$> p(F43` 1` K4 \overline{K2} \ \overline{K3} K1) := 1 - (1 - p(F43` 1` K1` \sim `)) \cdot (1 - p(F43` 1` K4` \sim `));$$
$$p(F43 \ ` 1` K4 \ K2 \ K3 \ K1) := 0.65 \tag{451}$$

$$> p(F44` 1` K4 \overline{K2} \ \overline{K3} K1) := 1 - (1 - p(F44` 1` K1` \sim `)) \cdot (1 - p(F44` 1` K4` \sim `));$$
$$p(F44 \ ` 1` K4 \ K2 \ K3 \ K1) := 0.80 \tag{452}$$

$$> p(F45` 1` K4 \overline{K2} \ \overline{K3} K1) := 1 - (1 - p(F45` 1` K1` \sim `)) \cdot (1 - p(F45` 1` K4` \sim `));$$
$$p(F45 \ ` 1` K4 \ K2 \ K3 \ K1) := 0.2 \tag{453}$$

$$> p(F46` 1` K4 \overline{K2} \ \overline{K3} K1) := 1 - (1 - p(F46` 1` K1` \sim `)) \cdot (1 - p(F46` 1` K4` \sim `));$$
$$p(F46 \ ` 1` K4 \ K2 \ K3 \ K1) := 0.4 \tag{454}$$

$$> b46 := \left( f41 \cdot p(F41` 1` K4 \overline{K2} \ \overline{K3} K1) + (1 - f41) \cdot (1 - p(F41` 1` K4 \overline{K2} \ \overline{K3} K1)) \right) \cdot \left( f42 \cdot p(F42` 1` K4 \overline{K2} \ \overline{K3} K1) \right.$$
$$+ (1 - f42) \cdot (1 - p(F42` 1` K4 \overline{K2} \ \overline{K3} K1)) \right) \cdot \left( f43 \cdot p(F43` 1` K4 \overline{K2} \ \overline{K3} K1) + (1 - f43) \cdot (1 - p(F43` 1` K4 \overline{K2} \right.$$
$$\left. \overline{K3} K1)) \right) \cdot \left( f44 \cdot p(F44` 1` K4 \overline{K2} \ \overline{K3} K1) + (1 - f44) \cdot (1 - p(F44` 1` K4 \overline{K2} \ \overline{K3} K1)) \right) \cdot \left( f45 \cdot p(F45` 1` K4 \overline{K2} \right.$$
$$\left. \overline{K3} K1) + (1 - f45) \cdot (1 - p(F45` 1` K4 \overline{K2} \ \overline{K3} K1)) \right) \cdot \left( f46 \cdot p(F46` 1` K4 \overline{K2} \ \overline{K3} K1) + (1 - f46) \cdot (1 \right.$$
$$\left. - p(F46` 1` K4 \overline{K2} \ \overline{K3} K1)) \right);$$
$$b46 := 0.13 \tag{455}$$

$$> p(F41` 1` K4 \overline{K2} \ \overline{K3} \overline{K1}) := p(F41` 1` K4` \sim `);$$

$$p(F41 \text{'\|'} K4 \overline{K2} \, \overline{K3} \, \overline{K1}) := 0.5 \tag{456}$$

$$> p(F42\text{'\|'}K4\overline{K2} \, \overline{K3} \, \overline{K1}) := p(F42\text{'\|'}K4\text{'--'});$$
$$p(F42 \text{'\|'} K4 \overline{K2} \, \overline{K3} \, \overline{K1}) := 0.3 \tag{457}$$

$$> p(F43\text{'\|'}K4\overline{K2} \, \overline{K3} \, \overline{K1}) := p(F43\text{'\|'}K4\text{'--'});$$
$$p(F43 \text{'\|'} K4 \overline{K2} \, \overline{K3} \, \overline{K1}) := 0.3 \tag{458}$$

$$> p(F44\text{'\|'}K4\overline{K2} \, \overline{K3} \, \overline{K1}) := p(F44\text{'\|'}K4\text{'--'});$$
$$p(F44 \text{'\|'} K4 \overline{K2} \, \overline{K3} \, \overline{K1}) := 0.5 \tag{459}$$

$$> p(F45\text{'\|'}K4\overline{K2} \, \overline{K3} \, \overline{K1}) := p(F45\text{'\|'}K4\text{'--'});$$
$$p(F45 \text{'\|'} K4 \overline{K2} \, \overline{K3} \, \overline{K1}) := 0.2 \tag{460}$$

$$> p(F46\text{'\|'}K4\overline{K2} \, \overline{K3} \, \overline{K1}) := p(F46\text{'\|'}K4\text{'--'});$$
$$p(F46 \text{'\|'} K4 \overline{K2} \, \overline{K3} \, \overline{K1}) := 0.4 \tag{461}$$

$$> b47 := \left( f41 \cdot p(F41\text{'\|'}K4\overline{K2} \, \overline{K3} \, \overline{K1}) + (1 - f41) \cdot (1 - p(F41\text{'\|'}K4\overline{K2} \, \overline{K3} \, \overline{K1})) \right) \cdot \left( f42 \cdot p(F42\text{'\|'}K4\overline{K2} \, \overline{K3} \, \overline{K1}) + (1 - f42) \cdot (1 - p(F42\text{'\|'}K4\overline{K2} \, \overline{K3} \, \overline{K1})) \right) \cdot \left( f43 \cdot p(F43\text{'\|'}K4\overline{K2} \, \overline{K3} \, \overline{K1}) + (1 - f43) \cdot (1 - p(F43\text{'\|'}K4\overline{K2} \, \overline{K3} \, \overline{K1})) \right) \cdot \left( f44 \cdot p(F44\text{'\|'}K4\overline{K2} \, \overline{K3} \, \overline{K1}) + (1 - f44) \cdot (1 - p(F44\text{'\|'}K4\overline{K2} \, \overline{K3} \, \overline{K1})) \right) \cdot \left( f45 \cdot p(F45\text{'\|'}K4\overline{K2} \, \overline{K3} \, \overline{K1}) + (1 - f45) \cdot (1 - p(F45\text{'\|'}K4\overline{K2} \, \overline{K3} \, \overline{K1})) \right) \cdot \left( f46 \cdot p(F46\text{'\|'}K4\overline{K2} \, \overline{K3} \, \overline{K1}) + (1 - f46) \cdot (1 - p(F46\text{'\|'}K4\overline{K2} \, \overline{K3} \, \overline{K1})) \right);$$
$$b47 := 0.011 \tag{462}$$

$$>$$
$$> c4 := 0.25;$$
$$c4 := 0.25 \tag{463}$$

$$> eq4 := x4 = 1 \left/ \left( 1 + (a40 \cdot x2 \cdot x3 \cdot x1 + a41 \cdot x2 \cdot x3 \cdot (1 - x1) + a42 \cdot x2 \cdot (1 - x3) \cdot x1 + a43 \cdot x2 \cdot (1 - x3) \cdot (1 - x1) \cdot \right. \right.$$
$$+ 0 + 0 + 0 + 0 + 0 + 0 + 0 + 0 + 0 + 0 + a44 \cdot (1 - x2) \cdot x3 \cdot x1 + a45 \cdot (1 - x2) \cdot x3 \cdot (1 - x1) + a46 \cdot (1 - x2) \cdot (1 - x3) \cdot x1 + a47 \cdot (1 - x2) \cdot (1 - x3) \cdot (1 - x1)) \left/ (b40 \cdot x2 \cdot x3 \cdot x1 + b41 \cdot x2 \cdot x3 \cdot (1 - x1) + b42 \cdot x2 \right.$$
$$\cdot (1 - x3) \cdot x1 + b43 \cdot x2 \cdot (1 - x3) \cdot (1 - x1) + b44 \cdot (1 - x2) \cdot x3 \cdot x1 + b45 \cdot (1 - x2) \cdot x3 \cdot (1 - x1) + b46 \cdot (1 - x2) \cdot (1 - x3) \cdot x1 + b47 \cdot (1 - x2) \cdot (1 - x3) \cdot (1 - x1)) \cdot \left. \left. \left( \frac{(1 - c4)}{c4} \right) \right) \right);$$

$$eq4 := x4 = 1 \left/ (1 + (3.0 \, (0.030 \, x2 \, x3 \, x1 + 0.0036 \, x2 \, x3 \, (1 - x1) + 0.084 \, x2 \, (1 - x3) \, x1 \right. \tag{464}$$
$$+ 0.042 \, (1 - x2) \, x3 \, x1 + 0.090 \, (1 - x2) \, (1 - x3) \, x1)) \left/ (0.023 \, x2 \, x3 \, x1 + 0.010 \, x2 \, x3 \, (1 - x1) + 0.072 \, x2 \, (1 - x3) \, x1 + 0.025 \, x2 \, (1 - x3) \, (1 - x1) + 0.049 \, (1 - x2) \, x3 \, x1 \right.$$
$$+ 0.0068 \, (1 - x2) \, x3 \, (1 - x1) + 0.13 \, (1 - x2) \, (1 - x3) \, x1 + 0.011 \, (1 - x2) \, (1 - x3) \, (1 - x1)))$$

$$>$$
$$> S := fsolve(\{ eq1, eq2, eq3, eq4 \}, \{ x1, x2, x3, x4 \}, \{ x1 = 0 .. 1, x2 = 0 .. 1, x3 = 0 .. 1, x4 = 0 .. 1 \});$$
$$S := \{ x1 = 0.096, x2 = 0.18, x3 = 0.032, x4 = 0.48 \} \tag{465}$$

$$>$$
$$> AF(1) := \frac{x1}{c1}; AF(2) := \frac{x2}{c2}; AF(3) := \frac{x3}{c3}; AF(4) := \frac{x4}{c4};$$
$$AF(1) := 4.0 \, x1$$
$$AF(2) := 4.0 \, x2$$
$$AF(3) := 4.0 \, x3$$
$$AF(4) := 4.0 \, x4 \tag{466}$$

```
 |> assign(S);
 |
 |> AF(1) := x1/c1 ; AF(2) := x2/c2 ; AF(3) := x3/c3 ; AF(4) := x4/c4 ;
```

$$AF(1) := 0.38$$
$$AF(2) := 0.72$$
$$AF(3) := 0.13$$
$$AF(4) := 1.9 \qquad\qquad (467)$$

```
 |>
```

**Patentansprüche**

1. Universell einsetzbares Verfahren zur Anwendung auf zahlreichen Wissensgebieten, zum Beispiel der Erdbebenforschung, der geologischen Prospektion, der Kriminalistik, der Flugunfalluntersuchungen, der on-board-Diagnostik im Automobil- und Flugzeugbau und in der Medizin, unter anderem zur Auswertung von EKG-Aufzeichnungen, immer dann, wenn entschieden werden muß über das Zutreffen mehrerer hypothetischer Annahmen oder Diagnosen, die Ursachen sind für eine Anzahl von Indizien oder Symptomen, wobei neben der Symptommenge berücksichtigt wird, daß die Zunahme der Wahrscheinlichkeit für eine beliebige Diagnose zu einer Abnahme der Wahrscheinlichkeit einer konkurrierenden Diagnose führt, verfahrenstechnisch **dadurch gekennzeichnet, daß** ein algebraisches Verfahren eingesetzt wird, derart, daß für vier konkurrierende Diagnosen $K_i'$, i:= 1,..., 4, der jeweilige Aufwertungsfaktor AF(i) und die a-posteriori-Wahrscheinlichkeit $x_i$ ermittelt wird, wobei der höchste Aufwertungsfaktor die zutreffende Diagnose bestimmt, und wobei jedem $K_i'$ eine Menge von Folge-Ereignissen $F_{ij}$ mit j:= 1,..., 6 zugehört, mit den Eigenschaften, daß jedes $F_{ij}$ die Folge von mindestens zwei Diagnosen ist, und daß die Elemente in {$K_i'$, i:= 1,..., 4} stochastisch unabhängig und stochastisch selbständig sind, und daß {$K_i'$, i:=1,..., 4} entweder alle Ursachen der $F_{ij}$ enthält oder durch weitere $K_i$ in negierter Form ergänzt wird, so daß die Wertungsgleichungen

$$x_1 := p(K_1 \mid F_{11} \dots F_{16}\, K_2'\, K_3'\, K_4'),$$

$$x_2 := p(K_2 \mid F_{21} \dots F_{26}\, K_1'\, K_3'\, K_4'),$$

$$x_3 := p(K_3 \mid F_{31} \dots F_{36}\, K_2'\, K_1'\, K_4'),$$

$$x_4 := p(K_4 \mid F_{41} \dots F_{46}\, K_2'\, K_3'\, K_1'),$$

folgen, die nach einer Umformung übergehen in $\quad x_i := \dfrac{1}{1+\dfrac{Z_i}{N_i}\cdot\dfrac{p(\overline{K_i})}{p(K_i)}},\ i:=1,\dots,4,$ mit

$$Z_1 := p(F_{11}\dots F_{16} \mid \overline{K_1}\, K_2'\, K_3'\, K_4')\ \text{und}\ N_1 := p(F_{11}\dots F_{16} \mid K_1\, K_2'\, K_3'\, K_4'),$$

Als <u>W</u>ertungsumgebung von $K_1'$ (in kurzer Schreibweise:$W(K_1')$) wird ein
Freignissen bezeichnet, der diejenigen Elemente der Kausalstruktur entl

$$Z_3 := p(F_{31}...F_{36} \,|\, \overline{K}_3 K_2{}' K_1{}' K_4{}') \text{ und } N_3 := p(F_{31}...F_{36} \,|\, K_3 K_2{}' K_1{}' K_4{}') \,,$$

$$Z_4 := p(F_{41}...F_{46} \,|\, \overline{K}_4 K_2{}' K_3{}' K_1{}') \text{ und } N_4 := p(F_{41}...F_{46} \,|\, K_4 K_2{}' K_3{}' K_1{}') \,,$$

wobei die $Z_i$ und $N_i$ einer Linearen Interpolation unterzogen werden, so daß man zum Beispiel für $Z_1$

$$p(F_{11}...F_{16} \,|\, \overline{K}_1 K_2{}' K_3{}' K_4{}') =$$

$$p(F_{11}...F_{16} \,|\, \overline{K}_1 K_2 K_3 K_4) \cdot x_2 \cdot x_3 \cdot x_4 + p(F_{11}...F_{16} \,|\, \overline{K}_1 K_2 K_3 \overline{K}_4) \cdot x_2 \cdot x_3 \cdot \overline{x} \;+$$

$$p(F_{11}...F_{16} \,|\, \overline{K}_1 K_2 \overline{K}_3 K_4) \cdot x_2 \cdot \overline{x}_3 \cdot x_4 + p(F_{11}...F_{16} \,|\, \overline{K}_1 K_2 \overline{K}_3 \overline{K}_4) \cdot x_2 \cdot \overline{x}_3 \cdot \overline{x}_4 \;+$$

$$p(F_{11}...F_{16} \,|\, \overline{K}_1 \overline{K}_2 K_3 K_4) \cdot \overline{x}_2 \cdot x_3 \cdot x_4 + p(F_{11}...F_{16} \,|\, \overline{K}_1 \overline{K}_2 K_3 \overline{K}_4) \cdot \overline{x}_2 \cdot x_3 \cdot \overline{x}_4 \;+$$

$$p(F_{11}...F_{16} \,|\, \overline{K}_1 \overline{K}_2 \overline{K}_3 K_4) \cdot \overline{x}_2 \cdot \overline{x}_3 \cdot x_4 + p(F_{11}...F_{16} \,|\, \overline{K}_1 \overline{K}_2 \overline{K}_3 \overline{K}_4) \cdot \overline{x}_2 \cdot \overline{x}_3 \cdot \overline{x}_4 \,,$$

erhält, und wobei für die bedingten Wahrscheinlichkeiten, die in derartigen Interpolationen stehen, die Bezeichnungen $a_{ik}$ und $b_{ik}$ gewählt werden, $k := 0,...,7$, und zwar $a_{ik}$ bei den Interpolationen der $Z_i$ und $b_{ik}$ bei den Interpolationen der $N_i$, so daß zum Beispiel der erste Faktor der obigen Gleichung ersetzt wird durch $a_{10}$ mit

$$a_{10} := p(F_{11}...F_{16} \,|\, \overline{K}_1 K_2 K_3 K_4),$$

und daß sich insgesamt mit $c_i := p(K_i)$ ein Gleichungssystem in den vier Unbekannten $x_i$,

$$x_1 = \cfrac{1}{1 + \cfrac{a_{10}x_2x_3x_4 + a_{11}x_2x_3\overline{x}_4 + a_{12}x_2\overline{x}_3x_4 + a_{13}x_2\overline{x}_3\overline{x}_4 + a_{14}\overline{x}_2x_3x_4 + a_{15}\overline{x}_2x_3\overline{x}_4 + a_{16}\overline{x}_2\overline{x}_3x_4 + a_{17}\overline{x}_2\overline{x}_3\overline{x}_4}{b_{10}x_2x_3x_4 + b_{11}x_2x_3\overline{x}_4 + b_{12}x_2\overline{x}_3x_4 + b_{13}x_2\overline{x}_3x_4 + b_{14}\overline{x}_2x_3x_4 + b_{15}\overline{x}_2x_3\overline{x}_4 + b_{16}\overline{x}_2\overline{x}_3x_4 + b_{17}\overline{x}_2\overline{x}_3\overline{x}_4} \left(\dfrac{\overline{c}_1}{c_1}\right)},$$

$$x_2 = \cfrac{1}{1 + \cfrac{a_{20}x_1x_3x_4 + a_{21}x_1x_3\overline{x}_4 + a_{22}x_1\overline{x}_3x_4 + a_{23}x_1\overline{x}_3\overline{x}_4 + a_{24}\overline{x}_1x_3x_4 + a_{25}\overline{x}_1x_3\overline{x}_4 + a_{26}\overline{x}_1\overline{x}_3x_4 + a_{27}\overline{x}_1\overline{x}_3\overline{x}_4}{b_{20}x_1x_3x_4 + b_{21}x_1x_3\overline{x}_4 + b_{22}x_1\overline{x}_3x_4 + b_{23}x_1\overline{x}_3\overline{x}_4 + b_{24}\overline{x}_1x_3x_4 + b_{25}\overline{x}_1x_3\overline{x}_4 + b_{26}\overline{x}_1\overline{x}_3x_4 + b_{27}\overline{x}_1\overline{x}_3\overline{x}_4} \left(\dfrac{\overline{c}_2}{c_2}\right)},$$

$$x_3 = \cfrac{1}{1 + \cfrac{a_{30}x_2x_1x_4 + a_{31}x_2x_1\overline{x}_4 + a_{32}x_2\overline{x}_1x_4 + a_{33}x_2\overline{x}_1\overline{x}_4 + a_{34}\overline{x}_2x_1x_4 + a_{35}\overline{x}_2x_1\overline{x}_4 + a_{36}\overline{x}_2\overline{x}_1x_4 + a_{37}\overline{x}_2\overline{x}_1\overline{x}_4}{b_{30}x_2x_1x_4 + b_{31}x_2x_1\overline{x}_4 + b_{32}x_2\overline{x}_1x_4 + b_{33}x_2\overline{x}_1\overline{x}_4 + b_{34}\overline{x}_2x_1x_4 + b_{35}\overline{x}_2x_1\overline{x}_4 + b_{36}\overline{x}_2\overline{x}_1x_4 + b_{37}\overline{x}_2\overline{x}_1\overline{x}_4} \left(\dfrac{\overline{c}_3}{c_3}\right)},$$

$$x_4 = \cfrac{1}{1 + \cfrac{a_{40}x_2x_3x_1 + a_{41}x_2x_3\overline{x}_1 + a_{42}x_2\overline{x}_3x_1 + a_{43}x_2\overline{x}_3\overline{x}_1 + a_{44}\overline{x}_2x_3x_1 + a_{45}\overline{x}_2x_3\overline{x}_1 + a_{46}\overline{x}_2\overline{x}_3x_1 + a_{47}\overline{x}_2\overline{x}_3\overline{x}_1}{b_{40}x_2x_3x_1 + b_{41}x_2x_3\overline{x}_1 + b_{42}x_2\overline{x}_3x_1 + b_{43}x_2\overline{x}_3\overline{x}_1 + b_{44}\overline{x}_2x_3x_1 + b_{45}\overline{x}_2x_3\overline{x}_1 + b_{46}\overline{x}_2\overline{x}_3x_1 + b_{47}\overline{x}_2\overline{x}_3\overline{x}_1}\left(\cfrac{\overline{c}_4}{c_4}\right)},$$

ergibt, wobei für alle $K_i$' zunächst Gleichwahrscheinlichkeit mit $p(K_i) := 0.25$ angenommen wird, und wobei zur Berechnung der $a_{ik}$ und $b_{ik}$ - unter Nutzung der bedingten stochastischen Unabhängigkeit der F-Elemente - eine Zerlegung bezüglich dieser F-Elemente erfolgt, zum Beispiel

$$a_{10} := p(F_{11} \mid \overline{K}_1 K_2 K_3 K_4) \cdot ... \cdot p(F_{16} \mid \overline{K}_1 K_2 K_3 K_4),$$

und wobei eine weitere Zerlegung der dadurch erscheinenden bedingten Wahrscheinlichkeiten angesetzt wird, unter Nutzung der stochastischen Selbständigkeit der $K_i$, zum Beispiel

$$p(F_{11} \mid \overline{K}_1 K_2 K_3 K_4) = \left[1 - p(\overline{F}_{11} \mid K_2 \sim) \cdot p(\overline{F}_{11} \mid K_3 \sim) \cdot p(\overline{F}_{11} \mid K_4 \sim)\right],$$

mit der Bezeichnung $\sim$ für einen Verbund, der mit Ausnahme des vor $\sim$ stehenden $K_i$ alle weiteren Elemente der konkurrierenden Diagnosen in negierter Form enthält, und wobei $(F_{ij} \mid K_i \sim) = 0$ gilt, falls $F_{ij}$ keine Folge von $K_i$ ist, und wobei zur Ausführung der fallbezogenen Diagnose die Diagnose die Faktoren $f_{ij}$ eingeführt werden mi $f_{ij} := 1$, falls $F_{ij}$ als Symptom vorhanden ist, und $f_{ij} := 0$, falls $F_{ij}$ nicht als Symptom vorhanden ist, so daß zum Beispiel $a_{10}$ übergeht in

$$a_{10} :=$$
$$[f_{11} \cdot p(F_{11} \mid \overline{K}_1 K_2 K_3 K_4) + (1 - f_{11}) \cdot (1 - p(F_{11} \mid \overline{K}_1 K_2 K_3 K_4))] \cdot$$
$$\vdots$$
$$\cdot [f_{16} \cdot p(F_{16} \mid \overline{K}_1 K_2 K_3 K_4) + (1 - f_{16}) \cdot (1 - p(F_{16} \mid \overline{K}_1 K_2 K_3 K_4))],$$

so daß man mit diesem Verfahren ein System von vier Gleichungen in den vier Unbekannten $x_i$ erhält, das durch Eingabe in ein Berechnungsprogramm gelöst wird und die Zahlenwerte der $x_i$ und der $AF(i) := \dfrac{x_i}{p(K_i)}$ liefert.

2. Verfahren wie in Anspruch 1, mit dem Unterschied, daß nur drei konkurrierende Diagnosen $K_i$', i:=1,..., 3, und alle weiteren Diagnosen in negierter Form berücksichtigt werden, mit dem daraus sich ergebenden Unterschied, daß jetzt die Wertungsgleichungen

$$x_1 := p(K_1 \mid F_{11}...F_{16} K_2' K_3' \overline{K}_4),$$

$$x_2 := p(K_2 \mid F_{21}...F_{26} K_1' K_3' \overline{K}_4),$$

$$x_3 := p(K_3 \mid F_{31}...F_{36} K_2' K_1' \overline{K}_4),$$

gelten, die nach einer Umformung übergehen in $x_i := \dfrac{1}{1 + \dfrac{Z_i}{N_i} \cdot \dfrac{p(\overline{K}_i)}{p(K_i)}}, i := 1,...,3,$ mit

$$Z_1 := p(F_{11}...F_{16} \mid \overline{K}_1 K_2' K_3' \overline{K}_4) \text{ und } N_1 := p(F_{11}...F_{16} \mid K_1 K_2' K_3' \overline{K}_4),$$

$$Z_2 := p(F_{21}...F_{26} \mid \overline{K}_2 K_1' K_3' \overline{K}_4) \text{ und } N_2 := p(F_{21}...F_{26} \mid K_2 K_1' K_3' \overline{K}_4),$$

$$Z_3 := p(F_{31}...F_{36} \mid \overline{K}_3 K_2' K_1' \overline{K}_4) \text{ und } N_3 := p(F_{31}...F_{36} \mid K_3 K_2' K_1' \overline{K}_4),$$

womit weiter wie in Anspruch 1 verfahren wird, mit dem Unterschied, daß sich die Gleihungen

$$x_1 = \dfrac{1}{1 + \dfrac{a_{10}x_2x_3 + a_{11}x_2\overline{x}_3 + + a_{12}\overline{x}_2 x_3 + a_{13}\overline{x}_2 \overline{x}_3}{b_{10}x_2x_3 + b_{11}x_2\overline{x}_3 + + b_{12}\overline{x}_2 x_3 + b_{13}\overline{x}_2 \overline{x}_3}\left(\dfrac{\overline{c}_1}{c_1}\right)},$$

$$x_2 = \dfrac{1}{1 + \dfrac{a_{20}x_1x_3 + a_{21}x_1\overline{x}_3 + a_{22}\overline{x}_1 x_3 + a_{23}\overline{x}_1 \overline{x}_3}{b_{20}x_1x_3 + b_{21}x_1\overline{x}_3 + b_{22}\overline{x}_1 x_3 + b_{23}\overline{x}_1 \overline{x}_3}\left(\dfrac{\overline{c}_2}{c_2}\right)},$$

$$x_3 = \dfrac{1}{1 + \dfrac{a_{30}x_2x_1 + a_{31}x_2\overline{x}_1 + a_{32}\overline{x}_2 x_1 + a_{33}\overline{x}_2 \overline{x}_1}{b_{30}x_2x_1 + b_{31}x_2\overline{x}_1 + b_{32}\overline{x}_2 x_1 + b_{33}\overline{x}_2 \overline{x}_1}\left(\dfrac{\overline{c}_3}{c_3}\right)}$$

ergeben, wobei zunächst für alle $K_i'$ Gleichwahrscheinlichkeit mit $p(K_i) := 0.33$, $i := 1,..., 3$, angenommen wird, und wobei für jedes $x_i$ wiederum $a_{ik}$ und $b_{ik}$, $k := 0,..., 3$, entwickelt werden, abhängig von den zu interpolierenden Ausdrücken, so daß man mit diesem Verfahren ein System von drei Gleichungen in den drei Unbekannten $x_i$ erhält.

3. Verfahren wie in den Ansprüchen 1 und 2, mit dem Unterschied, daß nur zwei konkurrierende Diagnosen $K_i'$, $i := 1,..., 2$, und alle weiteren Diagnosen in negierter Form berücksichtigt werden, mit dem daraus sich ergebenden Unterschied, daß jetzt die Wertungsgleichungen

$$x_1 := p(K_1 \mid F_{11}...F_{16} K_2' \overline{K}_3 \overline{K}_4),$$

$$x_2 := p(K_2 \mid F_{21}...F_{26} K_1' \overline{K}_3 \overline{K}_4),$$

gelten, die nach einer Umformung übergehen in
$$x_i := \cfrac{1}{1 + \cfrac{Z_i}{N_i} \cdot \cfrac{p(\overline{K_i})}{p(K_i)}}, i := 1,...,2,$$
mit

$$Z_1 := p(F_{11}...F_{16} \mid \overline{K_1} K_2' \overline{K_3} \overline{K_4}) \text{ und } N_1 := p(F_{11}...F_{16} \mid K_1 K_2' \overline{K_3} \overline{K_4}),$$

$$Z_2 := p(F_{21}...F_{26} \mid \overline{K_2} K_1' \overline{K_3} \overline{K_4}) \text{ und } N_2 := p(F_{21}...F_{26} \mid K_2 K_1' \overline{K_3} \overline{K_4}),$$

womit wie in den Ansprüchen 1 und 2 verfahren wird, mit dem Unterschied, daß sich die Gleihungen

$$x_1 = \cfrac{1}{1 + \cfrac{a_{10}x_2 + a_{11}\overline{x_2}}{b_{10}x_2 + b_{11}\overline{x_2}} \left( \cfrac{\overline{c_1}}{c_1} \right)},$$

$$x_2 = \cfrac{1}{1 + \cfrac{a_{20}x_1 + a_{21}\overline{x_1}}{b_{20}x_1 + b_{21}\overline{x_1}} \left( \cfrac{\overline{c_2}}{c_2} \right)}$$

ergeben, wobei zunächst für alle $K_i'$ Gleichwahrscheinlichkeit mit $p(K_i) := 0.5$, $i := 1,..., 2$ angenommen wird, und wobei für jedes $x_i$ eigene $a_{ik}$ und $b_{ik}$, $k := 0,..., 1$, entwickelt werden, abhängig von den zu interpolierenden Ausdrücken, so daß man mit diesem Verfahren ein System von zwei Gleichungen in den zwei Unbekannten $x_i$ erreicht.

4. Verfahren wie in den Ansprüchen 1, 2 und 3, mit dem Unterschied, daß jetzt die $a_{ik}$ und $b_{ik}$ nicht erst nach der Linearen Interpolation der $Z_i$ und der $N_i$ gebildet werden, sondern daß sie sich anhand eines schematisierten Vorgehens unmittelbar aus den $Z_i$ und $N_i$ ergeben, insbesondere zu verwenden bei fünf oder mehr strichgekennzeichneten Diagnosen, wobei das Schema am Beispiel

$$p(F_{11}...F_{16} \mid \overline{K_1} K_2' K_3' K_4')$$

dargestellt wird, und wobei in einem ersten Schritt für einen beliebigen Koeffizienten $a_{ik}$, zum Beispiel für $a_{14}$, die im Index stehende zweite Ziffer k, hier: 4, binär geschrieben wird als (100), und wobei in einem zweiten Schritt die binäre Zahl rechtsbündig auf die strichgekennzeichneten Elemente projiziert wird, im Beispiel

$$\begin{matrix} (1 & 0 & 0) \\ \downarrow & \downarrow & \downarrow \end{matrix}$$
$$p(F_{11}...F_{16} \mid \overline{K_1} K_2' K_3' K_4'),$$

wobei nachfolgend die Strichkennung entfällt, und wobei die Ziffern "1" in der binären Zahl die auszuführenden Negierungen anzeigen, so daß hier

$$a_{14} := p(F_{11}...F_{16} \mid \overline{K}_1 \overline{K}_2 K_3 K_4)$$

entsteht, wobei nun die nicht-negierten Ereignisse im Bedingungsverbund diejenigen Elemente anzeigen, die für die p($F_{ij}$ | $K_i$ ~) verbleiben, so daß sich in einem dritten Schritt durch Faktorzerlegung bezüglich der F-Elemente und durch Ursachen-Faktorzerlegung

$$a_{14} := [1 - p(\overline{F}_{11} \mid K_3 \sim) \cdot p(\overline{F}_{11} \mid K_4 \sim)] \cdot ... \cdot [1 - p(\overline{F}_{16} \mid K_3 \sim) \cdot p(\overline{F}_{16} \mid K_4 \sim)]$$

ergibt, und in einem vierten Schritt das zu dem Koeffizienten $a_{14}$ gehörende Produkt aus Unbekannten bestimmt werden kann, wobei die einzelnen Elemente des Unbekannten-Produkts dieselben Negierungen und Indizes besitzen, wie sie in Schritt 2 gewonnen wurden, das heißt, es wird

$$
\begin{array}{c}
(1 \quad 0 \quad 0) \\
\downarrow \quad \downarrow \quad \downarrow \\
p(F_{11}...F_{16} \mid \overline{K}_1 K_2' K_3' K_4') \\
\downarrow \quad \downarrow \quad \downarrow \\
p(F_{11}...F_{16} \mid \overline{K}_1 \overline{K}_2 K_3 K_4) \\
\downarrow \quad \downarrow \quad \downarrow \\
\overline{x}_2 \cdot x_3 \cdot x_4
\end{array}
$$

ausgeführt, und $\overline{x}_2 \cdot x_3 \cdot x_4$ ist das zum Koeffizienten $a_{14}$ gehörende Produkt von Unbekannten, so daß das Ergebnis steht als

$$a_{14}\overline{x}_2 x_3 x_4 := [1 - p(\overline{F}_{11} \mid K_3 \sim) \cdot p(\overline{F}_{11} \mid K_4 \sim)] \cdot ... \cdot [1 - p(\overline{F}_{16} \mid K_3 \sim) \cdot p(\overline{F}_{16} \mid K_4 \sim)] \cdot \overline{x}_2 x_3 x_4.$$

5. Verfahren wie in den Ansprüchen 1 bis 4, wobei jetzt fünf oder mehr konkurrierende Diagnosen $K_i'$ und beliebige weitere Diagnosen in negierter Form berücksichtigt werden, und wobei sich für jede weitere strichgekennzeichnete Diagnose die Anzahl der $a_{ik}$ und $b_{ik}$ jeweils verdoppelt, zum Beispiel k:= 0,..., 15 für fünf und k:= 0,..., 31 für sechs strichgekennzeichnete Diagnosen, so daß sich zur Berechnung Systeme von fünf oder mehr Gleichungen in fünf oder mehr Unbekannten $x_i$ ergeben.

6. Verfahren wie in den Ansprüchen 1 bis 5 mit dem Zusatz, daß für eine beliebige $K_i'$-Gruppierung zum Zweck geordneter Vorgehensweise zunächst für alle Diagnosen $K_i'$, zum Beispiel für $K_{i_0}$, die Zahlenwerte der $p(F_{i_0 j} \mid K_{i_0} \sim)$, j:= 1,..., 6, ermittelt werden, wobei hier die i-Indizierung bei F und K gleich ist, und daß zum Zweck einer übersichtlichen Zuordnung der Diagnosen, der Folge-Ereignisse, der Symptome und der $p(F_{i_0} \mid K_{i_0} \sim)$-Zahlenwerte die tabellarische Anordnung des nachstehenden Aufbaus verwendet wird, wobei

- alle insgesamt zu berücksichtigenden Symptome in die erste Spalte aufgenommen werden, und wobei
- für alle Diagnosen jeweils eine Spalte angelegt wird, und wobei
- alle Folge-Ereignisse einer Diagnose in der dieser Diagnose zugehörigen Spalte einzutragen sind, zusammen mit ihren $p(F_{i_0 j} \mid K_{i_0} \sim)$-Zahlenwerten, und wobei
- es so eingerichtet wird, daß identische Folge-Ereignisse, d.h. Ereignisse mit unterschiedlicher $F_{ij}$-Indizierung aber derselben Symptom-Zugehörigkeit, in einer Zeile stehen, wodurch der Zahlenwert zum Beispiel für p($F_{26}$ |$K_4$ ~) mühelos abgelesen werden kann als Null, wenn es am Kreuzungspunkt der $F_{26}$-Zeile und der $K_4$-Spalte keine Eintragung gibt, oder aber im Fall einer Eintragung als Zahlenwert, der für ein zu $K_4$ gehörendes Folge-Ereignis bereits ermittelt ist, zum Beispiel für p($F_{45}$ | $K_4$ ~), wenn nämlich - im Beispiel - die beiden Folge-Ereignisse $F_{26}$ und $F_{45}$ zum selben Symptom in der ersten Spalte der Tabelle gehören.

**7.** Verfahren wie in den Ansprüchen 1 bis 6 mit dem Unterschied, daß für ein beliebiges $K_i'$ die Anzahl der zugehörigen Folge-Ereignisse nicht mit $j := 6$ fest vorgegeben wird, sondern daß die Anzahl der Folge-Ereignisse beliebig und frei wählbar ist.

**8.** Verfahren wie in den Ansprüchen 1 bis 7 mit dem Unterschied, daß für die Diagnosen die a-priori-Wahrscheinlichkeiten nicht als gleich angenommen werden, sondern daß für die $c_i$ die wirkliche a-priori-Wahrscheinlichkeit $p(K_i)$ verwendet wird mit $c_i := p(K_i)$, wobei anhand des errechneten Endergebnisses dann entschieden werden muß, ob das höchste $AF(i)$ oder das höchste $x_i$ die zutreffende Diagnose bestimmt, so daß bei einer Nicht-Übereinstimmung die Möglichkeit herangezogen werden kann, die $c_i$ zu verändern, indem für beliebiges $K_i'$ und beliebig gewählte Ursachen von $K_i'$, zum Beispiel $U_{i1}$, $U_{i2}$..., anstatt bisher $c_i := p(K)$ jetzt $c_i := p(K_i \mid U_{i1} U_{i2} ...)$ verwendet wird, um so zugunsten größerer Diagnosesicherheit die zur Diskussion stehende Nicht-Übereinstimmung zu beseitigen, wobei bei fortbestehender Nicht-Übereinstimmung das höchste $x_i$ die zutreffende Diagnose bestimmt.

**9.** Verfahren wie in den Ansprüchen 1 bis 8, mit dem Unterschied, daß die a-priori-Wahrscheinlichkeiten $p(K_i)$ überhaupt nur dann verwendet werden, wenn die Ursachen der $K_i'$ nicht berücksichtigt werden können, und daß man bei Mitberücksichtigung von beliebigen Ursachen in einer frei wählbaren Anzahl, zum Beispiel der beliebig gewählten Ursachen $U_{i1}$ bis $U_{i4}$ eines beliebigen $K_i'$, eine Verbesserung der Zuverlässigkeit erzielt vermittels der bloßen Ersetzung von vormals $c_i := p(K_i)$ durch

$$c_i := p(K_i \mid U_{i1}), \text{ oder}$$

$$c_i := p(K_i \mid U_{i1} U_{i2}) = 1 - \frac{p(\overline{K}_i \mid U_{i1} \overline{U}_{i2}) \cdot p(\overline{K}_i \mid \overline{U}_{i1} U_{i2})}{p(\overline{K}_i \mid \overline{U}_{i1} \overline{U}_{i2})},$$

oder

$$c_i := p(K_i \mid U_{i1} U_{i2} U_{i3}) = 1 - \frac{p(\overline{K}_i \mid U_{i1} \overline{U}_{i2} \overline{U}_{i3}) \cdot p(\overline{K}_i \mid \overline{U}_{i1} U_{i2} \overline{U}_{i3}) \cdot p(\overline{K}_i \mid \overline{U}_{i1} \overline{U}_{i2} U_{i3})}{p(\overline{K}_i \mid \overline{U}_{i1} \overline{U}_{i2} \overline{U}_{i3})^2},$$

oder

$$c_i := p(K_i \mid U_{i1} U_{i2} U_{i3} U_{i4}) =$$
$$1 - \frac{p(\overline{K}_i \mid U_{i1} \overline{U}_{i2} \overline{U}_{i3} \overline{U}_{i4}) \cdot p(\overline{K}_i \mid \overline{U}_{i1} U_{i2} \overline{U}_{i3} \overline{U}_{i4}) \cdot p(\overline{K}_i \mid \overline{U}_{i1} \overline{U}_{i2} U_{i3} \overline{U}_{i4}) \cdot p(\overline{K}_i \mid \overline{U}_{i1} \overline{U}_{i2} \overline{U}_{i3} U_{i4})}{p(\overline{K}_i \mid \overline{U}_{i1} \overline{U}_{i2} \overline{U}_{i3} \overline{U}_{i4})^3},$$

wobei die $U_{i1}$ bis $U_{i4}$ stochastisch unabhängig sein müssen, und wobei ein beliebiges K'-Element, zum Beispiel $K_1'$, die Ursachen von $K_1'$ gegenüber den Folgen von $K_1'$ separiert, und wobei im Endergebnis das höchste $x_i$ die zutreffende Diagnose bestimmt.

**10.** Verfahren wie in Anspruch 9, mit dem Unterschied, daß bei beliebigem $K_i'$ die beliebig gewählten Ursachen $U_{i1}$ bis $U_{i4}$ mit ihren jeweils zugehörigen Inhibitoren $I$ ausgestattet werden, das heißt, daß ein beliebiges $U_{i1}$ einen Ereignisverbund bildet mit jenen Ereignissen $I_{U_{i1} \to K_i'}$ die genau den Kausalweg $U_{i1} \to K_i$ hemmen, und zwar mit einer Wahrscheinlichkeit $0 < p < 1$, und daß ein solcher Ereignisverbund, zum Beispiel $(U_{i1} I_{U_{i1} \to Ki})$, an die Stelle des nicht-negierten $U_{i1}$ tritt, zum Beispiel in

$$c_i := p(K_i \mid U_{i1}U_{i2}) = 1 - \frac{p(\overline{K}_i \mid U_{i1}I_{U_{i1}\to K_i}\overline{U}_{i2}) \cdot p(\overline{K}_i \mid \overline{U}_{i1}U_{i2})}{p(\overline{K}_i \mid \overline{U}_{i1}\overline{U}_{i2})},$$

und daß man so eine Verbesserung der Zuverlässigkeit und der Trennschärfe erzielt vermittels der bloßen Ergänzung der nicht-negierten $U_i$ in den Ausdrücken zur Bestimmung der $c_i$, wobei vorausgesetzt ist, daß die Elemente aus der Vereinigung von U- und I-Elementen stochastisch unabhängig sind.

11. Verfahren wie in den Ansprüchen 1 bis 10, mit dem Unterschied, daß jetzt die $K_i'$ mit ihren jeweils zugehörigen Inhibitoren J ausgestattet werden, das heißt, daß für eine beliebig herausgegriffene Wahrscheinlichkeit, zum Beispiel für $p(F_{16} \mid K_3 \sim)$, das Element $K_3$ einen Ereignisverbund bildet mit jenen Ereignissen $J_{K_3\to F_{16}}$, die genau den Kausalweg $K_3 \to F_{16}$ hemmen, und zwar mit einer Wahrscheinlichkeit $0 < p < 1$, und daß ein solcher Ereignisverbund, zum Beispiel $(K_3J_{K_3\to F_{16}\#1}J_{K_3\to F_{16}\#2})$, an die Stelle des nicht-negierten $K_i$ tritt, zum Beispiel $p(F_{16}\mid K_3 J_{K_3\to F_{16}\#1}J_{K_3\to F_{16}\#2} \sim)$, wobei #1 und #2 lediglich die fortlaufende Nummerierung bei mehr als einem Inhibitor darstellen, und daß man so eine Verbesserung der Zuverlässigkeit erzielt vermittels der bloßen Ergänzung der nicht-negierten $K_i$ bei den nach obigen Kriterien in Frage kommenden Wahrscheinlichkeiten der Form $p(F_{ij} \mid K_i \sim)$, und wobei vorausgesetzt ist, daß die Elemente aus der Vereinigung von K- und J-Elementen stochastisch unabhängig sind.

12. Verfahren wie in Anspruch 11 mit dem Unterschied, daß bei zwei oder mehr Inhibitoren eines Kausalwegs, zum Beispiel $K_3 \to F_{16}$, eine Faktorisierung bezüglich der Inhibitoren verwendet werden kann, die nach einem einfachen Muster gestaltet ist, zum Beispiel

$$p(F_{16} \mid K_3 J_{K_3\to F_{16}\#1} J_{K_3\to F_{16}\#2} J_{K_3\to F_{16}\#3} \sim) :=$$

$$\frac{p(F_{16} \mid K_3 J_{K_3\to F_{16}\#1} \sim) \cdot p(F_{16} \mid K_3 J_{K_3\to F_{16}\#2} \sim) \cdot p(F_{16} \mid K_3 J_{K_3\to F_{16}\#3} \sim)}{[p(F_{16} \mid K_3 \sim)]^{s-1}},$$

mit s = Anzahl der Inhibitoren des Kausalwegs $K_3 \to F_{16}$, und wobei vorausgesetzt ist, daß keine verdeckten Ursachen von $F_{16}$ existieren, und daß die Inhibitoren des Kausalwegs $K_3 \to F_{16}$ selbständige Ursachen des Ereignisses $\overline{(K_3 \to F_{16})}$ sind.

13. Verfahren wie in den Ansprüchen 1 bis 11, mit dem Unterschied, daß jetzt die Ursachen-Faktorzerlegung von Ausdrücken der Form $p(F_{ij} \mid K_1 \ldots K_t)$, t beliebige natürliche Zahl, bei beliebiger Negierung der $K_i$, ausgeführt wird unter Berücksichtigung verdeckter Ursachen, herrührend von der Möglichkeit, daß auch Diagnosen außerhalb der festgesetzten Menge von $K_i'$-Elementen, unter Umständen ergänzt durch zusätzliche K-Elemente in negierter Form, für die Verursachung eines einzelnen $F_{ij}$ in Frage kommen, so daß zum Beispiel die Ausdrücke $p(F_{11}\mid K_1K_2K_3K_4)$, $p(F_{11}\mid K_1K_2K_3\overline{K}_4)$, $p(F_{11}\mid K_1K_2\overline{K}_3K_4)$ zerlegt werden in

$$p(F_{11} \mid K_1K_2K_3K_4) =$$

$$1 - \frac{p(\overline{F}_{11} \mid K_1\overline{K}_2\overline{K}_3\overline{K}_4) \cdot p(\overline{F}_{11} \mid \overline{K}_1K_2\overline{K}_3\overline{K}_4) \cdot p(\overline{F}_{11} \mid \overline{K}_1\overline{K}_2K_3\overline{K}_4) \cdot p(\overline{F}_{11} \mid \overline{K}_1\overline{K}_2\overline{K}_3K_4)}{\left(p(\overline{F}_{11} \mid \overline{K}_1\overline{K}_2\overline{K}_3\overline{K}_4)\right)^3},$$

$$p(F_{11} \mid K_1 K_2 K_3 \overline{K}_4) =$$

$$1 - \frac{p(\overline{F}_{11} \mid K_1 \overline{K}_2 \overline{K}_3 \overline{K}_4) \cdot p(\overline{F}_{11} \mid \overline{K}_1 K_2 \overline{K}_3 \overline{K}_4) \cdot p(\overline{F}_{11} \mid \overline{K}_1 \overline{K}_2 K_3 \overline{K}_4)}{\left( p(\overline{F}_{11} \mid \overline{K}_1 \overline{K}_2 \overline{K}_3 \overline{K}_4) \right)^2},$$

$$p(F_{11} \mid K_1 K_2 \overline{K}_3 \overline{K}_4) = 1 - \frac{p(\overline{F}_{11} \mid K_1 \overline{K}_2 \overline{K}_3 \overline{K}_4) \cdot p(\overline{F}_{11} \mid \overline{K}_1 K_2 \overline{K}_3 \overline{K}_4)}{p(\overline{F}_{11} \mid \overline{K}_1 \overline{K}_2 \overline{K}_3 \overline{K}_4)},$$

wobei im Unterschied zu den Ansprüchen 1 bis 12 $p(F_{11} \mid \overline{K_1 K_2 K_3 K_4})$ nicht Null wird.

**14.** Verfahren wie in den Ansprüchen 1 bis 13, mit dem Unterschied, daß jetzt die Zahlenwerte der Wahrscheinlichkeiten $p(F_{i0j} \mid K_{i0} \sim)$ nicht unverändert erhalten bleiben, sondern daß nach einer als zutreffend erkannten Diagnose $K_{i0}$ die Wahrscheinlichkeit $p(F_{i0j} \mid K_{i0} \sim)$ einer Aktualisierung unterzogen wird und mit neuem Zahlenwert in das Verfahren eingeht, derart, daß zum Beispiel ein Wert

$p(F_{16} \mid K_1 \sim) = 68/100 = 0.6800$
bei bestätigt vorliegendem $K_1$ und bestätigt vorliegendem $F_{16}$ übergeht in $P(F_{16} \mid K_1 \sim) = 69/101 = 0.6832$,
und bei bestätigt vorlegendem $K_1$ und nicht vorliegendem $F_{16}$ übergeht in $p(F_{16} \mid K_1 \sim) = 68/101 = 0.6733$,
und daß auch die $p(K_i)$ aktualisiert werden, daß zum Beispiel ein Wert $p(K_i) = 150/1000 = 0.1500$
bei bestätigt vorliegendem bzw. nicht vorliegendem $K_i$ übergeht in $p(K_i) = 151/1001 = 0.1508$ bzw. $p(K_i) = 150/1001 = 0.1499$,
auszuführen im laufenden Betrieb oder nach Sammlung über einen beliebigen Zeitraum.

## EUROPÄISCHER RECHERCHENBERICHT

**Nummer der Anmeldung**

EP 14 00 2593

| **EINSCHLÄGIGE DOKUMENTE** | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (IPC) |
| A,D | EP 1 026 616 A1 (LIEBEL FRANZ-PETER DR) 9. August 2000 (2000-08-09) * das ganze Dokument * ----- | 1-14 | INV. G06F17/18 G06F19/00 |
| A | EP 1 026 615 A1 (LIEBEL FRANZ-PETER DR) 9. August 2000 (2000-08-09) * Zusammenfassung * ----- | 1-14 | |
| A | EP 0 504 457 A1 (LIEBEL FRANZ PETER DR) 23. September 1992 (1992-09-23) * Zusammenfassung * ----- | 1-14 | |
| A | EP 0 400 354 A2 (LIEBEL FRANZ-PETER DR) 5. Dezember 1990 (1990-12-05) * Zusammenfassung * ----- | 1-14 | |

**RECHERCHIERTE SACHGEBIETE (IPC)**

G06F
G06N

Der vorliegende Recherchenbericht wurde für alle Patentansprüche erstellt

| Recherchenort | Abschlußdatum der Recherche | Prüfer |
|---|---|---|
| Berlin | 31. März 2015 | Domingo Vecchioni, M |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
...........................................................................
& : Mitglied der gleichen Patentfamilie, übereinstimmendes Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 14 00 2593

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

31-03-2015

| Im Recherchenbericht angeführtes Patentdokument | Datum der Veröffentlichung | Mitglied(er) der Patentfamilie | Datum der Veröffentlichung |
|---|---|---|---|
| EP 1026616 A1 | 09-08-2000 | KEINE | |
| EP 1026615 A1 | 09-08-2000 | KEINE | |
| EP 0504457 A1 | 23-09-1992 | KEINE | |
| EP 0400354 A2 | 05-12-1990 | EP 0399082 A1<br>EP 0400354 A2 | 28-11-1990<br>05-12-1990 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 99105884 A